(19) Europäisches Patentamt
European Patent Office
Office européen des brevets

(11) EP 4 778 924 A1

(12) EUROPEAN PATENT APPLICATION
published in accordance with Art. 153(4) EPC

(43) Date of publication:
**22.07.2026 Bulletin 2026/30**

(21) Application number: **24865898.1**

(22) Date of filing: **13.09.2024**

(51) International Patent Classification (IPC):
***C07D 471/04*** *(2006.01)* ***A61K 31/437*** *(2006.01)*
***A61P 1/00*** *(2006.01)* ***A61P 1/04*** *(2006.01)*
***A61P 29/00*** *(2006.01)* ***A23L 33/10*** *(2016.01)*

(52) Cooperative Patent Classification (CPC):
**A23L 33/10; A61K 31/437; A61P 1/00; A61P 1/04; A61P 29/00; C07D 471/04**

(86) International application number:
**PCT/KR2024/013975**

(87) International publication number:
**WO 2025/058459 (20.03.2025 Gazette 2025/12)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.09.2023 KR 20230122935**
**25.06.2024 KR 20240082879**

(71) Applicant: **Huons Co., Ltd.**
**Seongnam-si, Gyeonggi-do 13449 (KR)**

(72) Inventors:
- **JIN, Yong Suk**
  **Suwon-si, Gyeonggi-do 16666 (KR)**
- **EUM, Hyun Ae**
  **Anyang-si, Gyeonggi-do 14027 (KR)**
- **KIM, Jae Hyun**
  **Yongin-si, Gyeonggi-do 17065 (KR)**
- **HA, Neul**
  **Suwon-si, Gyeonggi-do 16318 (KR)**
- **SONG, In Jae**
  **Suwon-si, Gyeonggi-do 16509 (KR)**
- **SHIN, Yeon Ju**
  **Ansan-si, Gyeonggi-do 15517 (KR)**
- **KIM, Hyun Jun**
  **Suwon-si, Gyeonggi-do 16418 (KR)**
- **KANG, Seung Jun**
  **Hwaseong-si, Gyeonggi-do 16418 (KR)**
- **LIM, Chae Jeong**
  **Ansan-si, Gyeonggi-do 15596 (KR)**
- **KIM, Hyeon**
  **Ansan-si, Gyeonggi-do 15223 (KR)**
- **KIM, Su Hyeon**
  **Suwon-si, Gyeonggi-do 16418 (KR)**

(74) Representative: **Engelhard, Markus**
**Boehmert & Boehmert**
**Anwaltspartnerschaft mbB**
**Pettenkoferstrasse 22**
**80336 München (DE)**

# (54) NOVEL IMIDAZOPYRIDINE-BASED COMPOUND AND COMPOSITION COMPRISING SAME AS ACTIVE INGREDIENT FOR PREVENTING, ALLEVIATING, OR TREATING GASTROINTESTINAL INFLAMMATORY DISEASES OR GASTRIC ACID-RELATED DISEASES

(57) The present invention relates to: a novel imidazopyridine-based compound or a pharmaceutically acceptable salt thereof; and a composition comprising same as an active ingredient for preventing, alleviating, or treating gastrointestinal inflammatory diseases or gastric acid-related diseases.

EP 4 778 924 A1

## Description

### TECHNICAL FIELD

The present invention relates to novel imidazopyridine-based compounds and compositions containing the same as an active ingredient for preventing, improving or treating gastrointestinal inflammatory diseases or gastric acid-related diseases.

### BACKGROUND ART

Gastrointestinal inflammatory diseases and gastric acid-related disorders, such as peptic ulcers, gastric and duodenal ulcers, gastritis, gastroesophageal reflux disease (GERD), and non-erosive reflux disease (NERD), are among the most common digestive disorders affecting a large portion of the population, including South Korea.

Proton pump inhibitors (PPIs) are widely used to suppress gastric acid secretion. However, existing PPIs are associated with problems in terms of efficacy and side effects. Specifically, PPIs are activated by acid secretion to form sulfenamides, which then irreversibly bind to cysteine residues in the $H^+/K^+$ ATPase, inhibiting gastric acid secretion for a long period. This can lead to adverse effects such as bacterial growth in the stomach, stimulation of proton pump activity, and the potential for tumorigenesis due to hypergastrinemia. Moreover, since existing PPIs are unstable under acidic conditions, they are often formulated as enteric-coated drugs, in which case it takes several hours for the onset of action, and it takes about 5 days for continuous administration to achieve maximum efficacy. In addition, PPIs are prodrugs activated by gastric acid and act only on the active proton pump, so there are disadvantages such as a delayed onset of maximum effect, a low effect in suppressing nocturnal acid secretion, and the need to take them before meals. Furthermore, PPIs are mainly metabolized through the CYP2C19 enzyme, and there is a large difference in pharmacological efficacy between individuals due to genetic polymorphisms of the CYP2C19 enzyme. Recently, it has been confirmed that long-term use increases the risk of hip, wrist, and spinal fractures by inhibiting calcium absorption ability and bone cell growth through gastric acid suppression.

Potassium-competitive acid blocker (P-CAB) is attracting attention to improve the above-mentioned shortcomings of PPIs. These P-CABs reversibly and competitively bind to the $K^+$ ion of proton pump ($H^+/K^+$ ATPase), an enzyme involved in the final step of gastric acid secretion in gastric parietal cells, thereby potently and rapidly inhibiting gastric acid secretion. Compared to PPIs, these P-CABs exhibit stronger inhibition at normal gastric acidity (pH 1-3). However, pharmacological activity that decreases with increasing pH is required for these P-CABs. However, some P-CABs maintain their pharmacological activity even at elevated pH, leading to some reported adverse effects. Furthermore, because P-CABs are primarily metabolized by the CYP3A4 enzyme, interindividual differences in efficacy are relatively small, and concerns about interactions with drugs metabolized by the CYP2C19 enzyme are relatively low. Furthermore, it is easy to take, regardless of whether it's before or after meals, and is expected to be highly effective in improving nocturnal symptoms, a problem with PPIs.

The present invention also relates to reversible proton pump inhibitors (P-CABs). Representative examples of previously known reversible proton pump inhibitors include fexuprazan, tegoprazan, and linaprazan.

### DISCLOSURE

#### Technical Problem

The present invention provides an imidazopyridine compound represented by the following chemical formula 1 or a pharmaceutically acceptable salt thereof as a reversible proton pump inhibitor useful for preventing, ameliorating or treating gastrointestinal inflammatory diseases and gastric acid-related diseases:

[Chemical formula 1]

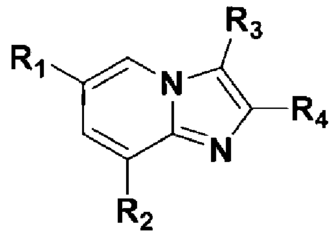

In chemical formula 1,

$R_1$ is

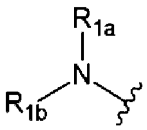, 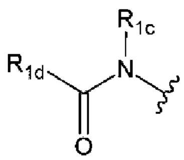, 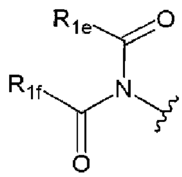, 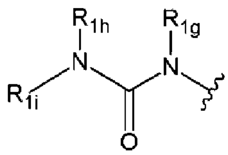,

or

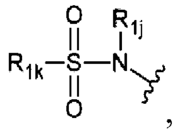,

wherein, $R_{1a}$ to $R_{1k}$ are independently hydrogen, cyano, substituted or unsubstituted C1 to C30 alkyl, substituted or unsubstituted C1 to C30 heteroalkyl, substituted or unsubstituted C2 to C30 alkenyl, substituted or unsubstituted C2 to C30 alkynyl, substituted or unsubstituted C3 to C30 cycloalkyl, substituted or unsubstituted C6 to C30 aryl, substituted or unsubstituted C7 to C30 arylalkyl, substituted or unsubstituted C2 to C30 heteroaryl, or substituted or unsubstituted C3 to C30 heteroarylalkyl, or adjacent groups among $R_{1a}$ to $R_{1k}$ are connected to each other to form a substituted or unsubstituted aliphatic ring type structure or a substituted or unsubstituted aromatic ring type structure;

$R_2$ is $-NR_{2a}R_{2b}$, wherein $R_{2a}$ or $R_{2b}$ is independently hydrogen, cyano, substituted or unsubstituted C1 to C30 alkyl, substituted or unsubstituted C2 to C30 alkenyl, substituted or unsubstituted C2 to C30 alkynyl, substituted or unsubstituted C3 to C30 cycloalkyl, substituted or unsubstituted C6 to C30 aryl, substituted or unsubstituted C7 to C30 arylalkyl, substituted or unsubstituted C2 to C30 heteroaryl, or substituted or unsubstituted C3 to C30 heteroarylalkyl;

$R_3$ or $R_4$ is independently hydrogen, cyano, substituted or unsubstituted C1 to C30 alkyl, substituted or unsubstituted C2 to C30 alkenyl, substituted or unsubstituted C2 to C30 alkynyl, substituted or unsubstituted C3 to C30 cycloalkyl, substituted or unsubstituted C6 to C30 aryl, substituted or unsubstituted C7 to C30 arylalkyl, substituted or unsubstituted C2 to C30 heteroaryl, or substituted or unsubstituted C3 to C30 heteroarylalkyl.

However, the technical problems to be solved by the present invention are not limited to the problems mentioned above, and other problems not mentioned will be clearly understood by those skilled in the art from the description below.

<u>Technical Solution</u>

The present invention provides an imidazopyridine compound represented by the following chemical formula 1 or a pharmaceutically acceptable salt thereof:

[Chemical formula 1]

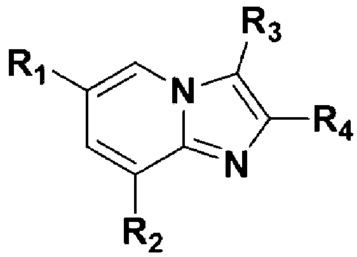

In chemical formula 1,

$R_1$ is

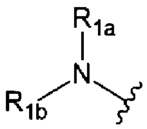, 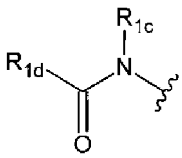, 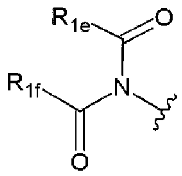, 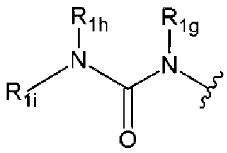,

or

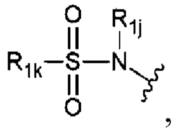,

wherein, $R_{1a}$ to $R_{1k}$ are independently hydrogen, cyano, substituted or unsubstituted C1 to C30 alkyl, substituted or unsubstituted C1 to C30 heteroalkyl, substituted or unsubstituted C2 to C30 alkenyl, substituted or unsubstituted C2 to C30 alkynyl, substituted or unsubstituted C3 to C30 cycloalkyl, substituted or unsubstituted C6 to C30 aryl, substituted or unsubstituted C7 to C30 arylalkyl, substituted or unsubstituted C2 to C30 heteroaryl, or substituted or unsubstituted C3 to C30 heteroarylalkyl, or adjacent groups among $R_{1a}$ to $R_{1k}$ are connected to each other to form a substituted or unsubstituted aliphatic ring type structure or a substituted or unsubstituted aromatic ring type structure;

$R_2$ is $-NR_{2a}R_{2b}$, wherein $R_{2a}$ or $R_{2b}$ is independently hydrogen, cyano, substituted or unsubstituted C1 to C30 alkyl, substituted or unsubstituted C2 to C30 alkenyl, substituted or unsubstituted C2 to C30 alkynyl, substituted or unsubstituted C3 to C30 cycloalkyl, substituted or unsubstituted C6 to C30 aryl, substituted or unsubstituted C7 to C30 arylalkyl, substituted or unsubstituted C2 to C30 heteroaryl, or substituted or unsubstituted C3 to C30 heteroarylalkyl;

$R_3$ or $R_4$ is independently hydrogen, cyano, substituted or unsubstituted C1 to C30 alkyl, substituted or unsubstituted C2 to C30 alkenyl, substituted or unsubstituted C2 to C30 alkynyl, substituted or unsubstituted C3 to C30 cycloalkyl, substituted or unsubstituted C6 to C30 aryl, substituted or unsubstituted C7 to C30 arylalkyl, substituted or unsubstituted C2 to C30 heteroaryl, or substituted or unsubstituted C3 to C30 heteroarylalkyl.

The above $R_1$ is

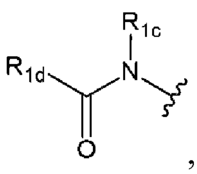, 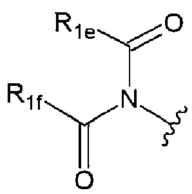, or 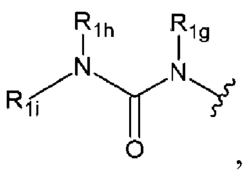,

and

at this time, $R_{1c}$ to $R_{1i}$ are independently hydrogen, substituted or unsubstituted C1 to C12 alkyl, substituted or

unsubstituted C1 to C12 heteroalkyl, substituted or unsubstituted C3 to C12 cycloalkyl, substituted or unsubstituted C6 to C12 aryl, substituted or unsubstituted C7 to C12 arylalkyl, substituted or unsubstituted C2 to C12 heteroaryl, or substituted or unsubstituted C3 to C12 heteroarylalkyl, or adjacent groups among $R_{1c}$ to $R_{1i}$ may be connected to each other to form a substituted or unsubstituted 12-membered or less aliphatic ring structure or a substituted or unsubstituted 12-membered or less aromatic ring structure.

The above $R_2$ is $-NR_{2a}R_{2b}$, wherein $R_{2a}$ is hydrogen, and $R_{2b}$ is substituted or unsubstituted C7 to C12 arylalkyl.

The above $R_3$ or $R_4$ is independently hydrogen, or substituted or unsubstituted C1 to C12 alkyl.

The compound may be at least one selected from the group consisting of

(1) N-(8-((2,6-dimethylbenzyl)amino)-2,3-dimethylimidazo[1,2-a]pyridin-6-yl)cyclopentanecarboxamide;
(2) N-(8-((2,6-dimethylbenzyl)amino)-2,3-dimethylimidazo[1,2-a]pyridin-6-yl)cyclopropanecarboxamide;
(3) N-(8-((2,6-dimethylbenzyl)amino)-2,3-dimethylimidazo[1,2-a]pyridin-6-yl)cyclohexanecarboxamide;
(4) N-(8-((2,6-dimethylbenzyl)amino)-2,3-dimethylimidazo[1,2-a]pyridin-6-yl)acetamide;
(5) N-(8-((2,6-dimethylbenzyl)amino)-2,3-dimethylimidazo[1,2-a]pyridin-6-yl)cyclobutanecarboxamide;
(6) N-(8-((2,6-dimethylbenzyl)amino)-2,3-dimethylimidazo[1,2-a]pyridin-6-yl)azetidine-1-carboxamide;
(7) N-(8-((2,6-dimethylbenzyl)amino)-2,3-dimethylimidazo[1,2-a]pyridin-6-yl)isobutyramide;
(8) N-(8-((2,6-dimethylbenzyl)amino)-2,3-dimethylimidazo[1,2-a]pyridin-6-yl)pivalamide;
(9) N-(8-((2,6-dimethylbenzyl)amino)-2,3-dimethylimidazo[1,2-a]pyridin-6-yl)-2,2-difluoroacetamide;
(10) 2-amino-N-(8-((2,6-dimethylbenzyl)amino)-2,3-dimethylimidazo[1,2-a]pyridin-6-yl)acetamide;
(11) N-(8-((2,6-dimethylbenzyl)amino)-2,3-dimethylimidazo[1,2-a]pyridin-6-yl)pyrrolidine-1-carboxamide;
(12) N-(8-((2,6-dimethylbenzyl)amino)-2,3-dimethylimidazo[1,2-a]pyridin-6-yl)bicyclo[1.1.1]pentane-1-carboxamide;
(13) N-(8-((2,6-dimethylbenzyl)amino)-2,3-dimethylimidazo[1,2-a]pyridin-6-yl)propionamide;
(14) N-(8-((2,6-dimethylbenzyl)amino)-2,3-dimethylimidazo[1,2-a]pyridin-6-yl)oxazole-2-carboxamide;
(15) N-(8-((2,6-dimethylbenzyl)amino)-2,3-dimethylimidazo[1,2-a]pyridin-6-yl)-2-methoxyacetamide;
(16) N-(8-((2,6-dimethylbenzyl)amino)-2,3-dimethylimidazo[1,2-a]pyridin-6-yl)-2,2-difluoropropanamide;
(17) N-(8-((2,6-dimethylbenzyl)amino)-2,3-dimethylimidazo[1,2-a]pyridin-6-yl)-3-fluorocyclobutane-1-carboxamide;
(18) N-(8-((2,6-dimethylbenzyl)amino)-2,3-dimethylimidazo[1,2-a]pyridin-6-yl)cyclobutanesulfonamide;
(19) (S)-2-amino-N-(8-((2,6-dimethylbenzyl)amino)-2,3-dimethylimidazo[1,2-a]pyridin-6-yl)propanamide;
(20) (R)-2-amino-N-(8-((2,6-dimethylbenzyl)amino)-2,3-dimethylimidazo[1,2-a]pyridin-6-yl)propanamide;
(21) N-(8-((4-fluoro-2,6-dimethylbenzyl)amino)-2,3-dimethylimidazo[1,2-a]pyridin-6-yl)acetamide;
(22) 1-(8-((2,6-dimethylbenzyl)amino)-2,3-dimethylimidazo[1,2-a]pyridin-6-yl)-3-ethylurea;
(23) 1-cyclobutyl-3-(8-((2,6-dimethylbenzyl)amino)-2,3-dimethylimidazo[1,2-a]pyridin-6-yl)urea;
(24) 1-cyclopropyl-3-(8-((2,6-dimethylbenzyl)amino)-2,3-dimethylimidazo[1,2-a]pyridin-6-yl)urea;
(25) 2-((8-((2,6-dimethylbenzyl)amino)-2,3-dimethylimidazo[1,2-a]pyridin-6-yl)amino)-2-oxoethyl acetate;
(26) N-(8-((2,6-dimethylbenzyl)amino)-2,3-dimethylimidazo[1,2-a]pyridin-6-yl)-2-hydroxyacetamide;
(27) 2-amino-N-(8-((2,6-dimethylbenzyl)amino)-2,3-dimethylimidazo[1,2-a]pyridin-6-yl)-2-methylpropanamide;
(28) 1-(8-((2,6-dimethylbenzyl)amino)-2,3-dimethylimidazo[1,2-a]pyridin-6-yl)azetidin-2-one;
(29) 1-(bicyclo[1.1.1]pentan-1-yl)-3-(8-((2,6-dimethylbenzyl)amino)-2,3-dimethylimidazo[1,2-a]pyridin-6-yl)urea;
(30) 1-(cyclopropylmethyl)-3-(8-((2,6-dimethylbenzyl)amino)-2,3-dimethylimidazo[1,2-a]pyridin-6-yl)urea;
(31) 1-(8-((2,6-dimethylbenzyl)amino)-2,3-dimethylimidazo[1,2-a]pyridin-6-yl)-3-isopropylurea;
(32) 3-(8-((2,6-dimethylbenzyl)amino)-2,3-dimethylimidazo[1,2-a]pyridin-6-yl)-1-ethyl-1-methylurea;
(33) 3-(8-((2,6-dimethylbenzyl)amino)-2,3-dimethylimidazo[1,2-a]pyridin-6-yl)-1-ethyl-1-methylurea;
(34) 3-((8-((2,6-dimethylbenzyl)amino)-2,3-dimethylimidazo[1,2-a]pyridin-6-yl)amino)-3-oxopropanoate;
(35) (S)-N-(8-((2,6-dimethylbenzyl)amino)-2,3-dimethylimidazo[1,2-a]pyridin-6-yl)azetidine-2-carboxamide;
(36) (R)-N-(8-((2,6-dimethylbenzyl)amino)-2,3-dimethylimidazo[1,2-a]pyridin-6-yl)azetidine-2-carboxamide;
(37) 1-(2-aminoethyl)-3-(8-((2,6-dimethylbenzyl)amino)-2,3-dimethylimidazo[1,2-a]pyridin-6-yl)urea;
(38) 4-((8-((2,6-dimethylbenzyl)amino)-2,3-dimethylimidazo[1,2-a]pyridin-6-yl)amino)-4-oxobutanoic acid;
(39) 3-(3-((8-((2,6-dimethylbenzyl)amino)-2,3-dimethylimidazo[1,2-a]pyridin-6-yl)amino)-3-oxopropyl)propanoic acid;
(40) ethyl 4-((8-((2,6-dimethylbenzyl)amino)-2,3-dimethylimidazo[1,2-a]pyridin-6-yl)amino)-4-oxobutanoate;
(41) tert-butyl 3-(3-((8-((2,6-dimethylbenzyl)amino)-2,3-dimethylimidazo[1,2-a]pyridin-6-yl)amino)-3-oxopropyl)propanoate;
(42) tert-butyl (2-(3-(8-((2,6-dimethylbenzyl)amino)-2,3-dimethylimidazo[1,2-a]pyridin-6-yl)ureido)ethyl)carbamate;
(43) 3-(8-((2,6-dimethylbenzyl)amino)-2,3-dimethylimidazo[1,2-a]pyridin-6-yl)-1,1-dimethylurea;
(44) 1-(8-((4-fluoro-2,6-dimethylbenzyl)amino)-2,3-dimethylimidazo[1,2-a]pyridin-6-yl)-3-methylurea;
(45) N-(8-((4-fluoro-2,6-dimethylbenzyl)amino)-2,3-dimethylimidazo[1,2-a]pyridin-6-yl)acetamide;

(46) methyl ((8-((2,6-dimethylbenzyl)amino)-2,3-dimethylimidazo[1,2-a]pyridin-6-yl)carbamoyl)glycinate;
(47) 2-(3-(8-((2,6-dimethylbenzyl)amino)-2,3-dimethylimidazo[1,2-a]pyridin-6-yl)ureido)-N-methylacetamide;
(48) 2-(3-(8-((2,6-dimethylbenzyl)amino)-2,3-dimethylimidazo[1,2-a]pyridin-6-yl)ureido)-N,N-dimethylacetamide;
(49) 3-(8-((4-fluoro-2,6-dimethylbenzyl)amino)-2,3-dimethylimidazo[1,2-a]pyridin-6-yl)-1,1-dimethylurea;
(50) 1-cyclopropyl-3-(8-((4-fluoro-2,6-dimethylbenzyl)amino)-2,3-dimethylimidazo[1,2-a]pyridin-6-yl)urea;
(51) N-(8-((4-fluoro-2,6-dimethylbenzyl)amino)-2,3-dimethylimidazo[1,2-a]pyridin-6-yl)azetidine-1-carboxamide;
(52) 2-acetamido-N-(8-((2,6-dimethylbenzyl)amino)-2,3-dimethylimidazo[1,2-a]pyridin-6-yl)acetamide;
(53) 2-(dimethylamino)-N-(8-((2,6-dimethylbenzyl)amino)-2,3-dimethylimidazo[1,2-a]pyridin-6-yl)acetamide;
(54) 1-butyl-3-(8-((2,6-dimethylbenzyl)amino)-2,3-dimethylimidazo[1,2-a]pyridin-6-yl)urea;
(55) N-(2-((8-((2,6-dimethylbenzyl)amino)-2,3-dimethylimidazo[1,2-a]pyridin-6-yl)amino)-2-oxoethyl)pivalamide;
(56) N-(2-((8-((2,6-dimethylbenzyl)amino)-2,3-dimethylimidazo[1,2-a]pyridin-6-yl)amino)-2-oxoethyl)-2,2-difluoroacetamide;
(57) N-(2-((8-((2,6-dimethylbenzyl)amino)-2,3-dimethylimidazo[1,2-a]pyridin-6-yl)amino)-2-oxoethyl)-2,2-difluoropropanamide;
(58) N-(2-((8-((2,6-dimethylbenzyl)amino)-2,3-dimethylimidazo[1,2-a]pyridin-6-yl)amino)-2-oxoethyl)-3-hydroxycyclobutane-1-carboxamide;
(59) N-(2-((8-((2,6-dimethylbenzyl)amino)-2,3-dimethylimidazo[1,2-a]pyridin-6-yl)amino)-2-oxoethyl)-3-fluorocyclobutane-1-carboxamide;
(60) 3-(8-((2,6-dimethylbenzyl)amino)-2,3-dimethylimidazo[1,2-a]pyridin-6-yl)imidazolidine-2,4-dione;
(61) N-(8-((2,6-dimethylbenzyl)amino)-2,3-dimethylimidazo[1,2-a]pyridin-6-yl)morpholine-4-carboxamide;
(62) 2-(3-(8-((2,6-dimethylbenzyl)amino)-2,3-dimethylimidazo[1,2-a]pyridin-6-yl)ureido)ethyl acetate;
(63) (S)-2-amino-N-(8-((4-fluoro-2,6-dimethylbenzyl)amino)-2,3-dimethylimidazo[1,2-a]pyridin-6-yl)propanamide;
(64) 2-amino-N-(8-((4-fluoro-2,6-dimethylbenzyl)amino)-2,3-dimethylimidazo[1,2-a]pyridin-6-yl)acetamide;
(65) (S)-N-(8-((4-fluoro-2,6-dimethylbenzyl)amino)-2,3-dimethylimidazo[1,2-a]pyridin-6-yl)azetidine-2-carboxamide;
(66) N-(8-((2,6-dimethylbenzyl)amino)-2,3-dimethylimidazo[1,2-a]pyridin-6-yl)-2-azaspiro[3.3]heptane-2-carboxamide;
(67) N-(8-((2,6-dimethylbenzyl)amino)-2,3-dimethylimidazo[1,2-a]pyridin-6-yl)-2-azaspiro[3.3]heptane-2-carboxamide;
(68) N-(8-((2,6-dimethylbenzyl)amino)-2,3-dimethylimidazo[1,2-a]pyridin-6-yl)-1,3-dihydro-2H-pyrrolo[3,4-c]pyridine-2-carboxamide;
(69) N-(8-((2,6-dimethylbenzyl)amino)-2,3-dimethylimidazo[1,2-a]pyridin-6-yl)-3-oxoazetidine-1-carboxamide;
(70) ((8-((2,6-dimethylbenzyl)amino)-2,3-dimethylimidazo[1,2-a]pyridin-6-yl)carbamoyl)glycine;
(71) 3-(3-(8-((2,6-dimethylbenzyl)amino)-2,3-dimethylimidazo[1,2-a]pyridin-6-yl)ureido)propionic acid;
(72) 3-(8-((2,6-dimethylbenzyl)amino)-2,3-dimethylimidazo[1,2-a]pyridin-6-yl)-1,3,5-triazepane-2,4-dione;
(73) N-(2-(3-(8-((2,6-dimethylbenzyl)amino)-2,3-dimethylimidazo[1,2-a]pyridin-6-yl)ureido)ethyl)acetamide;
(74) 1-(8-((2,6-dimethylbenzyl)amino)-2,3-dimethylimidazo[1,2-a]pyridin-6-yl)-3-((tetrohydrofuran-3-yl)methyl)urea;
(75) (R)-N-(8-((2,6-dimethylbenzyl)amino)-2,3-dimethylimidazo[1,2-a]pyridin-6-yl)-2-methylazetidine-2-carboxamide;
(76) (R)-2-(azetidin-2-yl)-N-(8-((2,6-dimethylbenzyl)amino)-2,3-dimethylimidazo[1,2-a]pyridin-6-yl)acetamide;
(77) N-(8-((2,6-dimethylbenzyl)amino)-2,3-dimethylimidazo[1,2-a]pyridin-6-yl)-3-phenylazetidine-1-carboxamide;
(78) (S)-1-(2,3-dihydro-1H-inden-1-yl)-3-(8-((2,6-dimethylbenzyl)amino)-2,3-dimethylimidazo[1,2-a]pyridin-6-yl)urea;
(79) (S)-N2-(8-((2,6-dimethylbenzyl)amino)-2,3-dimethylimidazo[1,2-a]pyridin-6-yl)-N1-methylazetidine-1,2-dicarboxamide;
(80) (R)-1-(2,3-dihydro-1H-inden-1-yl)-3-(8-((2,6-dimethylbenzyl)amino)-2,3-dimethylimidazo[1,2-a]pyridin-6-yl)urea;
(81) N-(8-((2,6-dimethylbenzyl)amino)-2,3-dimethylimidazo[1,2-a]pyridin-6-yl)-2-(2-oxoazetidin-1-yl)acetamide;
(82) N-(8-((2,6-dimethylbenzyl)amino)-2,3-dimethylimidazo[1,2-a]pyridin-6-yl)-4-phenylpiperidine-1-carboxamide;
(83) N-(8-((2,6-dimethylbenzyl-1)amino)-2,3-dimethylimidazo[1,2-a]pyridin-6-yl)-3-phenylpiperidine-1-carboxamide;
(84) N-(8-((2,6-dimethylbenzyl)amino)-2,3-dimethylimidazo[1,2-a]pyridin-6-yl)-3-phenylpyrrolidine-1-carboxamide;
(85) (R)-N-(8-((2,6-dimethylbenzyl)amino)-2,3-dimethylimidazo[1,2-a]pyridin-6-yl)piperazine-2-carboxamide;
(86) N-(8-((4-fluoro-2,6-dimethylbenzyl)amino)-2,3-dimethylimidazo[1,2-a]pyridin-6-yl)-2-hydroxyacetamide;
(87) 1-(2-aminoethyl)-3-(8-((2,6-dimethylbenzyl)amino)-2,3-dimethylimidazo[1,2-a]pyridin-6-yl)-1-methylurea;
(88) 1-(8-((2,6-dimethylbenzyl)amino)-2,3-dimethylimidazo[1,2-a]pyridin-6-yl)-3-(2-hydroxyethyl)urea;
(89) (R)-N-(8-((2,6-dimethylbenzyl)amino)-2,3-dimethylimidazo[1,2-a]pyridin-6-yl)pyrrolidine-3-carboxamide;

(90) (R)-N-(8-((2,6-dimethylbenzyl)amino)-2,3-dimethylimidazo[1,2-a]pyridin-6-yl)piperidine-2-carboxamide;
(91) (R)-N-(8-((2,6-dimethylbenzyl)amino)-2,3-dimethylimidazo[1,2-a]pyridin-6-yl)-2-(pyrrolidin-2-yl)acetamide;
(92) $N^8$-(2,6-dimethylbenzyl)-N6-isopropyl-2,3-dimethylimidazo[1,2-a]pyridin-6,8-diamine;
(93) (S)-N-(8-((2,6-dimethylbenzyl)amino)-2,3-dimethylimidazo[1,2-a]pyridin-6-yl)-2-(pyrrolidin-2-yl)acetamide;
(94) (S)-N-(8-((2,6-dimethylbenzyl)amino)-2,3-dimethylimidazo[1,2-a]pyridin-6-yl)piperazine-2-carboxamide;
(95) N-(8-((2,6-dimethylbenzyl)amino)-2,3-dimethylimidazo[1,2-a]pyridin-6-yl)-2-(2-hydroxyacetamido)acetamide;
(96) (S)-N-(8-((2,6-dimethylbenzyl)amino)-2,3-dimethylimidazo[1,2-a]pyridin-6-yl)-2-hydroxypropanamide;
(97) (R)-N-(8-((2,6-dimethylbenzyl)amino)-2,3-dimethylimidazo[1,2-a]pyridin-6-yl)-2-hydroxypropanamide;
(98) N-(8-((2,6-dimethylbenzyl)amino)-2,3-dimethylimidazo[1,2-a]pyridin-6-yl)-2-(methylamino)acetamide;
(99) 3-amino-N-(8-((2,6-dimethylbenzyl)amino)-2,3-dimethylimidazo[1,2-a]pyridin-6-yl)propanamide;
(100) N-(8-((4-fluoro-2,6-dimethylbenzyl)amino)-2,3-dimethylimidazo[1,2-a]pyridin-6-yl)-2-methoxyacetamide;
(101) N-(8-((4-fluoro-2,6-dimethylbenzyl)amino)-2,3-dimethylimidazo[1,2-a]pyridin-6-yl)-3-hydroxypropanamide;
(102) N-(8-((2,6-dimethylbenzyl)amino)-2,3-dimethylimidazo[1,2-a]pyridin-6-yl)-3-hydroxypropanamide;
(103) N-(8-((2,6-dimethylbenzyl)amino)-2,3-dimethylimidazo[1,2-a]pyridin-6-yl)piperazine-1-carboxamide;
(104) N-(8-((2,6-dimethylbenzyl)amino)-2,3-dimethylimidazo[1,2-a]pyridin-6-yl)benzamide;
(105) (S)-N-(8-((2,6-dimethylbenzyl)amino)-2,3-dimethylimidazo[1,2-a]pyridin-6-yl)pyrrolidine-3-carboxamide;
(106) (S)-N-(8-((2,6-dimethylbenzyl)amino)-2,3-dimethylimidazo[1,2-a]pyridin-6-yl)piperidine-2-carboxamide;
(107) (E)-4-(3-((8-((2,6-dimethylbenzyl)amino)-2,3-dimethylimidazo[1,2-a]pyridin-6-yl)amino)-3-oxoprop-1-en-1-yl) phenyl acetate;
(108) (E)-4-(3-((8-((2,6-dimethylbenzyl)amino)-2,3-dimethylimidazo[1,2-a]pyridin-6-yl)amino)-3-oxoprop-1-en-1-yl)-2-methoxyphenyl acetate;
(109) (S)-N-(8-((2,6-dimethylbenzyl)amino)-2,3-dimethylimadizo[1,2-a]pyridin-6-yl)aziridine-2-carboxamide;
(110) (S)-N-(8-((2,6-dimethylbenzyl)amino)-2,3-dimethylimidazo[1,2-a]pyridin-6-yl)-1-(2-hydroxyacetyl)pyrrolidine-3-carboxamide;
(111) (R)-1-((8-((4-fluoro-2,6-dimethylbenzyl)amino)-2,3-dimethylimidazo[1,2-a]pyridin-6-yl)amino)-1-oxopropan-2-yl acetate;
(112) (S)-1-((8-((4-fluoro-2,6-dimethylbenzyl)amino)-2,3-dimethylimidazo[1,2-a]pyridin-6-yl)amino)-1-oxopropan-2-yl acetate;
(113) 1-((8-((4-fluoro-2,6-dimethylbenzyl)amino)-2,3-dimethylimidazo[1,2-a]pyridin-6-yl)carbamoyl)cyclopropyl acetate;
(114) (S)-N-(8-((4-fluoro-2,6-dimethylbenzyl)amino)-2,3-dimethylimidazo[1,2-a]pyridin-6-yl)-2-hydroxypropanamide;
(115) N-(8-((4-fluoro-2,6-dimethylbenzyl)amino)-2,3-dimethylimidazo[1,2-a]pyridin-6-yl)-1-hydroxycyclopropane-1-carboxamide;
(116) 3-(8-((4-fluoro-2,6-dimethylbenzyl)amino)-2,3-dimethylimidazo[1,2-a]pyridin-6-yl)-1-methylimidazolidine-2,4-dione;
(117) (R)-N-(8-((4-fluoro-2,6-dimethylbenzyl)amino)-2,3-dimethylimidazo[1,2-a]pyridin-6-yl)-2-hydroxypropanamide;
(118) (S)-N-(8-((2,6-dimethylbenzyl)amino)-2,3-dimethylimidazo[1,2-a]pyridin-6-yl)piperidine-3-carboxamide;
(119) N-(8-((2,6-dimethylbenzyl)amino)-2,3-dimethylimidazo[1,2-a]pyridin-6-yl)-3-(2-hydroxyacetamido)propanamide;
(120) (S)-N-(8-((2,6-dimethylbenzyl)amino)-2,3-dimethylimidazo[1,2-a]pyridin-6-yl)-1-(2-hydroxyacetyl)piperidine-3-carboxamide;
(121) $N^8$-(2,6-dimethylbenzyl)-2,3-dimethylimidazo[1,2-a]pyridine-6,8-diamine; and
(122) $N^8$-(4-fluoro-2,6-dimethylbenzyl)-2,3-dimethylimidazo[1,2-a]pyridine-6,8-diamine.

The compound or a pharmaceutically acceptable salt thereof may have the ability to inhibit proton pump activity.

In one embodiment, the present invention provides a pharmaceutical composition for preventing or treating gastrointestinal inflammatory diseases or gastric acid-related diseases, comprising the imidazopyridine compound or a pharmaceutically acceptable salt thereof as an active ingredient.

The compound or a pharmaceutically acceptable salt thereof can act as a reversible proton pump inhibitor.

The compound or a pharmaceutically acceptable salt thereof can inhibit gastric acid secretion.

The gastrointestinal inflammatory disease or gastric acid-related disease may be at least one selected from the group consisting of peptic ulcer, gastric·duodenal ulcer, nonsteroidal anti-inflammatory drug (NSAID)-induced ulcer, Helicobacter pylori infection, functional dyspepsia, Zollinger-Ellison syndrome, gastritis, gastroesophageal reflux disease (GERD), and non-erosive reflux disease (NERD).

In another embodiment, the present invention provides a health functional food composition for preventing or improving gastrointestinal inflammatory disease or gastric acid-related disease, comprising the imidazopyridine compound or a pharmaceutically acceptable salt thereof as an active ingredient.

## Advantageous Effects

The novel imidazopyridine compound or pharmaceutically acceptable salt thereof according to the present invention can act as a reversible proton pump inhibitor due to its proton pump inhibitory activity. Furthermore, it has the advantage of being useful for the prevention, improvement, or treatment of gastrointestinal inflammatory diseases or gastric acid-related diseases by effectively suppressing gastric acid secretion.

## BEST MODE

The present inventors have made efforts to develop a reversible proton pump inhibitor that can be useful for preventing, improving, or treating gastrointestinal inflammatory diseases and gastric acid-related diseases such as peptic ulcer, gastric duodenal ulcer, gastritis, gastroesophageal reflux disease (GERD), and non-erosive reflux disease (NERD). As a result, the present inventors have synthesized novel imidazopyridine compounds, and confirmed that they have proton pump inhibitory activity in *in vitro* experiments and effectively inhibit gastric acid secretion in *in vivo* experiments, thereby completing the present invention.

The present invention will be described in detail below.

## Novel Imidazopyridine Compound or Pharmaceutically Acceptable Salt Thereof

The present invention provides an imidazopyridine compound represented by the following chemical formula 1 or a pharmaceutically acceptable salt thereof:

[Chemical formula 1]

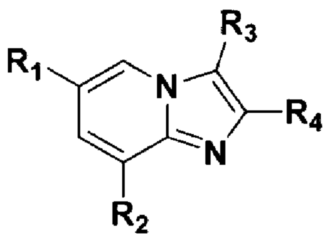

In chemical formula 1,

$R_1$ is

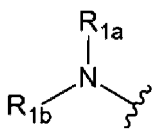,
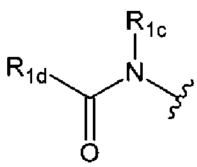,
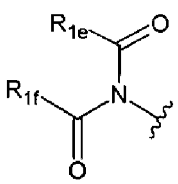,
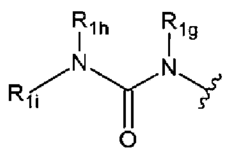,

or

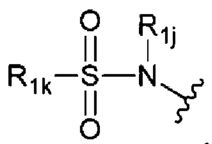,

wherein, $R_{1a}$ to $R_{1k}$ are independently hydrogen, cyano, substituted or unsubstituted C1 to C30 alkyl, substituted or unsubstituted C1 to C30 heteroalkyl, substituted or unsubstituted C2 to C30 alkenyl, substituted or unsubstituted C2 to

C30 alkynyl, substituted or unsubstituted C3 to C30 cycloalkyl, substituted or unsubstituted C6 to C30 aryl, substituted or unsubstituted C7 to C30 arylalkyl, substituted or unsubstituted C2 to C30 heteroaryl, or substituted or unsubstituted C3 to C30 heteroarylalkyl, or adjacent groups among $R_{1a}$ to $R_{1k}$ are connected to each other to form a substituted or unsubstituted aliphatic ring type structure or a substituted or unsubstituted aromatic ring type structure;

$R_2$ is $-NR_{2a}R_{2b}$, wherein $R_{2a}$ or $R_{2b}$ is independently hydrogen, cyano, substituted or unsubstituted C1 to C30 alkyl, substituted or unsubstituted C2 to C30 alkenyl, substituted or unsubstituted C2 to C30 alkynyl, substituted or unsubstituted C3 to C30 cycloalkyl, substituted or unsubstituted C6 to C30 aryl, substituted or unsubstituted C7 to C30 arylalkyl, substituted or unsubstituted C2 to C30 heteroaryl, or substituted or unsubstituted C3 to C30 heteroarylalkyl;

$R_3$ or $R_4$ is independently hydrogen, cyano, substituted or unsubstituted C1 to C30 alkyl, substituted or unsubstituted C2 to C30 alkenyl, substituted or unsubstituted C2 to C30 alkynyl, substituted or unsubstituted C3 to C30 cycloalkyl, substituted or unsubstituted C6 to C30 aryl, substituted or unsubstituted C7 to C30 arylalkyl, substituted or unsubstituted C2 to C30 heteroaryl, or substituted or unsubstituted C3 to C30 heteroarylalkyl.

As used herein, "heteroalkyl" refers to an alkyl group containing a heteroatom. Here, "C" represents the number of carbon atoms not substituted with a heteroatom.

Furthermore, as used herein, "cycloalkyl" refers to an aliphatic ring structure composed of carbon atoms, and "aryl" refers to an aromatic ring structure composed of carbon atoms. Meanwhile, as used herein, "heteroaryl" encompasses both aliphatic ring structures containing heteroatoms and substituted or unsubstituted aromatic ring structures. Here, "C" represents the number of carbon atoms not substituted with a heteroatom.

As used herein, "substituted" refers to substitution by halogen; hydroxyl; C1 to C30 (particularly C1 to C12) alkoxy; amine; C1 to C30 (particularly C1 to C12) alkylamine; carboxyl; further substituted or unsubstituted C6 to C30 (particularly C6 to C12) aryl; further substituted or unsubstituted C1 to C30 (particularly C1 to C12) alkyl linked via an ester, amide, ether, or carbonyl bond; or further substituted or unsubstituted C3 to C30 (particularly C3 to C12) cycloalkyl linked via an ester, amide, ether, or carbonyl bond.

In addition, as used herein, "further substituted" refers to substitution by halogen; hydroxyl; C1 to C30 (particularly C1 to C12) alkoxy; amine; C1 to C30 (especially C1 to C12) alkylamine; carboxyl; C6 to C30 (especially C6 to C12) aryl; C1 to C30 (especially C1 to C12) alkyl linked via an ester, amide, ether or carbonyl bond; or C3 to C30 (especially C3 to C12) cycloalkyl linked via an ester, amide, ether or carbonyl bond.

Specifically, in chemical formula 1, when $R_1$ is

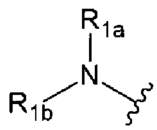
,

$R_{1a}$ or $R_{1b}$ can independently be hydrogen, or substituted or unsubstituted C1 to C30 alkyl, and it is preferable that $R_{1a}$ or $R_{1b}$ independently be hydrogen, or substituted or unsubstituted C1 to C12 alkyl, but this is not limited thereto. For example, $R_{1a}$ or $R_{1b}$ can independently be hydrogen or isopropyl.

In addition, in the above chemical formula 1, when $R_1$ is

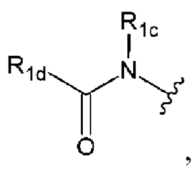
,

first, $R_{1c}$ may be hydrogen, or substituted or unsubstituted C1 to C30 alkyl, and it is preferable that $R_{1c}$ is hydrogen, or substituted or unsubstituted C1 to C12 alkyl, but is not limited thereto. For example, $R_{1c}$ may be hydrogen. Meanwhile, $R_{1d}$ may be hydrogen, substituted or unsubstituted C1 to C30 alkyl, substituted or unsubstituted C1 to C30 heteroalkyl, substituted or unsubstituted C2 to C30 alkenyl, substituted or unsubstituted C3 to C30 cycloalkyl, substituted or unsubstituted C6 to C30 aryl, substituted or unsubstituted C7 to C30 arylalkyl, substituted or unsubstituted C2 to C30 heteroaryl, or substituted or unsubstituted C3 to C30 heteroarylalkyl. Preferably, $R_{1d}$ may be hydrogen, substituted or unsubstituted C1 to C12 alkyl, substituted or unsubstituted C1 to C12 heteroalkyl, substituted or unsubstituted C3 to C12 cycloalkyl, substituted or unsubstituted C6 to C12 aryl, substituted or unsubstituted C7 to C12 arylalkyl, substituted or unsubstituted C2 to C12 heteroaryl, or substituted or unsubstituted C3 to C12 heteroarylalkyl. For example, $R_{1d}$ may be cyclopentyl, cyclopropyl, cyclohexyl, methyl, cyclobutyl, isopropyl, tert-butyl, difluoromethyl, aminomethyl, bicyclo[1.1.1]pentan-1-yl, ethyl, oxazol-2-yl, methoxymethyl, 1,1-difluoroethyl, 3-fluorocyclobutyl, 1-aminoethyl, methoxy, 1-aminoisopropyl, dimethylaminomethyl, 1-aminoethyl, 1-ethanol, methylaminomethyl, 2-aminoethyl, 2-ethanol, phenyl, cyclopropanol,

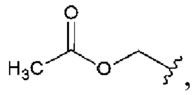, 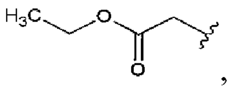, 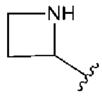, 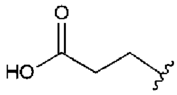,

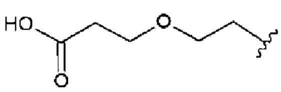, 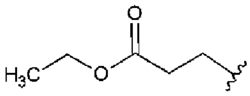, 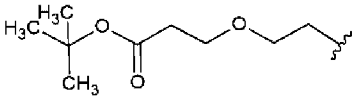, 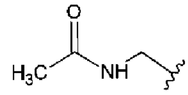,

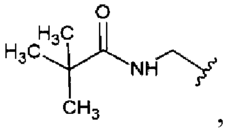, 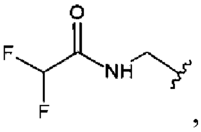, 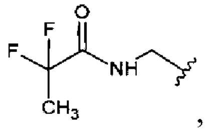, 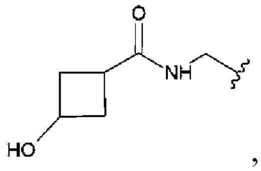, 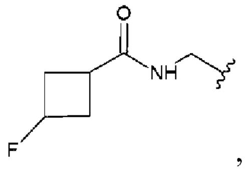,

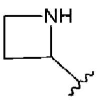, 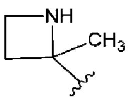, 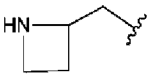, 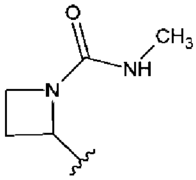, 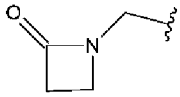, 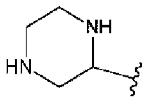, 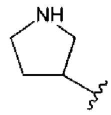,

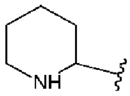, 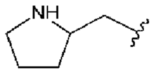, 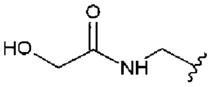, 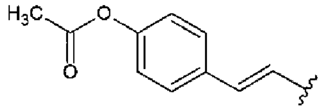,

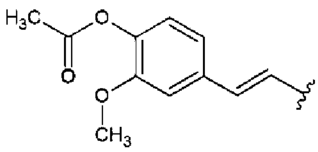, 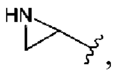, 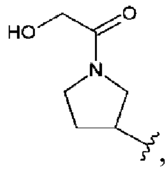, 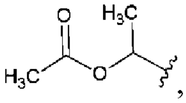, 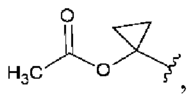, 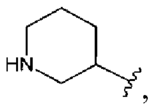,

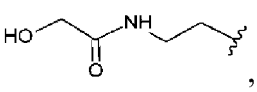, or 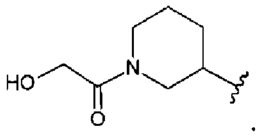.

In particular, it is preferable that $R_{1c}$ is hydrogen, and $R_{1d}$ is methyl, aminomethyl, methoxymethyl, methoxy,

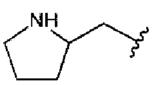, 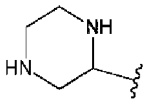,

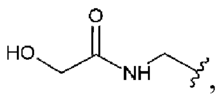,

2-ethanol, cyclopropanol, or 1-ethanol in terms of inhibition of proton pump activity.

Or, $R_{1c}$ and $R_{1d}$ may be connected to each other to form a substituted or unsubstituted aliphatic ring structure or a

substituted or unsubstituted aromatic ring structure, and it is preferable that $R_{1c}$ and $R_{1d}$ are connected to each other to form a substituted or unsubstituted 12-membered or less aliphatic ring structure or a substituted or unsubstituted 12-membered or less aromatic ring structure, but is not limited thereto. For example, $R_1$ itself may be

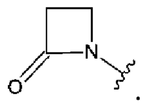

.

In addition, in chemical formula 1, when $R_1$ is

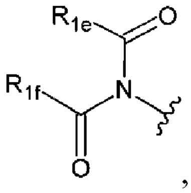

,

$R_{1e}$ and $R_{1f}$ may be connected to each other to form a substituted or unsubstituted aliphatic ring structure or a substituted or unsubstituted aromatic ring structure, and it is preferable that $R_{1e}$ and $R_{1r}$ be connected to each other to form a substituted or unsubstituted 12-membered or less aliphatic ring structure or a substituted or unsubstituted 12-membered or less aromatic ring structure, but it is not limited thereto. For example, $R_1$ itself may be

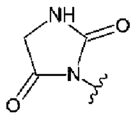

,

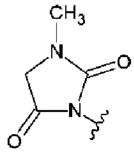

,

or

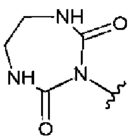

.

In particular, it is preferable that $R_1$ itself is

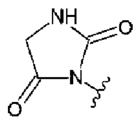

from the perspective of inhibition of proton pump activity.

In addition, in chemical formula 1, when $R_1$ is

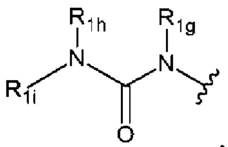

,

first, $R_{1g}$ may be hydrogen, or substituted or unsubstituted C1 to C30 alkyl, and it is preferable that $R_{1g}$ be hydrogen, or substituted or unsubstituted C1 to C12 alkyl, but it is not limited thereto. For example, $R_{1g}$ may be hydrogen. Meanwhile, $R_{1h}$ or $R_{1i}$ may independently be hydrogen, substituted or unsubstituted C1 to C30 alkyl, substituted or unsubstituted C1 to C30 heteroalkyl, substituted or unsubstituted C2 to C30 alkenyl, substituted or unsubstituted C3 to C30 cycloalkyl, substituted or unsubstituted C6 to C30 aryl, substituted or unsubstituted C7 to C30 arylalkyl, substituted or unsubstituted

C2 to C30 heteroaryl, or substituted or unsubstituted C3 to C30 heteroarylalkyl. Preferably, $R_{1d}$ may be hydrogen, substituted or unsubstituted C1 to C12 alkyl, substituted or unsubstituted C1 to C12 heteroalkyl, substituted or unsubstituted C3 to C12 cycloalkyl, substituted or unsubstituted C6 to C12 aryl, substituted or unsubstituted C7 to C12 arylalkyl, substituted or unsubstituted C2 to C12 heteroaryl, or substituted or unsubstituted C3 to C12 heteroarylalkyl. For example, $R_{1h}$ or $R_{1i}$ may independently be hydrogen, ethyl, cyclobutyl, cyclopropyl, bicyclo[1.1.1]pentan-1-yl, cyclopropylmethyl, isopropyl, 2-aminoethyl, cyano, methyl, 2-ethanol,

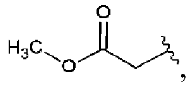,

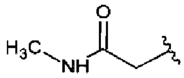, 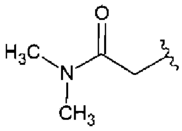, 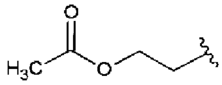, 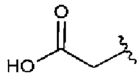, 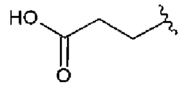,

or

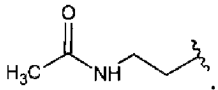.

In particular, it is preferable in terms of inhibition of proton pump activity that $R_{1g}$ is hydrogen, $R_{1h}$ is hydrogen or methyl, and $R_{1i}$ is cyclopropyl, methyl, 2-aminoethyl, or 2-ethanol.

Alternatively, $R_{1h}$ and $R_{1i}$ may be connected to each other to form a substituted or unsubstituted aliphatic ring structure or a substituted or unsubstituted aromatic ring structure, and it is preferable that $R_{1h}$ and $R_{1i}$ be connected to each other to form a substituted or unsubstituted 12-membered or less aliphatic ring structure or a substituted or unsubstituted 12-membered or less aromatic ring structure, but this is not limited thereto. For example, $R_1$ itself may be

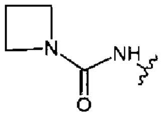, 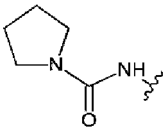, 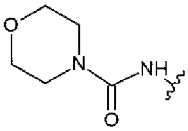, 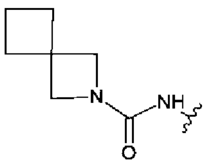,

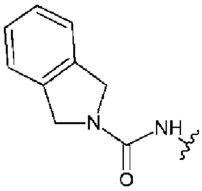, 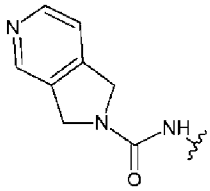, 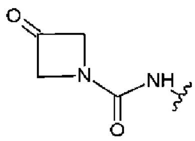, 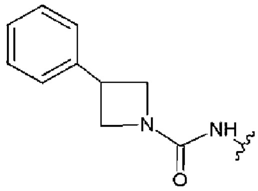,

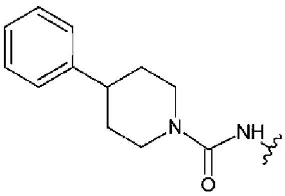, 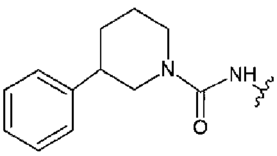, 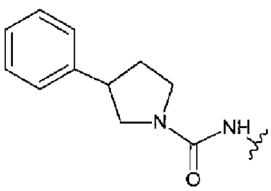, or 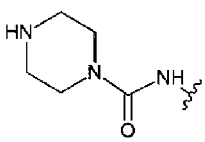.

In particular, $R_1$ itself is preferably

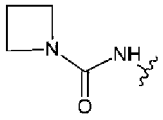, or 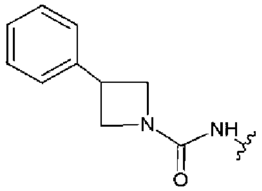

in terms of inhibition of proton pump activity.

In addition, when $R_1$ is

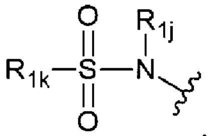,

first, $R_{1j}$ may be hydrogen, or substituted or unsubstituted C1 to C30 alkyl, and it is preferable that $R_{1j}$ is hydrogen, or substituted or unsubstituted C1 to C12 alkyl, but is not limited thereto. For example, $R_{1j}$ may be hydrogen. On the other hand, $R_{1k}$ may be hydrogen, substituted or unsubstituted C1 to C30 alkyl, substituted or unsubstituted C1 to C30 heteroalkyl, substituted or unsubstituted C2 to C30 alkenyl, substituted or unsubstituted C3 to C30 cycloalkyl, substituted or unsubstituted C6 to C30 aryl, substituted or unsubstituted C7 to C30 arylalkyl, substituted or unsubstituted C2 to C30 heteroaryl, or substituted or unsubstituted C3 to C30 heteroarylalkyl. Preferably, $R_{1d}$ may be hydrogen, substituted or unsubstituted C1 to C12 alkyl, substituted or unsubstituted C1 to C12 heteroalkyl, substituted or unsubstituted C3 to C12 cycloalkyl, substituted or unsubstituted C6 to C12 aryl, substituted or unsubstituted C7 to C12 arylalkyl, substituted or unsubstituted C2 to C12 heteroaryl, or substituted or unsubstituted C3 to C12 heteroarylalkyl. For example, $R_{1k}$ may be cyclobutyl.

Specifically, in chemical formula 1, when $R_2$ is $-NR_{2a}R_{2b}$, then $R_{2a}$ may be hydrogen. Meanwhile, $R_{2b}$ may be substituted or unsubstituted C7 to C30 arylalkyl, and is preferably substituted or unsubstituted C7 to C12 arylalkyl, but is not limited thereto. For example, $R_{2b}$ may be 2,6-dimethyl benzyl or 4-fluoro-2,6-dimethyl benzyl.

Specifically, in chemical formula 1, $R_3$ or $R_4$ may independently be hydrogen, or substituted or unsubstituted C1 to C30 alkyl, and it is preferable that $R_3$ or $R_4$ independently be hydrogen, or substituted or unsubstituted C1 to C12 alkyl, but is not limited thereto. For example, $R_3$ or $R_4$ may independently be methyl.

The compound may be at least one selected from the group consisting of

(1) N-(8-((2,6-dimethylbenzyl)amino)-2,3-dimethylimidazo[1,2-a]pyridin-6-yl)cyclopentanecarboxamide;
(2) N-(8-((2,6-dimethylbenzyl)amino)-2,3-dimethylimidazo[1,2-a]pyridin-6-yl)cyclopropanecarboxamide;
(3) N-(8-((2,6-dimethylbenzyl)amino)-2,3-dimethylimidazo[1,2-a]pyridin-6-yl)cyclohexanecarboxamide;
(4) N-(8-((2,6-dimethylbenzyl)amino)-2,3-dimethylimidazo[1,2-a]pyridin-6-yl)acetamide;
(5) N-(8-((2,6-dimethylbenzyl)amino)-2,3-dimethylimidazo[1,2-a]pyridin-6-yl)cyclobutanecarboxamide;
(6) N-(8-((2,6-dimethylbenzyl)amino)-2,3-dimethylimidazo[1,2-a]pyridin-6-yl)azetidine-1-carboxamide;
(7) N-(8-((2,6-dimethylbenzyl)amino)-2,3-dimethylimidazo[1,2-a]pyridin-6-yl)isobutyramide;
(8) N-(8-((2,6-dimethylbenzyl)amino)-2,3-dimethylimidazo[1,2-a]pyridin-6-yl)pivalamide;
(9) N-(8-((2,6-dimethylbenzyl)amino)-2,3-dimethylimidazo[1,2-a]pyridin-6-yl)-2,2-difluoroacetamide;
(10) 2-amino-N-(8-((2,6-dimethylbenzyl)amino)-2,3-dimethylimidazo[1,2-a]pyridin-6-yl)acetamide;
(11) N-(8-((2,6-dimethylbenzyl)amino)-2,3-dimethylimidazo[1,2-a]pyridin-6-yl)pyrrolidine-1-carboxamide;
(12) N-(8-((2,6-dimethylbenzyl)amino)-2,3-dimethylimidazo[1,2-a]pyridin-6-yl)bicyclo[1.1.1]pentane-1-carboxamide;
(13) N-(8-((2,6-dimethylbenzyl)amino)-2,3-dimethylimidazo[1,2-a]pyridin-6-yl)propionamide;
(14) N-(8-((2,6-dimethylbenzyl)amino)-2,3-dimethylimidazo[1,2-a]pyridin-6-yl)oxazole-2-carboxamide;
(15) N-(8-((2,6-dimethylbenzyl)amino)-2,3-dimethylimidazo[1,2-a]pyridin-6-yl)-2-methoxyacetamide;
(16) N-(8-((2,6-dimethylbenzyl)amino)-2,3-dimethylimidazo[1,2-a]pyridin-6-yl)-2,2-difluoropropanamide;
(17) N-(8-((2,6-dimethylbenzyl)amino)-2,3-dimethylimidazo[1,2-a]pyridin-6-yl)-3-fluorocyclobutane-1-carboxamide;
(18) N-(8-((2,6-dimethylbenzyl)amino)-2,3-dimethylimidazo[1,2-a]pyridin-6-yl)cyclobutanesulfonamide;
(19) (S)-2-amino-N-(8-((2,6-dimethylbenzyl)amino)-2,3-dimethylimidazo[1,2-a]pyridin-6-yl)propanamide;
(20) (R)-2-amino-N-(8-((2,6-dimethylbenzyl)amino)-2,3-dimethylimidazo[1,2-a]pyridin-6-yl)propanamide;
(21) N-(8-((4-fluoro-2,6-dimethylbenzyl)amino)-2,3-dimethylimidazo[1,2-a]pyridin-6-yl)acetamide;
(22) 1-(8-((2,6-dimethylbenzyl)amino)-2,3-dimethylimidazo[1,2-a]pyridin-6-yl)-3-ethylurea;

(23) 1-cyclobutyl-3-(8-((2,6-dimethylbenzyl)amino)-2,3-dimethylimidazo[1,2-a]pyridin-6-yl)urea;
(24) 1-cyclopropyl-3-(8-((2,6-dimethylbenzyl)amino)-2,3-dimethylimidazo[1,2-a]pyridin-6-yl)urea;
(25) 2-((8-((2,6-dimethylbenzyl)amino)-2,3-dimethylimidazo[1,2-a]pyridin-6-yl)amino)-2-oxoethyl acetate;
(26) N-(8-((2,6-dimethylbenzyl)amino)-2,3-dimethylimidazo[1,2-a]pyridin-6-yl)-2-hydroxyacetamide;
(27) 2-amino-N-(8-((2,6-dimethylbenzyl)amino)-2,3-dimethylimidazo[1,2-a]pyridin-6-yl)-2-methylpropanamide;
(28) 1-(8-((2,6-dimethylbenzyl)amino)-2,3-dimethylimidazo[1,2-a]pyridin-6-yl)azetidin-2-one;
(29) 1-(bicyclo[1.1.1]pentan-1-yl)-3-(8-((2,6-dimethylbenzyl)amino)-2,3-dimethylimidazo[1,2-a]pyridin-6-yl)urea;
(30) 1-(cyclopropylmethyl)-3-(8-((2,6-dimethylbenzyl)amino)-2,3-dimethylimidazo[1,2-a]pyridin-6-yl)urea;
(31) 1-(8-((2,6-dimethylbenzyl)amino)-2,3-dimethylimidazo[1,2-a]pyridin-6-yl)-3-isopropylurea;
(32) 3-(8-((2,6-dimethylbenzyl)amino)-2,3-dimethylimidazo[1,2-a]pyridin-6-yl)-1-ethyl-1-methylurea;
(33) 3-(8-((2,6-dimethylbenzyl)amino)-2,3-dimethylimidazo[1,2-a]pyridin-6-yl)-1-ethyl-1-methylurea;
(34) 3-((8-((2,6-dimethylbenzyl)amino)-2,3-dimethylimidazo[1,2-a]pyridin-6-yl)amino)-3-oxopropanoate;
(35) (S)-N-(8-((2,6-dimethylbenzyl)amino)-2,3-dimethylimidazo[1,2-a]pyridin-6-yl)azetidine-2-carboxamide;
(36) (R)-N-(8-((2,6-dimethylbenzyl)amino)-2,3-dimethylimidazo[1,2-a]pyridin-6-yl)azetidine-2-carboxamide;
(37) 1-(2-aminoethyl)-3-(8-((2,6-dimethylbenzyl)amino)-2,3-dimethylimidazo[1,2-a]pyridin-6-yl)urea;
(38) 4-((8-((2,6-dimethylbenzyl)amino)-2,3-dimethylimidazo[1,2-a]pyridin-6-yl)amino)-4-oxobutanoic acid;
(39) 3-(3-((8-((2,6-dimethylbenzyl)amino)-2,3-dimethylimidazo[1,2-a]pyridin-6-yl)amino)-3-oxopropyl)propanoic acid;
(40) ethyl 4-((8-((2,6-dimethylbenzyl)amino)-2,3-dimethylimidazo[1,2-a]pyridin-6-yl)amino)-4-oxobutanoate;
(41) tert-butyl 3-(3-((8-((2,6-dimethylbenzyl)amino)-2,3-dimethylimidazo[1,2-a]pyridin-6-yl)amino)-3-oxopropyl)propanoate;
(42) tert-butyl (2-(3-(8-((2,6-dimethylbenzyl)amino)-2,3-dimethylimidazo[1,2-a]pyridin-6-yl)ureido)ethyl)carbamate;
(43) 3-(8-((2,6-dimethylbenzyl)amino)-2,3-dimethylimidazo[1,2-a]pyridin-6-yl)-1,1-dimethylurea;
(44) 1-(8-((4-fluoro-2,6-dimethylbenzyl)amino)-2,3-dimethylimidazo[1,2-a]pyridin-6-yl)-3-methylurea;
(45) N-(8-((4-fluoro-2,6-dimethylbenzyl)amino)-2,3-dimethylimidazo[1,2-a]pyridin-6-yl)acetamide;
(46) methyl ((8-((2,6-dimethylbenzyl)amino)-2,3-dimethylimidazo[1,2-a]pyridin-6-yl)carbamoyl)glycinate;
(47) 2-(3-(8-((2,6-dimethylbenzyl)amino)-2,3-dimethylimidazo[1,2-a]pyridin-6-yl)ureido)-N-methylacetamide;
(48) 2-(3-(8-((2,6-dimethylbenzyl)amino)-2,3-dimethylimidazo[1,2-a]pyridin-6-yl)ureido)-N,N-dimethylacetamide;
(49) 3-(8-((4-fluoro-2,6-dimethylbenzyl)amino)-2,3-dimethylimidazo[1,2-a]pyridin-6-yl)-1,1-dimethylurea;
(50) 1-cyclopropyl-3-(8-((4-fluoro-2,6-dimethylbenzyl)amino)-2,3-dimethylimidazo[1,2-a]pyridin-6-yl)urea;
(51) N-(8-((4-fluoro-2,6-dimethylbenzyl)amino)-2,3-dimethylimidazo[1,2-a]pyridin-6-yl)azetidine-1-carboxamide;
(52) 2-acetamido-N-(8-((2,6-dimethylbenzyl)amino)-2,3-dimethylimidazo[1,2-a]pyridin-6-yl)acetamide;
(53) 2-(dimethylamino)-N-(8-((2,6-dimethylbenzyl)amino)-2,3-dimethylimidazo[1,2-a]pyridin-6-yl)acetamide;
(54) 1-butyl-3-(8-((2,6-dimethylbenzyl)amino)-2,3-dimethylimidazo[1,2-a]pyridin-6-yl)urea;
(55) N-(2-((8-((2,6-dimethylbenzyl)amino)-2,3-dimethylimidazo[1,2-a]pyridin-6-yl)amino)-2-oxoethyl)pivalamide;
(56) N-(2-((8-((2,6-dimethylbenzyl)amino)-2,3-dimethylimidazo[1,2-a]pyridin-6-yl)amino)-2-oxoethyl)-2,2-difluoroacetamide;
(57) N-(2-((8-((2,6-dimethylbenzyl)amino)-2,3-dimethylimidazo[1,2-a]pyridin-6-yl)amino)-2-oxoethyl)-2,2-difluoropropanamide;
(58) N-(2-((8-((2,6-dimethylbenzyl)amino)-2,3-dimethylimidazo[1,2-a]pyridin-6-yl)amino)-2-oxoethyl)-3-hydroxycyclobutane-1-carboxamide;
(59) N-(2-((8-((2,6-dimethylbenzyl)amino)-2,3-dimethylimidazo[1,2-a]pyridin-6-yl)amino)-2-oxoethyl)-3-fluorocyclobutane-1-carboxamide;
(60) 3-(8-((2,6-dimethylbenzyl)amino)-2,3-dimethylimidazo[1,2-a]pyridin-6-yl)imidazolidine-2,4-dione;
(61) N-(8-((2,6-dimethylbenzyl)amino)-2,3-dimethylimadizo[1,2-a]pyridin-6-yl)morpholine-4-carboxamide;
(62) 2-(3-(8-((2,6-dimethylbenzyl)amino)-2,3-dimethylimidazo[1,2-a]pyridin-6-yl)ureido)ethyl acetate;
(63) (S)-2-amino-N-(8-((4-fluoro-2,6-dimethylbenzyl)amino)-2,3-dimethylimidazo[1,2-a]pyridin-6-yl)propanamide;
(64) 2-amino-N-(8-((4-fluoro-2,6-dimethylbenzyl)amino)-2,3-dimethylimidazo[1,2-a]pyridin-6-yl)acetamide;
(65) (S)-N-(8-((4-fluoro-2,6-dimethylbenzyl)amino)-2,3-dimethylimidazo[1,2-a]pyridin-6-yl)azetidine-2-carboxamide;
(66) N-(8-((2,6-dimethylbenzyl)amino)-2,3-dimethylimidazo[1,2-a]pyridin-6-yl)-2-azaspiro[3.3]heptane-2-carboxamide;
(67) N-(8-((2,6-dimethylbenzyl)amino)-2,3-dimethylimidazo[1,2-a]pyridin-6-yl)-2-azaspiro[3.3]heptane-2-carboxamide;
(68) N-(8-((2,6-dimethylbenzyl)amino)-2,3-dimethylimidazo[1,2-a]pyridin-6-yl)-1,3-dihydro-2H-pyrrolo[3,4-c]pyridine-2-carboxamide;
(69) N-(8-((2,6-dimethylbenzyl)amino)-2,3-dimethylimidazo[1,2-a]pyridin-6-yl)-3-oxoazetidine-1-carboxamide;
(70) ((8-((2,6-dimethylbenzyl)amino)-2,3-dimethylimidazo[1,2-a]pyridin-6-yl)carbamoyl)glycine;

(71) 3-(3-(8-((2,6-dimethylbenzyl)amino)-2,3-dimethylimidazo[1,2-a]pyridin-6-yl)ureido)propionic acid;
(72) 3-(8-((2,6-dimethylbenzyl)amino)-2,3-dimethylimidazo[1,2-a]pyridin-6-yl)-1,3,5-triazepane-2,4-dione;
(73) N-(2-(3-(8-((2,6-dimethylbenzyl)amino)-2,3-dimethylimidazo[1,2-a]pyridin-6-yl)ureido)ethyl)acetamide;
(74) 1-(8-((2,6-dimethylbenzyl)amino)-2,3-dimethylimidazo[1,2-a]pyridin-6-yl)-3-((tetrohydrofuran-3-yl)methyl) urea;
(75) (R)-N-(8-((2,6-dimethylbenzyl)amino)-2,3-dimethylimidazo[1,2-a]pyridin-6-yl)-2-methylazetidine-2-carboxamide;
(76) (R)-2-(azetidin-2-yl)-N-(8-((2,6-dimethylbenzyl)amino)-2,3-dimethylimidazo[1,2-a]pyridin-6-yl)acetamide;
(77) N-(8-((2,6-dimethylbenzyl)amino)-2,3-dimethylimidazo[1,2-a]pyridin-6-yl)-3-phenylazetidine-1-carboxamide;
(78) (S)-1-(2,3-dihydro-1H-inden-1-yl)-3-(8-((2,6-dimethylbenzyl)amino)-2,3-dimethylimidazo[1,2-a]pyridin-6-yl) urea;
(79) (S)-N2-(8-((2,6-dimethylbenzyl)amino)-2,3-dimethylimidazo[1,2-a]pyridin-6-yl)-N1-methylazetidine-1,2-dicarboxamide;
(80) (R)-1-(2,3-dihydro-1H-inden-1-yl)-3-(8-((2,6-dimethylbenzyl)amino)-2,3-dimethylimidazo[1,2-a]pyridin-6-yl) urea;
(81) N-(8-((2,6-dimethylbenzyl)amino)-2,3-dimethylimidazo[1,2-a]pyridin-6-yl)-2-(2-oxoazetidin-1-yl)acetamide;
(82) N-(8-((2,6-dimethylbenzyl)amino)-2,3-dimethylimidazo[1,2-a]pyridin-6-yl)-4-phenylpiperidine-1-carboxamide;
(83) N-(8-((2,6-dimethylbenzyl-1)amino)-2,3-dimethylimidazo[1,2-a]pyridin-6-yl)-3-phenylpiperidine-1-carboxamide;
(84) N-(8-((2,6-dimethylbenzyl)amino)-2,3-dimethylimidazo[1,2-a]pyridin-6-yl)-3-phenylpyrrolidine-1-carboxamide;
(85) (R)-N-(8-((2,6-dimethylbenzyl)amino)-2,3-dimethylimidazo[1,2-a]pyridin-6-yl)piperazine-2-carboxamide;
(86) N-(8-((4-fluoro-2,6-dimethylbenzyl)amino)-2,3-dimethylimidazo[1,2-a]pyridin-6-yl)-2-hydroxyacetamide;
(87) 1-(2-aminoethyl)-3-(8-((2,6-dimethylbenzyl)amino)-2,3-dimethylimidazo[1,2-a]pyridin-6-yl)-1-methylurea;
(88) 1-(8-((2,6-dimethylbenzyl)amino)-2,3-dimethylimidazo[1,2-a]pyridin-6-yl)-3-(2-hydroxyethyl)urea;
(89) (R)-N-(8-((2,6-dimethylbenzyl)amino)-2,3-dimethylimidazo[1,2-a]pyridin-6-yl)pyrrolidine-3-carboxamide;
(90) (R)-N-(8-((2,6-dimethylbenzyl)amino)-2,3-dimethylimidazo[1,2-a]pyridin-6-yl)piperidine-2-carboxamide;
(91) (R)-N-(8-((2,6-dimethylbenzyl)amino)-2,3-dimethylimidazo[1,2-a]pyridin-6-yl)-2-(pyrrolidin-2-yl)acetamide;
(92) $N^8$-(2,6-dimethylbenzyl)-N6-isopropyl-2,3-dimethylimidazo[1,2-a]pyridin-6,8-diamine;
(93) (S)-N-(8-((2,6-dimethylbenzyl)amino)-2,3-dimethylimidazo[1,2-a]pyridin-6-yl)-2-(pyrrolidin-2-yl)acetamide;
(94) (S)-N-(8-((2,6-dimethylbenzyl)amino)-2,3-dimethylimidazo[1,2-a]pyridin-6-yl)piperazine-2-carboxamide;
(95) N-(8-((2,6-dimethylbenzyl)amino)-2,3-dimethylimidazo[1,2-a]pyridin-6-yl)-2-(2-hydroxyacetamido)acetamide;
(96) (S)-N-(8-((2,6-dimethylbenzyl)amino)-2,3-dimethylimidazo[1,2-a]pyridin-6-yl)-2-hydroxypropanamide;
(97) (R)-N-(8-((2,6-dimethylbenzyl)amino)-2,3-dimethylimidazo[1,2-a]pyridin-6-yl)-2-hydroxypropanamide;
(98) N-(8-((2,6-dimethylbenzyl)amino)-2,3-dimethylimidazo[1,2-a]pyridin-6-yl)-2-(methylamino)acetamide;
(99) 3-amino-N-(8-((2,6-dimethylbenzyl)amino)-2,3-dimethylimidazo[1,2-a]pyridin-6-yl)propanamide;
(100) N-(8-((4-fluoro-2,6-dimethylbenzyl)amino)-2,3-dimethylimidazo[1,2-a]pyridin-6-yl)-2-methoxyacetamide;
(101) N-(8-((4-fluoro-2,6-dimethylbenzyl)amino)-2,3-dimethylimidazo[1,2-a]pyridin-6-yl)-3-hydroxypropanamide;
(102) N-(8-((2,6-dimethylbenzyl)amino)-2,3-dimethylimidazo[1,2-a]pyridin-6-yl)-3-hydroxypropanamide;
(103) N-(8-((2,6-dimethylbenzyl)amino)-2,3-dimethylimidazo[1,2-a]pyridin-6-yl)piperazine-1-carboxamide;
(104) N-(8-((2,6-dimethylbenzyl)amino)-2,3-dimethylimidazo[1,2-a]pyridin-6-yl)benzamide;
(105) (S)-N-(8-((2,6-dimethylbenzyl)amino)-2,3-dimethylimidazo[1,2-a]pyridin-6-yl)pyrrolidine-3-carboxamide;
(106) (S)-N-(8-((2,6-dimethylbenzyl)amino)-2,3-dimethylimidazo[1,2-a]pyridin-6-yl)piperidine-2-carboxamide;
(107) (E)-4-(3-((8-((2,6-dimethylbenzyl)amino)-2,3-dimethylimidazo[1,2-a]pyridin-6-yl)amino)-3-oxoprop-1-en-1-yl) phenyl acetate;
(108) (E)-4-(3-((8-((2,6-dimethylbenzyl)amino)-2,3-dimethylimidazo[1,2-a]pyridin-6-yl)amino)-3-oxoprop-1-en-1-yl)-2-methoxyphenyl acetate;
(109) (S)-N-(8-((2,6-dimethylbenzyl)amino)-2,3-dimethylimidazo[1,2-a]pyridin-6-yl)aziridine-2-carboxamide;
(110) (S)-N-(8-((2,6-dimethylbenzyl)amino)-2,3-dimethylimidazo[1,2-a]pyridin-6-yl)-1-(2-hydroxyacetyl)pyrrolidine-3-carboxamide;
(111) (R)-1-((8-((4-fluoro-2,6-dimethylbenzyl)amino)-2,3-dimethylimidazo[1,2-a]pyridin-6-yl)amino)-1-oxopropan-2-yl acetate;
(112) (S)-1-((8-((4-fluoro-2,6-dimethylbenzyl)amino)-2,3-dimethylimidazo[1,2-a]pyridin-6-yl)amino)-1-oxopropan-2-yl acetate;
(113) 1-((8-((4-fluoro-2,6-dimethylbenzyl)amino)-2,3-dimethylimidazo[1,2-a]pyridin-6-yl)carbamoyl)cyclopropyl acetate;
(114) (S)-N-(8-((4-fluoro-2,6-dimethylbenzyl)amino)-2,3-dimethylimidazo[1,2-a]pyridin-6-yl)-2-hydroxypropanamide;
(115) N-(8-((4-fluoro-2,6-dimethylbenzyl)amino)-2,3-dimethylimidazo[1,2-a]pyridin-6-yl)-1-hydroxycyclopropane-1-carboxamide;
(116) 3-(8-((4-fluoro-2,6-dimethylbenzyl)amino)-2,3-dimethylimidazo[1,2-a]pyridin-6-yl)-1-methylimidazolidine-2,4-dione;
(117) (R)-N-(8-((4-fluoro-2,6-dimethylbenzyl)amino)-2,3-dimethylimidazo[1,2-a]pyridin-6-yl)-2-hydroxypropanamide;
(118) (S)-N-(8-((2,6-dimethylbenzyl)amino)-2,3-dimethylimidazo[1,2-a]pyridin-6-yl)piperidine-3-carboxamide;
(119) N-(8-((2,6-dimethylbenzyl)amino)-2,3-dimethylimidazo[1,2-a]pyridin-6-yl)-3-(2-hydroxyacetamido)propanamide;
(120) (S)-N-(8-((2,6-dimethylbenzyl)amino)-2,3-dimethylimidazo[1,2-a]pyridin-6-yl)-1-(2-hydroxyacetyl)piperidine-3-carboxamide;
(121) $N^8$-(2,6-dimethylbenzyl)-2,3-dimethylimidazo[1,2-a]pyridine-6,8-diamine; and
(122) $N^8$-(4-fluoro-2,6-dimethylbenzyl)-2,3-dimethylimidazo[1,2-a]pyridine-6,8-diamine.

Preferably, the compound may be at least one selected from the group consisting of

(4) N-(8-((2,6-dimethylbenzyl)amino)-2,3-dimethylimidazo[1,2-a]pyridin-6-yl)acetamide;
(10) 2-amino-N-(8-((2,6-dimethylbenzyl)amino)-2,3-dimethylimidazo[1,2-a]pyridin-6-yl)acetamide;
(15) N-(8-((2,6-dimethylbenzyl)amino)-2,3-dimethylimidazo[1,2-a]pyridin-6-yl)-2-methoxyacetamide;
(21) N-(8-((4-fluoro-2,6-dimethylbenzyl)amino)-2,3-dimethylimidazo[1,2-a]pyridin-6-yl)acetamide;
(24) 1-cyclopropyl-3-(8-((2,6-dimethylbenzyl)amino)-2,3-dimethylimidazo[1,2-a]pyridin-6-yl)urea;
(26) N-(8-((2,6-dimethylbenzyl)amino)-2,3-dimethylimidazo[1,2-a]pyridin-6-yl)-2-hydroxyacetamide;
(33) 3-(8-((2,6-dimethylbenzyl)amino)-2,3-dimethylimidazo[1,2-a]pyridin-6-yl)-1-ethyl-1-methylurea;
(43) 3-(8-((2,6-dimethylbenzyl)amino)-2,3-dimethylimidazo[1,2-a]pyridin-6-yl)-1,1-dimethylurea;
(44) 1-(8-((4-fluoro-2,6-dimethylbenzyl)amino)-2,3-dimethylimidazo[1,2-a]pyridin-6-yl)-3-methylurea;
(45) N-(8-((4-fluoro-2,6-dimethylbenzyl)amino)-2,3-dimethylimidazo[1,2-a]pyridin-6-yl)acetamide;
(49) 3-(8-((4-fluoro-2,6-dimethylbenzyl)amino)-2,3-dimethylimidazo[1,2-a]pyridin-6-yl)-1,1-dimethylurea;
(50) 1-cyclopropyl-3-(8-((4-fluoro-2,6-dimethylbenzyl)amino)-2,3-dimethylimidazo[1,2-a]pyridin-6-yl)urea;
(51) N-(8-((4-fluoro-2,6-dimethylbenzyl)amino)-2,3-dimethylimidazo[1,2-a]pyridin-6-yl)azetidine-1-carboxamide;
(60) 3-(8-((2,6-dimethylbenzyl)amino)-2,3-dimethylimidazo[1,2-a]pyridin-6-yl)imidazolidine-2,4-dione;
(64) 2-amino-N-(8-((4-fluoro-2,6-dimethylbenzyl)amino)-2,3-dimethylimidazo[1,2-a]pyridin-6-yl)acetamide;
(77) N-(8-((2,6-dimethylbenzyl)amino)-2,3-dimethylimidazo[1,2-a]pyridin-6-yl)-3-phenylazetidine-1-carboxamide;
(86) N-(8-((4-fluoro-2,6-dimethylbenzyl)amino)-2,3-dimethylimidazo[1,2-a]pyridin-6-yl)-2-hydroxyacetamide;
(87) 1-(2-aminoethyl)-3-(8-((2,6-dimethylbenzyl)amino)-2,3-dimethylimidazo[1,2-a]pyridin-6-yl)-1-methylurea;
(88) 1-(8-((2,6-dimethylbenzyl)amino)-2,3-dimethylimidazo[1,2-a]pyridin-6-yl)-3-(2-hydroxyethyl)urea;
(93) (S)-N-(8-((2,6-dimethylbenzyl)amino)-2,3-dimethylimidazo[1,2-a]pyridin-6-yl)-2-(pyrrolidin-2-yl)acetamide;
(94) (S)-N-(8-((2,6-dimethylbenzyl)amino)-2,3-dimethylimidazo[1,2-a]pyridin-6-yl)piperazine-2-carboxamide;
(95) N-(8-((2,6-dimethylbenzyl)amino)-2,3-dimethylimidazo[1,2-a]pyridin-6-yl)-2-(2-hydroxyacetamido)acetamide;
(100) N-(8-((4-fluoro-2,6-dimethylbenzyl)amino)-2,3-dimethylimidazo[1,2-a]pyridin-6-yl)-2-methoxyacetamide;
(101) N-(8-((4-fluoro-2,6-dimethylbenzyl)amino)-2,3-dimethylimidazo[1,2-a]pyridin-6-yl)-3-hydroxypropanamide;
(102) N-(8-((2,6-dimethylbenzyl)amino)-2,3-dimethylimidazo[1,2-a]pyridin-6-yl)-3-hydroxypropanamide;
(115) N-(8-((4-fluoro-2,6-dimethylbenzyl)amino)-2,3-dimethylimidazo[1,2-a]pyridin-6-yl)-1-hydroxycyclopropane-1-carboxamide; and
(117) (R)-N-(8-((4-fluoro-2,6-dimethylbenzyl)amino)-2,3-dimethylimidazo[1,2-a]pyridin-6-yl)-2-hydroxypropanamide.

More preferably, the compound may be at least one selected from the group consisting of

(4) N-(8-((2,6-dimethylbenzyl)amino)-2,3-dimethylimidazo[1,2-a]pyridin-6-yl)acetamide;
(15) N-(8-((2,6-dimethylbenzyl)amino)-2,3-dimethylimidazo[1,2-a]pyridin-6-yl)-2-methoxyacetamide;
(21) N-(8-((4-fluoro-2,6-dimethylbenzyl)amino)-2,3-dimethylimidazo[1,2-a]pyridin-6-yl)acetamide;
(26) N-(8-((2,6-dimethylbenzyl)amino)-2,3-dimethylimidazo[1,2-a]pyridin-6-yl)-2-hydroxyacetamide;
(45) N-(8-((4-fluoro-2,6-dimethylbenzyl)amino)-2,3-dimethylimidazo[1,2-a]pyridin-6-yl)acetamide;
(60) 3-(8-((2,6-dimethylbenzyl)amino)-2,3-dimethylimidazo[1,2-a]pyridin-6-yl)imidazolidine-2,4-dione;
(86) N-(8-((4-fluoro-2,6-dimethylbenzyl)amino)-2,3-dimethylimidazo[1,2-a]pyridin-6-yl)-2-hydroxyacetamide;
(100) N-(8-((4-fluoro-2,6-dimethylbenzyl)amino)-2,3-dimethylimidazo[1,2-a]pyridin-6-yl)-2-methoxyacetamide;
(101) N-(8-((4-fluoro-2,6-dimethylbenzyl)amino)-2,3-dimethylimidazo[1,2-a]pyridin-6-yl)-3-hydroxypropanamide; and
(102) N-(8-((2,6-dimethylbenzyl)amino)-2,3-dimethylimidazo[1,2-a]pyridin-6-yl)-3-hydroxypropanamide.

The compound or its pharmaceutically acceptable salt exhibits inhibitory activity against proton pump activity, as confirmed by measuring its inhibitory activity against proton pump ($H^+$, $K^+$-ATPase) activity. Therefore, it can act as a reversible proton pump inhibitor and effectively suppress gastric acid secretion, thereby providing the advantage of being useful in the prevention, improvement, or treatment of gastrointestinal inflammatory diseases or gastric acid-related diseases.

## Pharmaceutical Composition for Preventing or Treating Gastrointestinal Inflammatory Diseases or Gastric Acid-Related Diseases

The present invention provides a pharmaceutical composition for preventing or treating gastrointestinal inflammatory diseases or gastric acid-related diseases, comprising the imidazopyridine compound or a pharmaceutically acceptable salt thereof as an active ingredient.

The specific structure of the compound or its pharmaceutically acceptable salt has been described above, so a detailed description will be omitted.

The compound or its pharmaceutically acceptable salt can act as a reversible proton pump inhibitor. These reversible proton pump inhibitors (PPIs) improve upon the shortcomings of PPIs. They reversibly and competitively bind to the $K^+$ ion of proton pump ($H^+/K^+$ ATPase), an enzyme involved in the final stage of gastric acid secretion in gastric parietal cells, thereby potently and rapidly inhibiting gastric acid secretion. Furthermore, because they are primarily metabolized by the CYP3A4 enzyme, interindividual differences in efficacy are relatively small, and concerns about interactions with drugs metabolized by the CYP2C19 enzyme are relatively low. Furthermore, they are easy to take, regardless of whether they are taken before or after meals, and are highly effective in alleviating nighttime symptoms.

Additionally, the compound or a pharmaceutically acceptable salt thereof can suppress gastric acid secretion.

Therefore, the compound or a pharmaceutically acceptable salt thereof can be used for the prevention or treatment of gastrointestinal inflammatory diseases or gastric acid-related diseases. The gastrointestinal inflammatory diseases or gastric acid-related diseases may be at least one selected from the group consisting of peptic ulcers, gastric/duodenal ulcers, nonsteroidal anti-inflammatory drug (NSAID)-induced ulcers, Helicobacter pylori infection, functional dyspepsia, Zollinger-Ellison syndrome, gastritis, gastroesophageal reflux disease (GERD), and non-erosive reflux disease (NERD).

The pharmaceutical composition according to the present invention can be formulated and used in the form of oral preparations such as powders, granules, tablets, capsules, suspensions, emulsions, syrups, aerosols; external preparations; suppositories; and sterile injection solutions, according to conventional methods, and may include appropriate carriers, excipients, or diluents commonly used in the manufacture of pharmaceutical compositions for formulation.

The carrier, excipient, or diluent may include various compounds or mixtures including lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate and mineral oil.

When formulated, they can be manufactured using commonly used diluents or excipients, such as fillers, bulking agents, binders, wetting agents, disintegrants, and surfactants.

Solid dosage forms for oral administration can be manufactured by mixing the imidazopyridine compound or its pharmaceutically acceptable salt with at least one excipient, such as starch, calcium carbonate, sucrose or lactose, or gelatin. In addition to simple excipients, lubricants such as magnesium stearate and talc can also be used.

Liquid dosage forms for oral administration include suspensions, oral solutions, emulsions, and syrups. In addition to commonly used simple diluents such as water and liquid paraffin, they can also include various excipients, such as wetting agents, sweeteners, flavoring agents, and preservatives.

Formulations for parenteral administration include sterile aqueous solutions, non-aqueous solutions, suspensions, emulsions, lyophilized preparations, and suppositories. Non-aqueous solutions and suspensions include propylene glycol, polyethylene glycol, vegetable oils such as olive oil, and injectable esters such as ethyl oleate. Suppository bases include witepsol, macrogol, Tween 61, cacao butter, laurin butter, and glycerol gelatin.

The preferred dosage of the pharmaceutical composition according to the present invention varies depending on the patient's condition, weight, severity of the disease, drug form, route of administration, and duration of administration, but can be appropriately selected by those skilled in the art. However, for optimal efficacy, the daily dosage ranges from 0.0001 to 2,000 mg/kg, preferably from 0.001 to 2,000 mg/kg. Administration may be administered once daily or in multiple divided doses. However, the scope of the present invention is not limited to the above dosage.

The pharmaceutical composition according to the present invention can be administered to mammals such as rats, mice, livestock, and humans via various routes. All modes of administration can be administered, for example, orally, rectally, or by intravenous, intramuscular, subcutaneous, intrauterine, or intracerebroventricular injection.

Health Functional Food Composition for Preventing or Ameliorating Gastrointestinal Inflammatory Diseases or Gastric Acid-Related Diseases

In another embodiment of the present invention, a health functional food composition for preventing or ameliorating gastrointestinal inflammatory diseases or gastric acid-related diseases is provided, comprising the imidazopyridine compound or a pharmaceutically acceptable salt thereof as an active ingredient.

The specific structure and action of the compound or its pharmaceutically acceptable salt have been described above, and therefore, a detailed description thereof will be omitted.

The gastrointestinal inflammatory disease or gastric acid-related disease may be one or more selected from the group consisting of peptic ulcer, gastric/duodenal ulcer, nonsteroidal anti-inflammatory drug (NSAID)-induced ulcer, Helicobacter pylori infection, functional dyspepsia, Zollinger-Ellison syndrome, gastritis, gastroesophageal reflux disease (GERD), and non-erosive reflux disease (NERD).

In the food or health functional food composition according to the present invention, when the imidazopyridine compound or its pharmaceutically acceptable salt is used as an additive in a health functional food, it can be added directly or combined with other foods or food ingredients, and can be used appropriately according to conventional methods. The amount of active ingredient can be appropriately determined depending on the intended use, such as prevention, health, or treatment.

The health functional food can be in the form of powders, granules, pills, tablets, capsules, or any other form of general food or beverage.

There are no specific restrictions on the type of food. Examples of foods to which the substance can be added include meat, sausage, bread, chocolate, candy, snacks, confectionery, pizza, ramen, other noodles, gum, dairy products including ice cream, various soups, beverages, tea, drinks, alcoholic beverages, and vitamin complexes. All foods in the conventional sense can be included.

Generally, when manufacturing food or beverages, the imidazopyridine compound or its pharmaceutically acceptable salt may be added in an amount of 15 parts by weight or less, preferably 10 parts by weight or less, per 100 parts by weight of the raw material. However, for long-term consumption for health and hygiene purposes or for health control, the amount may be less than the above range.

Among the health functional foods according to the present invention, beverages may contain various flavoring agents or natural carbohydrates as additional ingredients, similar to conventional beverages. The natural carbohydrates may include monosaccharides such as glucose and fructose, disaccharides such as maltose and sucrose, polysaccharides such as dextrin and cyclodextrin, and sugar alcohols such as xylitol, sorbitol, and erythritol. Sweeteners may include natural sweeteners such as thaumatin and stevia extract, or synthetic sweeteners such as saccharin and aspartame. The proportion of the above natural carbohydrates may be about 0.01 to 0.04 g, preferably about 0.02 to 0.03 g, per 100 mL of the beverage according to the present invention.

In addition to the above, the health functional food composition according to the present invention may contain various nutrients, vitamins, electrolytes, flavoring agents, coloring agents, pectic acid and its salts, alginic acid and its salts, organic acids, protective colloid thickeners, pH adjusters, stabilizers, preservatives, glycerin, alcohol, and carbonating agents used in carbonated beverages. In addition, the health functional food composition according to the present invention may contain fruit pulp for the production of natural fruit juice, fruit juice drinks, and vegetable drinks. These ingredients may be used independently or in mixtures. The ratio of these additives is not limited, but is generally selected in the range of 0.01 to 0.1 parts by weight relative to 100 parts by weight of the health functional food composition according to the present invention.

Alternatively, the present invention provides a use of the imidazopyridine compound or a pharmaceutically acceptable salt thereof for preparing a composition comprising the compound or a salt thereof as an active ingredient for preventing, treating, or ameliorating gastrointestinal inflammatory diseases or gastric acid-related diseases.

Alternatively, the present invention provides a method for preventing, treating, or ameliorating gastrointestinal inflammatory diseases or gastric acid-related diseases, comprising administering the imidazopyridine compound or a pharmaceutically acceptable salt thereof to a subject. Herein, the term "subject" refers to a subject requiring treatment for a disease, and more specifically, a mammal such as a human or non-human primate, mouse, rat, dog, cat, horse, or cow.

Hereinafter, preferred examples are presented to facilitate understanding of the present invention. However, the following examples are provided solely to facilitate understanding of the present invention and are not intended to limit the scope of the present invention.

**<Examples>**

**[Preliminary Example 1] Preparation of $N^8$-(2,6-dimethylbenzyl)-2,3-dimethylimidazo[1,2-a]pyridine-6,8-diamine (Intermediate 1)**

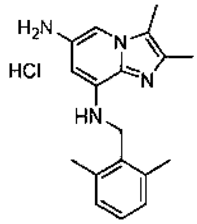

Step 1: Preparation of 6-bromo-2,3-dimethylimidazo[1,2-a]pyridin-8-amine

5-Bromopyridine-2,3-diamine (20 g, 106 mmol) was added to tetrahydrofuran (212 mL), followed by 3-bromo-2-butanone (12.9 mL, 127 mmol) at room temperature. The mixture was stirred at 100°C for 2 hours. After completion of the reaction, the mixture was cooled to room temperature. The precipitated solid was filtered and dried to obtain the target compound (21.4 g, 84.4%).

Step 2: Preparation of 6-bromo-N-(2,6-dimethylbenzyl)-2,3-dimethylimidazo[1,2-a]pyridin-8-amine

The compound prepared in Step 1 (10 g, 41.84 mmol) was dissolved in isopropyl alcohol (418 mL). Potassium carbonate (14.4 g, 104.6 mmol) and sodium iodide (6.2 g, 41.8 mmol) were added, and the mixture was stirred at room temperature. 2-(chloromethyl)-1,3-dimethylbenzene (7 g, 46 mmol) was then added, and the mixture was stirred at 70°C for 3 hours. Upon completion of the reaction, the mixture was cooled to room temperature, dichloromethane and distilled water were added, and the layers were separated. The organic layer was recovered, dried over anhydrous magnesium sulfate, and filtered. The filtrate was concentrated under reduced pressure and purified by column chromatography to obtain the target compound (9.6 g, 64.2%).

Step 3: Preparation of N-(2,6-dimethylbenzyl)-6-((diphenylmethylene)amino)-2,3-dimethylimidazo[1,2-a]pyridin-8-amine

The compound prepared in Step 2 (9.5 g, 26.6 mmol) was dissolved in toluene (133 mL). Diphenylmethanimine (5.7 g, 31.9 mmol), tris(dibenzylideneacetone)dipalladium (2.4 g, 2.66 mmol), (S)-2,2'-bis(diphenylphosphino)-1,1'-binaphthyl (3.3 g, 5.32 mmol), and sodium tert-butoxide (7.6 g, 79.8 mmol) were added and stirred at 80°C for 12 hours. After the reaction was completed, it was cooled to room temperature, and dichloromethane and distilled water were added to separate the layers. The organic layer was recovered, dried over anhydrous magnesium sulfate, and filtered. The filtrate was concentrated under reduced pressure and purified by column chromatography to obtain the target compound (8.4 g, 68.9%).

Step 4: Preparation of $N^8$-(2,6-dimethylbenzyl)-2,3-dimethylimidazo[1,2-alpyridine-6,8-diamine hydrochloride

The compound prepared in Step 3 (8.4 g, 18.3 mmol) was dissolved in ethyl acetate (91.5 mL). 4 N hydrochloric acid (36.6 mL, 0.5 M) dissolved in 1,4-dioxane was added and stirred at room temperature for 12 hours. Upon completion of the reaction, the residue was filtered and dried to obtain the target compound (5.8 g, 96%).
$^1$H NMR (500 MHz, DMSO-$d_6$) δ 7.20-7.11 (m, 4H), 6.58 (s, 1H), 6.21 (s, 1H), 4.28 (s, 2H), 2.36 (s, 3H), 2.34 (s, 6H), 2.33 (s, 3H)

**[Preliminary Example 2] Preparation of $N^8$-(4-fluoro-2,6-dimethylbenzyl)-2,3-dimethylimidazo[1,2-a]pyridine-6,8-diamine hydrochloride (Intermediate 2)**

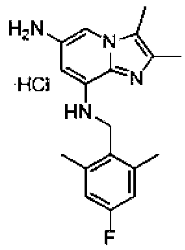

Step 1: Preparation of 6-bromo-N-(4-fluoro-2,6-dimethylbenzyl)-2,3-dimethylimidazo[1,2-a]pyridin-8-amine

The compound prepared in Step 1 of Preliminary Example 1 (2.0 g, 8.36 mmol) was dissolved in acetonitrile (83.6 mL). Potassium carbonate (3.4 g, 25.08 mmol) and sodium iodide (1.2 g, 8.36 mmol) were added, and the mixture was heated to 70°C with stirring. 2-(bromomethyl)-5-fluoro-1,3-dimethylbenzene (2.16 g, 10.03 mmol) was added, and the mixture was stirred at 70°C for 3 hours. Upon completion of the reaction, the mixture was cooled to room temperature, dichloromethane and distilled water were added, and the layers were separated. The organic layer was recovered, dried over anhydrous magnesium sulfate, and filtered. The filtrate was concentrated under reduced pressure and purified by column chromatography to obtain the target compound (1.6 g, 50%).

Step 2: Preparation of 6-((diphenylmethylene)amino)-N-(4-fluoro-2,6-dimethylbenzyl)-2,3-dimethylimidazo[1,2-a]pyridin-8-amine

The compound prepared in Step 1 (1.4 g, 3.72 mmol) was dissolved in toluene (19 mL). Diphenylmethanimine (808 mg, 4.46 mmol), tris(dibenzylideneacetone)dipalladium (338 mg, 0.37 mmol), (S)-2,2'-bis(diphenylphosphino)-1,1'-binaphthyl (462 mg, 0.74 mmol), and sodium tert-butoxide (1.07 g, 11.16 mmol) were added and stirred at 80°C for 12 hours. After the reaction was completed, it was cooled to room temperature, and dichloromethane and distilled water were added to separate the layers. The organic layer was recovered, dried over anhydrous magnesium sulfate, and filtered. The filtrate was concentrated under reduced pressure and purified by column chromatography to obtain the target compound (1.5 g, 84%).

Step 3: Preparation of $N^8$-(4-fluoro-2,6-dimethylbenzyl)-2,3-dimethylimidazo[1,2-a]pyridine-6,8-diamine hydrochloride

The compound prepared in Step 2 (1.5 g, 3.15 mmol) was dissolved in ethyl acetate (15.7 mL). 4 N hydrochloric acid (6.3 mL, 0.5 M) dissolved in 1,4-dioxane was added and stirred at room temperature for 12 hours. Upon completion of the reaction, the residue was filtered and dried to obtain the target compound (1.0 g, 77%).
$^1$H NMR (500 MHz, DMSO-$d_6$) $\delta$ 13.16 (s, 1H), 7.24-7.14 (d, 1H), 7.07-6.96 (m, 2H), 6.53 (s, 1H), 5.99-5.98 (m, 1H), 4.24-4.23 (d, 2H), 2.40-2.32 (m, 12H)

**[Example 1] Preparation of N-(8-((2,6-dimethylbenzyl)amino)-2,3-dimethylimidazo[1,2-a]pyridin-6-yl)cyclopentanecarboxamide**

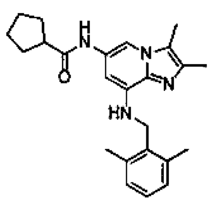

$N^8$-(2,6-Dimethylbenzyl)-2,3-dimethylimidazo[1,2-a]pyridine-6,8-diamine hydrochloride (Intermediate 1, 40.0 mg, 0.12 mmol) was dissolved in dichloromethane. 1-[Bis(dimethylamino)methylene]-1H-1,2,3-triazolo[4,5-B]pyridinium 3-oxide hexafluorophosphate (59.0 mg, 0.15 mmol), diisopropylethylamine (0.06 mL, 0.36 mmol), and cyclopentanecarboxylic acid (14.0 mg, 0.13 mmol) were added, and the mixture was stirred at room temperature for 6 h. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (10 mg, 21%).

$^1H$ NMR (500 MHz, $CDCl_3$) δ 8.25 (s, 1H), δ 7.13-7.10 (t, 1H), 7.05-7.02 (m, 3H), 5.90 (s, 1H), 4.84 (s, 1H), 4.31-4.30 (d, 2H), 2.74-2.70 (m, 1H), 2.37 (s, 3H), 2.33 (s, 6H), 2.31 (s, 3H), 2.04-1.97 (m, 4H), 1.84-1.81 (m, 2H), 1.67-1.64 (m, 2H)

**[Example 2] Preparation of N-(8-((2,6-dimethylbenzyl)amino)-2,3-dimethylimidazo[1,2-a]pyridin-6-yl)cyclopropanecarboxamide**

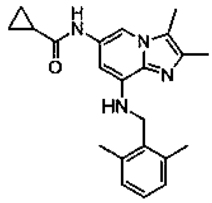

$N^8$-(2,6-Dimethylbenzyl)-2,3-dimethylimidazo[1,2-a]pyridine-6,8-diamine hydrochloride (Intermediate 1, 40.0 mg, 0.12 mmol) was dissolved in dichloromethane. Triethylamine (0.05 mL, 0.36 mmol) and cyclopropanecarbonyl chloride (12.0 mg, 0.12 mmol) were added and stirred at room temperature for 6 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (15 mg, 34%).
$^1H$ NMR (500 MHz, $CDCl_3$) δ 8.17 (s, 1H), δ 7.37 (s, 1H), 7.13-7.10 (t, 1H), 5.95 (s, 1H), 4.88 (s, 1H), 4.30-4.29 (d, 2H), 2.36 (s, 6H), 2.31 (s, 6H), δ 1.54 (s, 1H), 1.11-1.10 (m, 2H), 0.88-0.86 (m, 2H)

**[Example 3] Preparation of N-(8-((2,6-dimethylbenzyl)amino)-2,3-dimethylimidazo[1,2-a]pyridin-6-yl)cyclohexanecarboxamide**

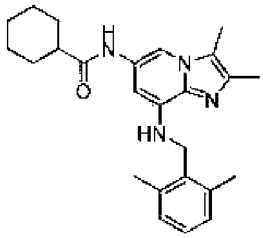

$N^8$-(2,6-dimethylbenzyl)-2,3-dimethylimidazo[1,2-a]pyridine-6,8-diamine hydrochloride (Intermediate 1, 40.0 mg, 0.12 mmol) was dissolved in dichloromethane. Triethylamine (0.05 mL, 0.36 mmol) and cyclohexanecarbonyl chloride (12.0 mg, 0.12 mmol) were added, and the mixture was stirred at room temperature for 6 hours. Upon completion of the reaction, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (13 mg, 26%).
$^1H$ NMR (500 MHz, $CDCl_3$) δ 8.25 (s, 1H), 7.13-7.10 (m, 1H), 7.07-7.03 (m, 3H), 5.89 (s, 1H), 4.84 (s, 1H), 4.30-4.29 (d, 2H), 2.36 (s, 3H), 2.33 (s, 6H), 2.31 (s, 3H), 2.27-2.26 (m, 2H), 2.04-2.01 (m, 2H), 1.98-1.87 (m, 2H), 1.74-1.72 (m, 2H), 1.59-1.57 (m, 2H), 1.35-1.30 (m, 2H)

**[Example 4] Preparation of N-(8-((2,6-dimethylbenzyl)amino)-2,3-dimethylimidazo[1,2-a]pyridin-6-yl)acetamide**

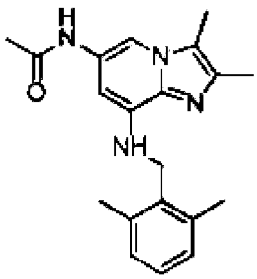

$N^8$-(2,6-Dimethylbenzyl)-2,3-dimethylimidazo[1,2-a]pyridine-6,8-diamine hydrochloride (Intermediate 1, 50.0 mg, 0.15 mmol) was dissolved in dichloromethane. Triethylamine (0.03 mL, 0.22 mmol) and acetic anhydride (0.01 mL, 0.16 mmol) were added and stirred at room temperature for 6 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (10 mg, 19%).
$^1$H NMR (500 MHz, $CDCl_3$) δ 8.14 (s, 1H), 7.12-7.10 (m, 1H), 7.04-7.03 (m, 3H), 5.91 (s, 1H), 4.88 (s, 1H), 4.30-4.29 (d, 2H), 2.36-2.32 (m, 12H), δ 2.22 (s, 3H)

**[Example 5] Preparation of N-(8-((2,6-dimethylbenzyl)amino)-2,3-dimethylimidazo[1,2-a]pyridin-6-yl)cyclobutanecarboxamide**

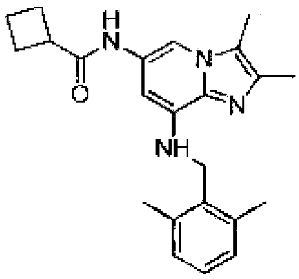

$N^8$-(2,6-Dimethylbenzyl)-2,3-dimethylimidazo[1,2-a]pyridine-6,8-diamine hydrochloride (Intermediate 1, 40.0 mg, 0.12 mmol) was dissolved in dichloromethane. Triethylamine (0.05 mL, 0.36 mmol) and cyclobutanecarbonyl chloride (14.0 mg, 0.12 mmol) were added, and the mixture was stirred at room temperature for 6 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (11 mg, 24%).
$^1$H NMR (500 MHz, $CDCl_3$) δ 8.26 (s, 1H), δ 7.13-7.10 (m, 1H), 7.05-7.03 (d, 2H), 6.90 (s, 1H), 5.90 (s, 1H), 4.88 (s, 1H), 4.30 (s, 2H), 3.21-3.18 (m, 1H), 2.45-2.43 (m, 2H), 2.39-2.34 (m, 12H), 2.28-2.26 (m, 2H)

**[Example 6] Preparation of N-(8-((2,6-dimethylbenzyl)amino)-2,3-dimethylimidazo[1,2-a]pyridin-6-yl)azetidine-1-carboxamide**

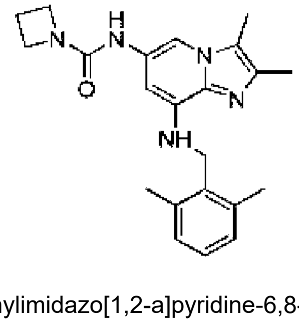

$N^8$-(2,6-Dimethylbenzyl)-2,3-dimethylimidazo[1,2-a]pyridine-6,8-diamine hydrochloride (Intermediate 1, 50.0

mg, 0.15 mmol) was dissolved in dichloromethane. 1,1'-Carbonyldiimidazole (24.0 mg, 0.15 mmol), triethylamine (0.04 mL, 0.3 mmol), and azetidine (0.01 mL, 0.15 mmol) were added, and the mixture was stirred at room temperature for 16 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (6 mg, 10%).
$^1$H NMR (500 MHz, $CDCl_3$) δ 7.94 (s, 1H), δ 7.11-7.10 (m, 2H), 7.04-7.02 (m, 2H), 5.96-5.90 (m, 1H), 5.87-5.77 (m, 1H), 4.30-4.29 (d, 2H), 4.17-4.07 (m, 6H), 2.40-2.36 (m, 6H), 2.34-2.31 (m, 6H)

**[Example 7] Preparation of N-(8-((2,6-dimethylbenzyl)amino)-2,3-dimethylimidazo[1,2-a]pyridin-6-yl)isobutyramide**

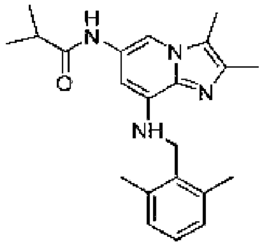

N$^8$-(2,6-Dimethylbenzyl)-2,3-dimethylimidazo[1,2-a]pyridine-6,8-diamine hydrochloride (Intermediate 1, 50.0 mg, 0.15 mmol) was dissolved in dichloromethane. Triethylamine (0.08 mL, 0.60 mmol) and isobutyryl chloride (16.0 mg, 0.16 mmol) were added and stirred at room temperature for 6 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (10 mg, 18%).
$^1$H NMR (500 MHz, $CDCl_3$) δ 8.29 (s, 1H), 7.13-7.02 (m, 4H), 5.95 (s, 1H), 4.83 (s, 1H), 4.30 (d, 2H), 2.36-2.33 (m, 12H), 1.30-1.25 (m, 6H)

**[Example 8] Preparation of N-(8-((2,6-dimethylbenzyl)amino)-2,3-dimethylimidazo[1,2-a]pyridin-6-yl)pivalamide**

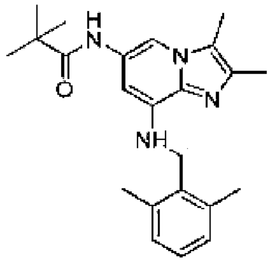

N$^8$-(2,6-Dimethylbenzyl)-2,3-dimethylimidazo[1,2-a]pyridine-6,8-diamine hydrochloride (Intermediate 1, 50.0 mg, 0.15 mmol) was dissolved in dichloromethane. 1-[Bis(dimethylamino)methylene]-1H-1,2,3-triazolo[4,5-B]pyridinium 3-oxide hexafluorophosphate (85.0 mg, 0.22 mmol), diisopropylethylamine (0.1 mL, 0.60 mmol), and pivalic acid (16.0 mg, 0.16 mmol) were added, and the mixture was stirred at room temperature for 6 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (8 mg, 14%).
$^1$H NMR (500 MHz, $CDCl_3$) δ 8.59 (s, 1H), 7.85 (s, 1H), 6.74 (s, 2H), 6.67 (s, 2H), 4.30-4.29 (d, 2H), 2.39-2.22 (m, 9H), 1.40-1.39 (m, 12H)

**[Example 9] Preparation of N-(8-((2,6-dimethylbenzyl)amino)-2,3-dimethylimidazo [1,2-a] pyridin-6-yl)-2,2-difluoroacetamide**

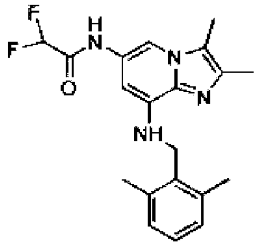

$N^8$-(2,6-Dimethylbenzyl)-2,3-dimethylimidazo[1,2-a]pyridine-6,8-diamine hydrochloride (Intermediate 1, 50.0 mg, 0.15 mmol) was dissolved in dichloromethane. 1-[Bis(dimethylamino)methylene]-1H-1,2,3-triazolo[4,5-B]pyridinium 3-oxide hexafluorophosphate (85.0 mg, 0.22 mmol), diisopropylethylamine (0.1 mL, 0.60 mmol), and 2,2-difluoroacetic acid (15.0 mg, 0.16 mmol) were added, and the mixture was stirred at room temperature for 6 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (10 mg, 17%).
$^1$H NMR (500 MHz, $CDCl_3$) δ 8.13 (s, 1H), 7.82-7.78 (m, 1H), 7.06-7.01 (m, 1H), 6.99-6.95 (m, 2H), 6.11-5.89 (m, 2H), 4.25-4.24 (d, 2H), 2.29-2.27 (m, 12H), 1.18 (s, 1H)

**[Example 10] Preparation of 2-amino-N-(8-((2,6-dimethylbenzyl)amino)-2,3-dimethylimidazo[1,2-a]pyridin-6-yl) acetamide hydrochloride**

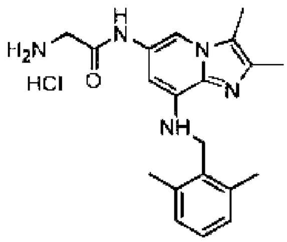

Step 1: Preparation of tert-butyl (2-((8-((2,6-dimethylbenzyl)amino)-2,3-dimethylimidazo[1,2-a]pyridin-6-yl)amino)-2-oxoethyl)carbamate

$N^8$-(2,6-dimethylbenzyl)-2,3-dimethylimidazo[1,2-a]pyridine-6,8-diamine hydrochloride (Intermediate 1, 50.0 mg, 0.15 mmol) was dissolved in dichloromethane. 1-[Bis(dimethylamino)methylene]-1H-1,2,3-triazolo[4,5-B]pyridinium 3-oxide hexafluorophosphate (85.0 mg, 0.22 mmol), diisopropylethylamine (0.1 mL, 0.60 mmol), and (tert-butoxycarbonyl)glycine (28.0 mg, 0.16 mmol) were added and stirred at room temperature for 6 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (35 mg, 85%).

Step 2: Preparation of 2-amino-N-(8-((2,6-dimethylbenzyl)amino)-2,3-dimethylimidazo[1,2-a]pyridin-6-yl)acetamide hydrochloride

tert-Butyl (2-((8-((2,6-dimethylbenzyl)amino)-2,3-dimethylimidazo[1,2-a]pyridin-6-yl)amino)-2-oxoethyl)carbamate (30.0 mg, 0.06 mmol) was dissolved in ethyl acetate. 4 N hydrochloric acid (0.16 mL, 0.4 M) dissolved in 1,4-dioxane was added and stirred at room temperature for 6 hours. Upon completion of the reaction, the mixture was filtered to obtain the target compound (9 mg, 38%).
$^1$H NMR (500 MHz, DMSO-$d_6$) δ 11.21 (s, 1H), 8.42 (s, 1H), 8.26 (m, 3H), 7.22-7.13 (m, 3H), 6.96 (s, 1H), 6.47 (s, 1H), 4.31-4.30 (d, 2H), 3.89-3.88 (d, 2H), 2.42-2.36 (m, 12H)

**[Example 11] Preparation of N-(8-((2,6-dimethylbenzyl)amino)-2,3-dimethylimidazo[1,2-a]pyridin-6-yl)pyrrolidine-1-carboxamide**

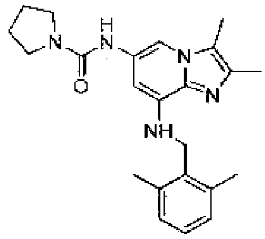

$N^8$-(2,6-Dimethylbenzyl)-2,3-dimethylimidazo[1,2-a]pyridine-6,8-diamine hydrochloride (Intermediate 1, 40.0 mg, 0.12 mmol) was dissolved in dichloromethane. 1,1'-Carbonyldiimidazole (19.0 mg, 0.12 mmol), triethylamine (0.06 mL, 0.24 mmol), and pyrrolidine (17 mg, 0.24 mmol) were added and stirred at room temperature for 16 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (6 mg, 12%).
$^1H$ NMR (500 MHz, $CDCl_3$) $\delta$ 7.14-7.12 (m, 2H), 7.08-7.07 (m, 2H), 7.02-7.01(m, 2H), 4.95-4.94 (d, 2H), 2.45-2.43 (m, 9H), 2.39-2.38 (m, 3H), 2.19-2.03 (m, 8H)

**[Example 12] Preparation of N-(8-((2,6-dimethylbenzyl)amino)-2,3-dimethylimidazo[1,2-a]pyridin-6-yl)bicyclo[1.1.1]pentane-1-carboxamide**

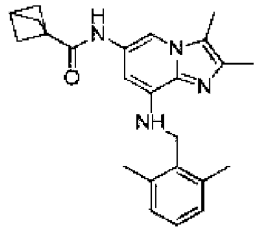

$N^8$-(2,6-Dimethylbenzyl)-2,3-dimethylimidazo[1,2-a]pyridine-6,8-diamine hydrochloride (Intermediate 1, 40.0 mg, 0.12 mmol) was dissolved in dichloromethane. 1-[Bis(dimethylamino)methylene]-1H-1,2,3-triazolo[4,5-B]pyridinium 3-oxide hexafluorophosphate (68.0 mg, 0.18 mmol), diisopropylethylamine (0.06 mL, 0.48 mmol), and bicyclo[1.1.1] pentane-1-carboxylic acid (14.0 mg, 0.13 mmol) were added and stirred at room temperature for 6 hours. When the reaction was complete, dichloromethane (50 mL) and distilled water (50 mL) were added, the layers were separated, the organic layer was recovered, dried over anhydrous magnesium sulfate, and filtered. The filtrate was concentrated under reduced pressure and purified by column chromatography to obtain the target compound (8 mg, 17%).
$^1H$ NMR (500 MHz, $CDCl_3$) $\delta$ 8.27 (s, 1H), 7.16-7.07 (m, 1H), 7.06-7.03 (m, 3H), 5.95 (s, 1H), 4.85 (s, 1H), 4.35-4.34 (d, 2H), 2.39-2.35 (m, 12H), 2.21 (m, 7H)

**[Example 13] Preparation of N-(8-((2,6-dimethylbenzyl)amino)-2,3-dimethylimidazo[1,2-a]pyridin-6-yl)propionamide**

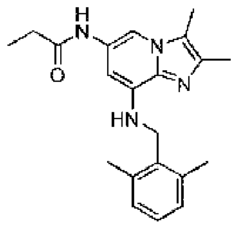

$N^8$-(2,6-Dimethylbenzyl)-2,3-dimethylimidazo[1,2-a]pyridine-6,8-diamine hydrochloride (Intermediate 1, 20.0 mg, 0.06 mmol) was dissolved in dichloromethane. Triethylamine (0.02 mL, 0.18 mmol) and propionyl chloride (5.0 mg, 0.06 mmol) were added, and the mixture was stirred at room temperature for 6 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (6 mg, 28%).
$^1$H NMR (500 MHz, $CDCl_3$) δ 8.22 (s, 1H), 7.14-7.12 (m, 2H), 7.05-7.04 (m, 1H), 5.91 (s, 1H), 4.85 (s, 1H), 4.32-4.31 (d, 2H), 2.47-2.42 (m, 2H), 2.34-2.32 (m, 12H), 1.31-1.28 (m, 3H)

**[Example 14] Preparation of N-(8-((2,6-dimethylbenzyl)amino)-2,3-dimethylimidazo[1,2-a]pyridin-6-yl)oxazole-2-carboxamide**

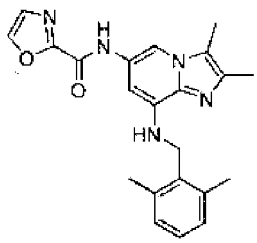

$N^8$-(2,6-Dimethylbenzyl)-2,3-dimethylimidazo[1,2-a]pyridine-6,8-diamine hydrochloride (Intermediate 1, 40.0 mg, 0.12 mmol) was dissolved in dichloromethane. 1-Ethyl-3-(3-dimethylaminopropyl)carbodiimide (34.0 mg, 0.15 mmol), 1-hydroxybenzotriazole hydrate (24.0 mg, 0.15 mmol), triethylamine (0.06 mL, 0.48 mmol), and oxazole-2-carboxylic acid (14.0 mg, 0.13 mmol) were added, and the mixture was stirred at room temperature for 6 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (6 mg, 12%).
$^1$H NMR (500 MHz, DMSO-$d_6$) δ 10.77 (s, 1H), 8.44 (s, 1H), 8.28 (s, 1H), 7.58 (s, 1H), 7.17-7.08 (m, 3H), 6.74 (s, 1H), 5.04-5.02 (m, 1H), 4.33-4.32 (d, 2H), 2.36-2.23 (m, 12H)

**[Example 15] Preparation of N-(8-((2,6-dimethylbenzyl)amino)-2,3-dimethylimidazo[1,2-a]pyridin-6-yl)-2-methoxyacetamide**

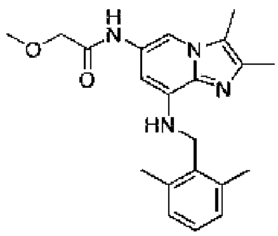

$N^8$-(2,6-Dimethylbenzyl)-2,3-dimethylimidazo[1,2-a]pyridine-6,8-diamine hydrochloride (Intermediate 1, 40.0 mg, 0.12 mmol) was dissolved in dichloromethane. Triethylamine (0.04 mL, 0.30 mmol) and 2-methoxyacetyl chloride (14.0 mg, 0.13 mmol) were added, and the mixture was stirred at room temperature for 6 hours. When the reaction was complete, dichloromethane (50 mL) and distilled water (50 mL) were added, the layers were separated, and the organic

layer was recovered, dried over anhydrous magnesium sulfate, and filtered. The filtrate was concentrated under reduced pressure and purified by column chromatography to obtain the target compound (9 mg, 20%).
$^1$H NMR (500 MHz, $CDCl_3$) δ 8.22 (s, 1H), 8.13 (s, 1H), 7.14-7.11 (m, 1H), 7.05-7.04 (m, 2H), 4.85 (s, 1H), 4.34-4.33 (d, 2H), 4.06 (s, 2H), 3.55 (s, 3H), 2.38-2.33 (m, 12H)

**[Example 16] Preparation of N-(8-((2,6-dimethylbenzyl)amino)-2,3-dimethylimidazo[1,2-a]pyridin-6-yl)-2,2-difluoropropanamide**

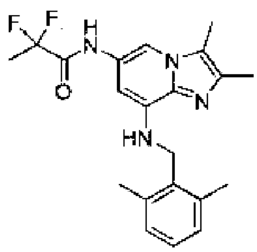

$N^8$-(2,6-Dimethylbenzyl)-2,3-dimethylimidazo[1,2-a]pyridine-6,8-diamine hydrochloride (Intermediate 1, 40.0 mg, 0.12 mmol) was dissolved in dichloromethane. 1-Ethyl-3-(3-dimethylaminopropyl)carbodiimide (34.0 mg, 0.15 mmol), 1-hydroxybenzotriazole hydrate (24.0 mg, 0.15 mmol), triethylamine (0.06 mL, 0.48 mmol), and 2,2-difluoropropanoic acid (14.0 mg, 0.13 mmol) were added and stirred at room temperature for 6 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (9 mg, 19%).
$^1$H NMR (500 MHz, $CDCl_3$) δ 8.19 (s, 1H), 7.85 (s, 1H), 7.15-7.12 (m, 1H), 7.06-7.04 (m, 2H), 4.99 (s, 1H), 4.93 (s, 1H), 4.34-4.33 (d, 2H), 2.38-2.33 (m, 12H), 1.98-1.90 (m, 3H)

**[Example 17] Preparation of N-(8-((2,6-dimethylbenzyl)amino)-2,3-dimethylimidazo[1,2-a]pyridin-6-yl)-3-fluorocyclobutane-1-carboxamide**

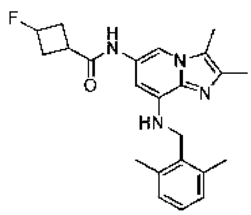

$N^8$-(2,6-Dimethylbenzyl)-2,3-dimethylimidazo[1,2-a]pyridine-6,8-diamine hydrochloride (Intermediate 1, 40.0 mg, 0.12 mmol) was dissolved in dichloromethane. 1-Ethyl-3-(3-dimethylaminopropyl)carbodiimide (34.0 mg, 0.15 mmol), 1-hydroxybenzotriazole hydrate (24.0 mg, 0.15 mmol), triethylamine (0.06 mL, 0.48 mmol), and 3-fluorocyclobutane-1-carboxylic acid (15.0 mg, 0.13 mmol) were added and stirred at room temperature for 6 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (10 mg, 21%).
$^1$H NMR (500 MHz, $CDCl_3$) δ 8.23 (s, 1H), 7.14-7.11 (m, 1H), 7.05-7.04 (m, 2H), 6.97 (s, 1H), 5.04-4.87 (m, 2H), 4.31-4.30 (d, 2H), 2.68-2.58 (m, 4H), 2.37-2.32 (m, 12H)

**[Example 18] Preparation of N-(8-((2,6-dimethylbenzyl)amino)-2,3-dimethylimidazo[1,2-a]pyridin-6-yl)cyclobutanesulfonamide**

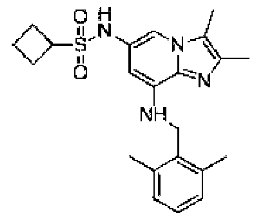

$N^8$-(2,6-Dimethylbenzyl)-2,3-dimethylimidazo[1,2-a]pyridine-6,8-diamine hydrochloride (Intermediate 1, 40.0 mg, 0.12 mmol) was dissolved in dichloromethane. Triethylamine (0.04 mL, 0.30 mmol) and cyclobutanesulfonyl chloride (20.0 mg, 0.13 mmol) were added, and the mixture was stirred at room temperature for 6 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (8 mg, 16%).
$^1$H NMR (500 MHz, $CDCl_3$) δ 7.16-7.13 (m, 2H), 7.08-7.05 (m, 4H), 5.92 (s, 1H), 5.03 (s, 1H), 4.38-4.36 (m, 2H), 3.42-3.40 (m, 1H), 2.42 (s, 6H), 2.40-2.43 (m, 4H), 1.66 (s, 3H)

**[Example 19] Preparation of (S)-2-amino-N-(8-((2,6-dimethylbenzyl)amino)-2,3-dimethylimidazo[1,2-a]pyridin-6-yl)propanamide hydrochloride**

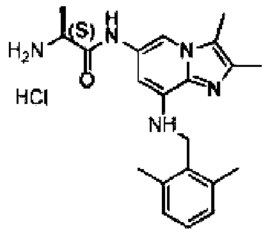

Step 1: Preparation of tert-butyl (S)-(1-((8-((2,6-dimethylbenzyl)amino)-2,3-dimethylimidazo[1,2-a]pyridin-6-yl)amino)-1-oxopropan-2-yl)carbamate

$N^8$-(2,6-dimethylbenzyl)-2,3-dimethylimidazo[1,2-a]pyridine-6,8-diamine hydrochloride (Intermediate 1, 40.0 mg, 0.12 mmol) was dissolved in dichloromethane. 1-[Bis(dimethylamino)methylene]-1H-1,2,3-triazolo[4,5-B]pyridinium 3-oxide hexafluorophosphate (59.0 mg, 0.15 mmol), diisopropylethylamine (0.08 mL, 0.48 mmol), and (tert-butoxycarbonyl)-L-alanine (24.0 mg, 0.13 mmol) were added and stirred at room temperature for 6 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (10 mg, 17%).

Step 2: Preparation of (S)-2-amino-N-(8-((2,6-dimethylbenzyl)amino)-2,3-dimethylimidazo[1,2-a]pyridin-6-yl)propanamide hydrochloride

Tert-butyl (S)-(1-((8-((2,6-dimethylbenzyl)amino)-2,3-dimethylimidazo[1,2-a]pyridin-6-yl)amino)-1-oxopropan-2-yl)carbamate (10.0 mg, 0.02 mmol), prepared in Step 1, was dissolved in ethyl acetate. 4 N hydrochloric acid (0.04 mL, 0.5 M) dissolved in 1,4-dioxane was added, and the mixture was stirred at room temperature for 6 hours. Upon completion of the reaction, the mixture was filtered to obtain the target compound (4 mg, 47%).
$^1$H NMR (500 MHz, DMSO-$d_6$) δ 13.89 (s, 1H), 11.31 (s, 1H), 8.47 (s, 1H), 8.38-8.37 (m, 3H), 4.31-4.30 (d, 2H), 4.17-4.14 (m, 1H), 2.51-2.41 (m, 15H)

**[Example 20] Preparation of (R)-2-amino-N-(8-((2,6-dimethylbenzyl)amino)-2,3-dimethylimidazo[1,2-a]pyridin-6-yl)propanamide hydrochloride**

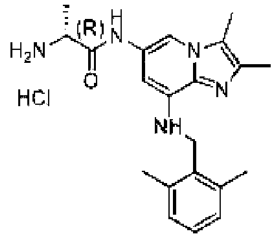

Step 1: Preparation of tert-butyl (R)-(1-((8-((2,6-dimethylbenzyl)amino)-2,3-dimethylimidazo[1,2-a]pyridin-6-yl)amino)-1-oxopropan-2-yl)carbamate

$N^8$-(2,6-dimethylbenzyl)-2,3-dimethylimidazo[1,2-a]pyridine-6,8-diamine hydrochloride (Intermediate 1, 40.0 mg, 0.12 mmol) was dissolved in dichloromethane. 1-[Bis(dimethylamino)methylene]-1H-1,2,3-triazolo[4,5-B]pyridinium 3-oxide hexafluorophosphate (59.0 mg, 0.15 mmol), diisopropylethylamine (0.08 mL, 0.48 mmol), and (tert-butoxycarbonyl)-D-alanine (24.0 mg, 0.13 mmol) were added and stirred at room temperature for 6 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (14 mg, 25%).

Step 2: Preparation of (R)-2-amino-N-(8-((2,6-dimethylbenzyl)amino)-2,3-dimethylimidazo[1,2-a]pyridin-6-yl)propanamide hydrochloride

Tert-butyl (R)-(1-((8-((2,6-dimethylbenzyl)amino)-2,3-dimethylimidazo[1,2-a]pyridin-6-yl)amino)-1-oxopropan-2-yl)carbamate (10.0 mg, 0.02 mmol), prepared in Step 1, was dissolved in ethyl acetate. 4 N hydrochloric acid (0.04 mL, 0.5 M) dissolved in 1,4-dioxane was added, and the mixture was stirred at room temperature for 6 hours. Upon completion of the reaction, the mixture was filtered to obtain the target compound (4 mg, 47%).
$^1$H NMR (500 MHz, DMSO-$d_6$) δ 13.86 (s, 1H), 11.29 (s, 1H), 8.46 (s, 1H), 8.37-8.36 (m, 3H), 7.23-7.21 (m, 1H), 7.16-7.14 (m, 2H), 7.05 (s, 1H), 6.45 (s, 1H), 4.31-4.30 (d, 2H), 4.16 (s, 1H), 2.51-2.37 (m, 15H)

**[Example 21] Preparation of N-(8-((4-fluoro-2,6-dimethylbenzyl)amino)-2,3-dimethylimidazo[1,2-a]pyridin-6-yl) acetamide**

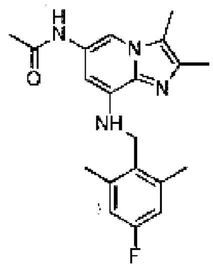

$N^8$-(4-Fluoro-2,6-dimethylbenzyl)-2,3-dimethylimidazo[1,2-a]pyridine-6,8-diamine hydrochloride (Intermediate 2, 47 mg, 0.13 mmol) and triethylamine (0.04 mL, 0.27 mmol) were dissolved in dimethylformamide (2 mL. Acetic anhydride (0.02 mL, 0.20 mmol) was added and stirred at room temperature for 2 hours. When the reaction was complete, a saturated aqueous sodium bicarbonate solution was added to precipitate a solid, which was filtered and dried to obtain the target compound (25 mg, 54%).
$^1$H NMR (500 MHz, DMSO-$d_6$) δ 9.86 (s, 1H), 8.17 (s, 1H), 6.98-6.94 (m, 3H), 6.20 (s, 1H), 5.09-5.08 (m, 1H), 4.49-4.48 (m, 1H), 4.25-4.25 (d, 2H), 2.36-2.21 (m, 12H), 2.07 (s, 3H)

**[Example 22] Preparation of 1-(8-((2,6-dimethylbenzyl)amino)-2,3-dimethylimidazo[1,2-a]pyridin-6-yl)-3-ethylurea**

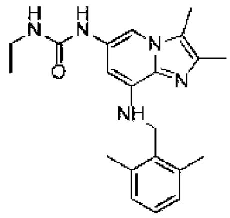

$N^8$-(2,6-Dimethylbenzyl)-2,3-dimethylimidazo[1,2-a]pyridine-6,8-diamine hydrochloride (Intermediate 1, 40.0 mg, 0.12 mmol) was dissolved in dichloromethane. 1,1'-Carbonyldiimidazole (19.0 mg, 0.12 mmol), triethylamine (0.06 mL, 0.24 mmol), and ethylamine diluted in methanol (16 mg, 0.36 mmol) were added and stirred at room temperature for 16 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (7 mg, 15%).
$^1$H NMR (500 MHz, $CDCl_3$) δ 7.16-7.13 (m, 2H), 7.08-7.03 (m, 4H), 6.02 (s, 1H), 4.30-4.28 (m, 2H), 3.33-3.30 (m, 2H), 2.34-2.22 (m, 12H), 1.18-1.15 (m, 3H)

**[Example 23] Preparation of 1-cyclobutyl-3-(8-((2,6-dimethylbenzyl)amino)-2,3-dimethylimidazo[1,2-a]pyridin-6-yl)urea hydrochloride**

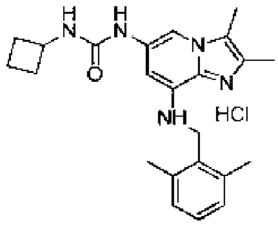

$N^8$-(2,6-Dimethylbenzyl)-2,3-dimethylimidazo[1,2-a]pyridine-6,8-diamine hydrochloride (intermediate 1, 40.0 mg, 0.12 mmol) was dissolved in dichloromethane. 1,1'-Carbonyldiimidazole (19.0 mg, 0.12 mmol), triethylamine (0.06 mL, 0.24 mmol), and cyclobutylamine (8 mg, 0.12 mmol) were added and stirred at room temperature for 16 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (19 mg, 40%). After dissolving in ethyl acetate and adding 4 N hydrochloric acid (0.04 mL, 0.5 M) dissolved in 1,4-dioxane, the mixture was stirred at room temperature for 12 hours. After the reaction was completed, the mixture was filtered to obtain the hydrochloride salt of the target compound.
$^1$H NMR (500 MHz, DMSO-$d_6$) δ 13.35 (s, 1H), 8.83 (s, 1H), 8.30 (s, 1H), 7.23-20 (m, 1H), 7.16-7.14 (m, 2H), 6.77-6.75 (m, 2H), 6.13 (s, 1H), 4.31-4.30 (d, 2H), 2.38-2.36 (m, 12H), 2.24-2.22 (m, 2H), 2.20-2.19 (m, 1H), 1.93-1.91 (m, 2H), 1.65-1.62 (m, 2H)

**[Example 24] Preparation of 1-cyclopropyl-3-(8-((2,6-dimethylbenzyl)amino)-2,3-dimethylimidazo[1,2-a]pyridin-6-yl)urea hydrochloride**

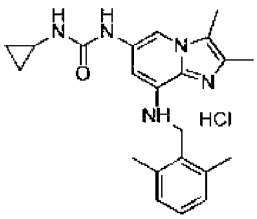

$N^8$-(2,6-Dimethylbenzyl)-2,3-dimethylimidazo[1,2-a]pyridine-6,8-diamine hydrochloride (Intermediate 1, 50.0 mg, 0.15 mmol) was dissolved in dichloromethane. 1,1'-Carbonyldiimidazole (24.0 mg, 0.15 mmol), triethylamine (0.04

mL, 0.3 mmol), and cyclopropylamine (8 mg, 0.15 mmol) were added and stirred at room temperature for 16 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (20 mg, 35%). 4 N hydrochloric acid (0.04 mL, 0.5 M) dissolved in ethyl acetate and 1,4-dioxane was added and stirred at room temperature for 12 hours. After the reaction was completed, the hydrochloride salt of the target compound was obtained by filtration.
$^1$H NMR (500 MHz, DMSO-$d_6$) δ 13.47 (s, 1H), 8.90 (s, 1H), 8.34 (s, 1H), 7.23-7.21 (m, 1H), 7.19-7.15 (m, 2H), 6.83-6.81 (m, 2H), 4.31-4.30 (d, 2H), 2.37-2.29 (m, 12H), 0.67-0.65 (m, 2H), 0.52-0.50 (m, 2H)

**[Example 25] Preparation of 2-((8-((2,6-dimethylbenzyl)amino)-2,3-dimethylimidazo[1,2-a]pyridin-6-yl)amino)-2-oxoethyl acetate**

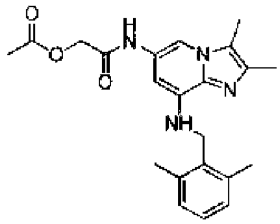

N$^8$-(2,6-Dimethylbenzyl)-2,3-dimethylimidazo[1,2-a]pyridine-6,8-diamine hydrochloride (Intermediate 1, 50.0 mg, 0.15 mmol) was dissolved in dichloromethane. Triethylamine (0.06 mL, 0.45 mmol) and 2-chloro-2-oxoethyl acetate (22.0 mg, 0.16 mmol) were added, and the mixture was stirred at room temperature for 6 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (10 mg, 16%).
$^1$H NMR (500 MHz, $CDCl_3$) δ 8.15 (s, 1H), 7.64 (s, 1H), 7.15-7.13 (m, 1H), 7.06-7.04 (m, 2H), 5.95 (s, 1H), 4.91 (s, 1H), 4.74 (s, 2H), 4.34-4.33 (d, 2H), 2.38-2.33 (m, 12H), 2.29 (s, 3H)

**[Example 26] Preparation of N-(8-((2,6-dimethylbenzyl)amino)-2,3-dimethylimidazo[1,2-a]pyridin-6-yl)-2-hydroxyacetamide**

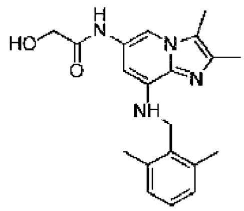

2-((8-((2,6-Dimethylbenzyl)amino)-2,3-dimethylimidazo[1,2-a]pyridin-6-yl)amino)-2-oxoethyl acetate (15.0 mg, 0.03 mmol) was dissolved in tetrahydrofuran (0.1 mL, 0.3 M), ethanol (0.1 mL, 0.3 M), and distilled water (0.1 mL, 0.3 M). Potassium hydroxide (6.0 mg, 0.11 mmol) was added and stirred at room temperature for 16 hours. After the reaction was completed, the pressure was reduced, the solvent was removed, dichloromethane (50 mL) and distilled water (50 mL) were added, the layers were separated, the organic layer was recovered, dried over anhydrous magnesium sulfate, and filtered. The filtrate was concentrated under reduced pressure and purified by column chromatography to obtain the target compound (5 mg, 47%).
$^1$H NMR (500 MHz, DMSO-$d_6$) δ 13.45 (s, 1H), 10.06 (s, 1H), 8.61 (s, 1H), 7.23-7.20 (m, 1H), 7.16-7.14 (m, 2H), 5.92 (s, 1H), 4.32-4.31 (d, 2H), 4.09-4.08 (d, 2H), 2.41-2.37 (m, 12H)

**[Example 27] Preparation of 2-amino-N-(8-((2,6-dimethylbenzyl)amino)-2,3-dimethylimidazo[1,2-a]pyridin-6-yl)-2-methylpropanamide hydrochloride**

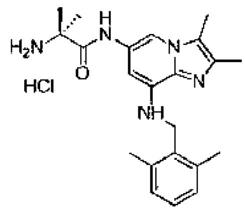

Step 1: Preparation of tert-butyl (1-((8-((2,6-dimethylbenzyl)amino)-2,3-dimethylimidazo[1,2-a]pyridin-6-yl)amino)-2-methyl-1-oxopropan-2-yl)carbamate

$N^8$-(2,6-dimethylbenzyl)-2,3-dimethylimidazo[1,2-a]pyridine-6,8-diamine hydrochloride (Intermediate 1, 45.0 mg, 0.13 mmol) was dissolved in dichloromethane. 1-[Bis(dimethylamino)methylene]-1H-1,2,3-triazolo[4,5-B]pyridinium 3-oxide hexafluorophosphate (64.0 mg, 0.16 mmol), diisopropylethylamine (0.09 mL, 0.52 mmol), and 2-((tert-butoxycarbonyl)amino)-2-methylpropanoic acid (30.0 mg, 0.14 mmol) were added and stirred at room temperature for 6 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (16 mg, 25%).

Step 2: Preparation of 2-amino-N-(8-((2,6-dimethylbenzyl)amino)-2,3-dimethylimidazo[1,2-a]pyridin-6-yl)-2-methylpropanamide hydrochloride

Tert-butyl (1-((8-((2,6-dimethylbenzyl)amino)-2,3-dimethylimidazo[1,2-a]pyridin-6-yl)amino)-2-methyl-1-oxopropan-2-yl)carbamate (15.0 mg, 0.03 mmol), prepared in Step 1, was dissolved in ethyl acetate. 4 N hydrochloric acid (0.06 mL, 0.5 M) dissolved in 1,4-dioxane was added, and the mixture was stirred at room temperature for 6 hours. Upon completion of the reaction, the mixture was filtered to obtain the target compound (10 mg, 77%).
$^1$H NMR (500 MHz, DMSO-$d_6$) δ 13.74 (s, 1H), 10.65 (s, 1H), 8.48-8.44 (m, 4H), 7.29-7.15 (m, 4H), 4.32-4.31 (d, 2H), 2.43-2.37 (m, 12H), 1.69 (s, 6H)

**[Example 28] Preparation of 1-(8-((2,6-dimethylbenzyl)amino)-2,3-dimethylimidazo[1,2-a]pyridin-6-yl)azetidin-2-one**

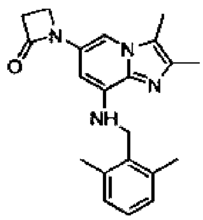

$N^8$-(2,6-Dimethylbenzyl)-2,3-dimethylimidazo[1,2-a]pyridine-6,8-diamine hydrochloride (Intermediate 1, 50 mg, 0.14 mmol) and triethylamine (0.06 mL, 0.43 mmol) were dissolved in tetrahydrofuran (2 mL) and dimethylformamide (1 mL), and the mixture was stirred at room temperature for 10 minutes. 3-Bromopropionyl chloride (0.16 mmol) was slowly added dropwise, and the mixture was stirred at room temperature for 2 hours. The reaction mixture was concentrated in vacuo, and the residue was purified by column chromatography to obtain the target compound (12 mg, 25%).
$^1$H NMR (500 MHz, $CDCl_3$) δ 7.14-7.11 (m, 2H), 7.05-7.04 (m, 2H), 5.70 (s, 1H), 4.90 (s, 1H), 4.31-4.30 (m, 2H), 3.57-3.54 (t, 2H), 2.78-2.75 (t, 2H), 2.63 (s, 3H), 2.38 (s, 6H), 2.30 (s, 3H)

**[Example 29] Preparation of 1-(bicyclo[1.1.1]pentan-1-yl)-3-(8-((2,6-dimethylbenzyl)amino)-2,3-dimethylimidazo[1,2-a]pyridin-6-yl)urea hydrochloride**

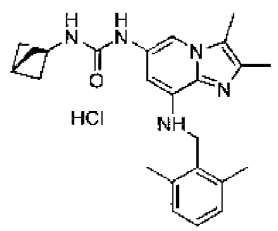

$N^8$-(2,6-Dimethylbenzyl)-2,3-dimethylimidazo[1,2-a]pyridine-6,8-diamine hydrochloride (Intermediate 1, 80.0 mg, 0.24 mmol) was dissolved in dichloromethane. 1,1'-Carbonyldiimidazole (38.0 mg, 0.24 mmol), triethylamine (0.06 mL, 0.48 mmol), and bicyclo[1.1.1]pentan-1-amine hydrochloride (28 mg, 0.24 mmol) were added and stirred at room temperature for 16 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (10 mg, 10%). After dissolving in ethyl acetate and adding 4 N hydrochloric acid (0.12 mL, 0.2 M) dissolved in 1,4-dioxane, the mixture was stirred at room temperature for 12 hours. After the reaction was completed, the mixture was filtered to obtain the hydrochloride salt of the target compound.
$^1$H NMR (500 MHz, DMSO-$d_6$) δ 8.96 (s, 1H), 8.30 (s, 1H), 7.22-7.19 (m, 2H), 7.15-7.11 (m, 2H), 6.71 (s, 1H), 6.19 (s, 1H), 4.31-4.30 (d, 2H), 2.43-2.36 (m, 14H), 2.02 (s, 6H)

**[Example 30] Preparation of 1-(cyclopropylmethyl)-3-(8-((2,6-dimethylbenzyl)amino)-2,3-dimethylimidazo[1,2-a]pyridin-6-yl)urea hydrochloride**

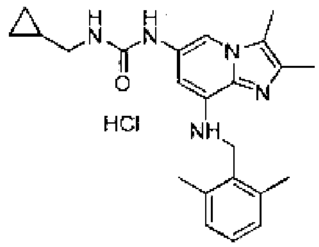

$N^8$-(2,6-Dimethylbenzyl)-2,3-dimethylimidazo[1,2-a]pyridine-6,8-diamine hydrochloride (Intermediate 1, 40.0 mg, 0.12 mmol) was dissolved in dichloromethane. 1,1'-Carbonyldiimidazole (19.0 mg, 0.12 mmol), triethylamine (0.03 mL, 0.24 mmol), and cyclopropylmethylamine (8.0 mg, 0.12 mmol) were added, and the mixture was stirred at room temperature for 16 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (10 mg, 21%). 4 N hydrochloric acid (0.05 mL, 0.5 M) dissolved in ethyl acetate and 1,4-dioxane was added and stirred at room temperature for 12 hours. After the reaction was completed, the hydrochloride salt of the target compound was obtained by filtration.
$^1$H NMR (500 MHz, DMSO-$d_6$) δ 13.44 (s, 1H), 9.04 (s, 1H), 8.31 (s, 1H), 7.22-7.19 (m, 3H), 6.73 (s, 1H), 6.57 (s, 1H), 6.18 (s, 1H), 4.30-4.29 (d, 2H), 3.02-2.99 (t, 2H), 2.21-2.19 (m, 12H), 0.98-0.95 (m, 1H) 0.44-0.43 (m, 2H), 0.21-0.20 (m, 2H)

**[Example 31] Preparation of 1-(8-((2,6-dimethylbenzyl)amino)-2,3-dimethylimidazo[1,2-a]pyridin-6-yl)-3-isopropylurea hydrochloride**

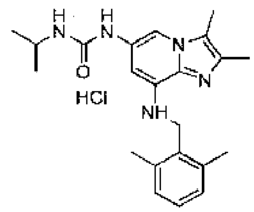

$N^8$-(2,6-Dimethylbenzyl)-2,3-dimethylimidazo[1,2-a]pyridine-6,8-diamine hydrochloride (Intermediate 1, 80.0 mg, 0.24 mmol) was dissolved in dichloromethane. 1,1'-Carbonyldiimidazole (14.0 mg, 0.24 mmol), triethylamine (0.06 mL, 0.48 mmol), and isopropylamine (14.0 mg, 0.24 mmol) were added and stirred at room temperature for 16 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (8 mg, 8%). 4 N hydrochloric acid (0.1 mL, 0.5 M) dissolved in ethyl acetate and 1,4-dioxane was added and stirred at room temperature for 12 hours. After the reaction was completed, the hydrochloride salt of the target compound was obtained by filtration.
$^1$H NMR (500 MHz, DMSO-$d_6$) δ 13.41 (s, 1H), 8.86 (s, 1H), 8.31 (s, 1H), 7.23-7.20 (m, 1H), 7.15-7.14 (m, 2H), 6.71 (s, 1H), 6.38-6.36 (d, 1H), 6.17 (s, 1H), 4.31-4.30 (d, 2H), 3.80-3.78 (m, 1H), 2.39-2.37 (m, 12H), 1.14-1.12 (m, 6H)

**[Example 32] Preparation of 3-(8-((2,6-dimethylbenzyl)amino)-2,3-dimethylimidazo[1,2-a]pyridin-6-yl)-1-ethyl-1-methylurea hydrochloride**

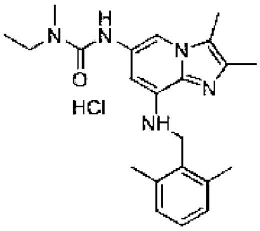

$N^8$-(2,6-Dimethylbenzyl)-2,3-dimethylimidazo[1,2-a]pyridine-6,8-diamine hydrochloride (Intermediate 1, 60.0 mg, 0.18 mmol) was dissolved in dichloromethane. 1,1'-Carbonyldiimidazole (29.1 mg, 0.18 mmol), triethylamine (0.07 mL, 0.54 mmol), and N-methylethanamine (10.0 mg, 0.18 mmol) were added and stirred at room temperature for 16 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (13 mg, 19%). 4 N hydrochloric acid (0.3 mL, 0.5 M) dissolved in ethyl acetate and 1,4-dioxane was added and stirred at room temperature for 12 hours. After the reaction was completed, the hydrochloride salt of the target compound was obtained by filtration.
$^1$H NMR (500 MHz, DMSO-$d_6$) δ 13.42 (s, 1H), 8.59 (s, 1H), 8.39 (s, 1H), 7.23-7.20 (m, 2H), 7.15-7.14 (m, 2H), 6.12 (s, 1H), 4.31-4.30 (d, 2H), 3.41-3.40 (m, 2H), 2.99 (s, 3H), 2.39-2.37 (m, 12H), 1.12-1.10 (t, 3H)

**[Example 33] Preparation of 3-(8-((2,6-dimethylbenzyl)amino)-2,3-dimethylimidazo[1,2-a]pyridin-6-yl)-1-ethyl-1-methylurea hydrochloride**

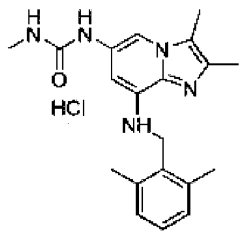

$N^8$-(2,6-Dimethylbenzyl)-2,3-dimethylimidazo[1,2-a]pyridine-6,8-diamine hydrochloride (Intermediate 1, 60.0 mg, 0.18 mmol) was dissolved in dichloromethane. 1,1'-Carbonyldiimidazole (29.1 mg, 0.18 mmol), triethylamine (0.07 mL, 0.54 mmol), and methylamine (14.0 mg, 0.45 mmol) diluted 40% in methanol were added, and the mixture was stirred at room temperature for 16 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (35 mg, 55%). 4 N hydrochloric acid (0.3 mL, 0.5 M) dissolved in ethyl acetate and 1,4-dioxane was added and stirred at room temperature for 12 hours. After the reaction was completed, the hydrochloride salt of the target

compound was obtained by filtration.
$^1$H NMR (500 MHz, DMSO-$d_6$) δ 13.35 (s, 1H), 9.04 (s, 1H), 8.33 (s, 1H), 7.23-7.20 (m, 1H), 7.16-7.14 (m, 2H), 6.79 (s, 1H), 6.36-6.35 (m, 1H), 6.13 (s, 1H), 4.30-4.29 (d, 2H), 2.69-2.68 (m, 3H), 2.39-2.37 (m, 12H)

**[Example 34] Preparation of ethyl 3-((8-((2,6-dimethylbenzyl)amino)-2,3-dimethylimidazo[1,2-a]pyridin-6-yl) amino)-3-oxopropanoate**

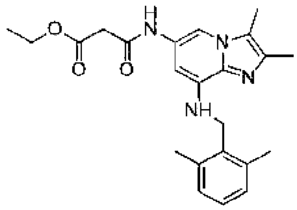

N$^8$-(2,6-Dimethylbenzyl)-2,3-dimethylimidazo[1,2-a]pyridine-6,8-diamine hydrochloride (Intermediate 1, 50 mg, 0.14 mmol) was dissolved in dichloromethane (2 mL). Ethyl hydrogen malonate (22 mg, 0.16 mmol), 1-[bis(dimethylamino)methylene]-1H-1,2,3-triazolo[4,5-b]pyridinium 3-oxide hexafluorophosphate (74 mg, 0.19 mmol), and diisopropylethylamine (0.9 mL, 0.60 mmol) were added dropwise and stirred at room temperature for 12 hours. The reaction mixture was concentrated in vacuo, and the residue was purified by column chromatography to obtain the target compound (46 mg, 75%).
$^1$H NMR (500 MHz, $CDCl_3$) δ 9.26 (s, 1H), 8.21 (s, 1H), 7.12-7.10 (m, 1H), 7.05-7.02 (m, 2H), 6.06 (s, 1H), 4.88 (s, 1H), 4.32-4.28 (m, 4H), 3.53(s, 2H), 2.35-2.31 (m, 12H), 1.37-1.34 (t, 3H)

**[Example 35] Preparation of (S)-N-(8-((2,6-dimethylbenzyl)amino)-2,3-dimethylimidazo[1,2-a]pyridin-6-yl)azetidine-2-carboxamide hydrochloride**

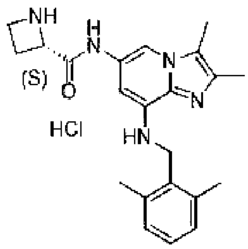

Step 1: Preparation of tert-butyl (S)-2-((8-((2,6-dimethylbenzyl)amino)-2,3-dimethylimidazo[1,2-a]pyridin-6-yl)carbamoyl)azetidine-1-carboxylate

N$^8$-(2,6-dimethylbenzyl)-2,3-dimethylimidazo[1,2-a]pyridine-6,8-diamine hydrochloride (Intermediate 1, 50.0 mg, 0.15 mmol) was dissolved in dichloromethane. 1-[Bis(dimethylamino)methylene]-1H-1,2,3-triazolo[4,5-B]pyridinium 3-oxide hexafluorophosphate (74.0 mg, 0.19 mmol), diisopropylethylamine (0.1 mL, 0.60 mmol), and (S)-1-(tert-butoxycarbonyl)azetidine-2-carboxylic acid (30.0 mg, 0.15 mmol) were added and stirred at room temperature for 6 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (20 mg, 27%).

Step 2: Preparation of (S)-N-(8-((2,6-dimethylbenzyl)amino)-2,3-dimethylimidazo[1,2-a]pyridin-6-yl)azetidine-2-carboxamide hydrochloride

Tert-butyl (S)-2-((8-((2,6-dimethylbenzyl)amino)-2,3-dimethylimidazo[1,2-a]pyridin-6-yl)carbamoyl)azetidine-1-carboxylate (20.0 mg, 0.04 mmol) prepared in Step 1 was dissolved in ethyl acetate. 4 N hydrochloric acid (0.08 mL, 0.5 M) dissolved in 1,4-dioxane was added and stirred at room temperature for 6 hours. Upon completion of the

reaction, the mixture was filtered to obtain the target compound (8 mg, 48%).
$^1H$ NMR (500 MHz, DMSO-$d_6$) δ 13.89 (s, 1H), 11.28 (s, 1H), 9.50 (s, 1H), 9.02 (s, 1H), 8.52 (s, 1H), 7.23-7.20 (m, 1H), 7.05-7.04 (m, 2H), 7.02 (s, 1H), 6.47 (s, 1H), 5.22-5.20 (m, 1H), 4.31-4.30 (m, 2H), 4.05-4.03 (m, 1H), 3.86-3.82 (m, 1H), 2.79-2.77 (m, 1H), 2.43-2.39 (m, 12H)

**[Example 36] Preparation of (R)-N-(8-((2,6-dimethylbenzyl)amino)-2,3-dimethylimidazo[1,2-a]pyridin-6-yl)azetidine-2-carboxamide hydrochloride**

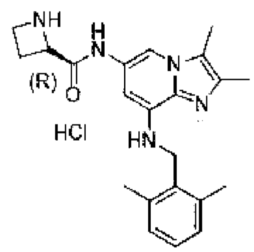

Step 1: Preparation of tert-butyl (R)-2-((8-((2,6-dimethylbenzyl)amino)-2,3-dimethylimidazo[1,2-a]pyridin-6-yl)carbamoyl)azetidine-1-carboxylate

$N^8$-(2,6-dimethylbenzyl)-2,3-dimethylimidazo[1,2-a]pyridine-6,8-diamine hydrochloride (Intermediate 1, 50.0 mg, 0.15 mmol) was dissolved in dichloromethane. 1-[Bis(dimethylamino)methylene]-1H-1,2,3-triazolo[4,5-B]pyridinium 3-oxide hexafluorophosphate (74.0 mg, 0.19 mmol), diisopropylethylamine (0.1 mL, 0.60 mmol), and (R)-1-(tert-butoxycarbonyl)azetidine-2-carboxylic acid (30.0 mg, 0.15 mmol) were added and stirred at room temperature for 6 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (20 mg, 27%).

Step 2: Preparation of (R)-N-(8-((2,6-dimethylbenzyl)amino)-2,3-dimethylimidazo[1,2-a]pyridin-6-yl)azetidine-2-carboxamide hydrochloride

Tert-butyl (R)-2-((8-((2,6-dimethylbenzyl)amino)-2,3-dimethylimidazo[1,2-a]pyridin-6-yl)carbamoyl)azetidine-1-carboxylate (20.0 mg, 0.04 mmol) prepared in Step 1 was dissolved in ethyl acetate. 4 N hydrochloric acid (0.08 mL, 0.5 M) dissolved in 1,4-dioxane was added and stirred at room temperature for 6 hours. Upon completion of the reaction, the mixture was filtered to obtain the target compound (8 mg, 48%).
$^1H$ NMR (500 MHz, DMSO-$d_6$) δ 14.05 (s, 1H), 11.42 (s, 1H), 9.62 (s, 1H), 9.02 (s, 1H), 8.52 (s, 1H), 7.22-7.06 (m, 4H), 6.54 (s, 1H), 5.21-5.20 (m, 1H), 4.31-4.30 (m, 2H), 4.05-4.04 (m, 1H), 3.84-3.81 (m, 1H), 2.42-2.36 (m, 12H)

**[Example 37] Preparation of 1-(2-aminoethyl)-3-(8-((2,6-dimethylbenzyl)amino)-2,3-dimethylimidazo[1,2-a]pyridin-6-yl)urea hydrochloride**

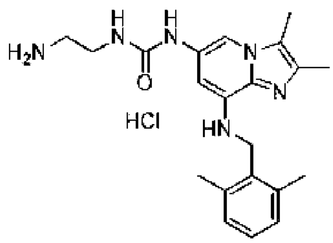

Step 1: Preparation of tert-butyl (2-(3-(8-((2,6-dimethylbenzyl)amino)-2,3-dimethylimidazo[1,2-a]pyridin-6-yl)ureido) ethyl)carbamate

$N^8$-(2,6-dimethylbenzyl)-2,3-dimethylimidazo[1,2-a]pyridine-6,8-diamine hydrochloride (Intermediate 1, 60.0 mg, 0.18 mmol) was dissolved in dichloromethane. 1,1'-Carbonyldiimidazole (58.0 mg, 0.36 mmol), triethylamine (0.07 mL, 0.54 mmol), and tert-butyl (2-aminoethyl)carbamate (28.0 mg, 0.18 mmol) were added, and the mixture was stirred at room temperature for 16 hours. After the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (20 mg, 23%).

Step 2: Preparation of 1-(2-aminoethyl)-3-(8-((2,6-dimethylbenzyl)amino)-2,3-dimethylimidazo[1,2-a]pyridin-6-yl)urea hydrochloride

Tert-butyl (2-(3-(8-((2,6-dimethylbenzyl)amino)-2,3-dimethylimidazo[1,2-a]pyridin-6-yl)ureido)ethyl)carbamate (15.0 mg, 0.03 mmol), prepared in Step 1, was dissolved in ethyl acetate. 4 N hydrochloric acid (0.6 mL, 0.5 M) dissolved in 1,4-dioxane was added and stirred at room temperature for 12 hours. After the reaction was completed, the target compound (10 mg, 77%) was obtained by filtration.
$^1$H NMR (500 MHz, DMSO-$d_6$) δ 13.72 (s, 1H), 9.52 (s, 1H), 8.29 (s, 1H), 7.96 (s, 3H), 7.21-7.12 (m, 3H), 6.86-6.84 (m, 2H), 6.33 (s, 1H), 4.30-4.29 (m, 2H), 3.39-3.26 (m, 2H), 2.93-2.90 (m, 2H), 2.41-2.38 (m, 12H)

**[Example 38] Preparation of 4-((8-((2,6-dimethylbenzyl)amino)-2,3-dimethylimidazo[1,2-a]pyridin-6-yl)amino)-4-oxobutanoic acid**

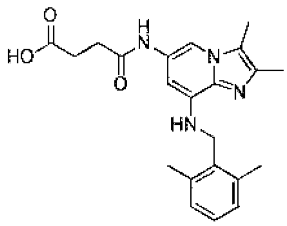

4-((8-((2,6-dimethylbenzyl)amino)-2,3-dimethylimidazo[1,2-a]pyridin-6-yl)amino)-4-oxobutanoic acid (Example 38, 37 mg, 0.09 mmol) was dissolved in tetrahydrofuran (1 mL), methanol (1 mL), and distilled water (1 mL). Potassium hydroxide (15 mg, 0.26 mmol) was added, and the mixture was stirred at room temperature for 5 hours. The reaction was terminated by adding 1 N aqueous hydrochloric acid solution, and the pH of the mixture was maintained at 2. After extraction with ethyl acetate, the organic layer was dried over anhydrous magnesium sulfate, and concentrated under reduced pressure. The concentrated residue was dried to obtain the target compound (21 mg, 60%).
$^1$H NMR (500 MHz, DMSO-$d_6$) δ 9.95 (s, 1H), 8.21 (s, 1H), 7.16-7.08 (m, 3H), 6.25 (s, 1H), 4.97(s, 1H), 4.30-4.29 (m, 2H), 2.59-2.55 (m, 2H), 2.34-2.20 (m, 12H)

**[Example 39] Preparation of 3-(3-((8-((2,6-dimethylbenzyl)amino)-2,3-dimethylimidazo[1,2-a]pyridin-6-yl)amino)-3-oxopropyl)propanoic acid**

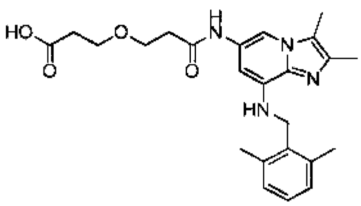

Tert-butyl 3-(3-((8-((2,6-dimethylbenzyl)amino)-2,3-dimethylimidazo[1,2-a]pyridin-6-yl)amino)-3-oxopropyl) propanoate (Example 40, 30 mg, 0.06 mmol) was dissolved in dichloromethane (2 mL). Trifluoroacetic acid (0.02 mL,

0.30 mmol) was added, and the mixture was stirred at room temperature for 3 hours. After the reaction was completed, the mixture was reduced in pressure and concentrated to obtain the target compound (13 mg, 49%).
$^1H$ NMR (500 MHz, DMSO-$d_6$) δ 10.35 (s, 1H), 8.53 (s, 1H), 7.23-7.14 (m, 3H), 6.90-6.84 (m, 1H), 6.10-6.08 (m, 1H), 4.31-4.30 (m, 2H), 3.63-3.61 (m, 4H), 2.61-2.50 (m, 2H), 2.46-2.36 (m, 15H)

**[Example 40] Preparation of ethyl 4-((8-((2,6-dimethylbenzyl)amino)-2,3-dimethylimidazo[1,2-a]pyridin-6-yl)amino)-4-oxobutanoate**

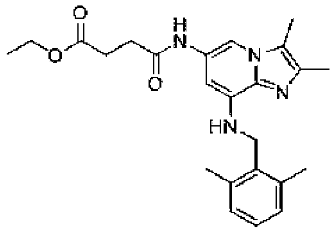

N8-(2,6-Dimethylbenzyl)-2,3-dimethylimidazo[1,2-a]pyridine-6,8-diamine hydrochloride (Intermediate 1, 50 mg, 0.14 mmol) and triethylamine (0.05 mL, 0.35 mmol) were dissolved in dichloromethane (2 mL). Ethyl succinyl chloride (19 mg, 0.16 mmol) was slowly added dropwise and stirred at room temperature for 12 hours. The reaction mixture was concentrated in vacuo, and the residue was purified by column chromatography to obtain the target compound (42 mg, 62%).
$^1H$ NMR (500 MHz, DMSO-$d_6$) δ 8.16 (s, 1H), 7.46 (s, 1H), 7.26-7.03 (m, 3H), 5.92 (s, 1H), 4.91(s, 2H), 4.31(s, 2H), 4.22-4.17 (m, 2H), 2.78-2.77 (m, 2H), 2.71-2.70 (m, 2H), 2.37-2.31 (m, 12H), 1.30-1.25 (m, 3H)

**[Example 41] Preparation of tert-butyl 3-(3-((8-((2,6-dimethylbenzyl)amino)-2,3-dimethylimidazo[1,2-a]pyridin-6-yl)amino)-3-oxopropyl)propanoate**

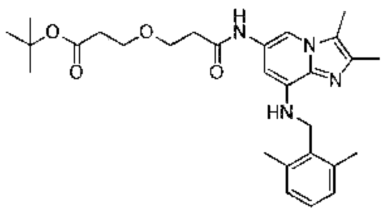

N8-(2,6-Dimethylbenzyl)-2,3-dimethylimidazo[1,2-a]pyridine-6,8-diamine hydrochloride (Intermediate 1, 50 mg, 0.15 mmol) was dissolved in dichloromethane (2 mL). 3-(3-(Tert-butoxy)-3-oxopropoxy)propionic acid (28 mg, 0.12 mmol), 1-[bis(dimethylamino)methylene]-1H-1,2,3-triazolo[4,5-b]pyridinium 3-oxide hexafluorophosphate (74 mg, 0.19 mmol), and diisopropylethylamine (0.9 mL, 0.60 mmol) were added dropwise and stirred at room temperature for 12 hours. The reaction mixture was concentrated in vacuo and the residue was purified by column chromatography to obtain the target compound (34 mg, 46%).
$^1H$ NMR (500 MHz, DMSO-$d_6$) δ 8.36 (s, 1H), 7.32-7.01 (m, 3H), 6.31-6.30 (m, 1H), 5.35-5.34 (m, 1H), 4.85 (s, 1H), 4.36-4.35 (m, 2H), 2.71-2.70 (m, 2H), 2.59-2.57 (m, 2H), 1.66-1.62 (m, 12H), 1.42-1.31 (m, 9H)

**[Example 42] Preparation of tert-butyl (2-(3-(8-((2,6-dimethylbenzyl)amino)-2,3-dimethylimidazo[1,2-a]pyridin-6-yl)ureido)ethyl)carbamate hydrochloride**

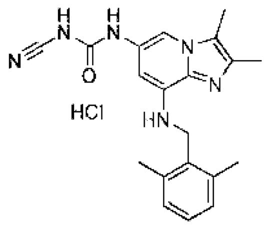

$N^8$-(2,6-Dimethylbenzyl)-2,3-dimethylimidazo[1,2-a]pyridine-6,8-diamine hydrochloride (Intermediate 1, 60.0 mg, 0.18 mmol) was dissolved in dichloromethane. 1,1'-Carbonyldiimidazole (58.0 mg, 0.36 mmol), triethylamine (0.07 mL, 0.54 mmol), and tert-butyl(2-aminoethyl)carbamate (28.0 mg, 0.18 mmol) were added and stirred at room temperature for 16 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (20 mg, 23%). 4 N hydrochloric acid (0.6 mL, 0.5 M) dissolved in 1,4-dioxane was added and stirred at room temperature for 12 hours. After the reaction was completed, the hydrochloride salt of the target compound was obtained by filtration. $^1H$ NMR (500 MHz, DMSO-$d_6$) δ 8.52 (s, 1H), 8.44 (s, 1H), 7.21-7.13 (m, 3H), 6.95 (s, 1H), 5.78-5.76 (m, 2H), 4.24-4.23 (m, 2H), 2.35 (s, 12H)

**[Example 43] Preparation of 3-(8-((2,6-dimethylbenzyl)amino)-2,3-dimethylimidazo[1,2-a]pyridin-6-yl)-1,1-dimethylurea hydrochloride**

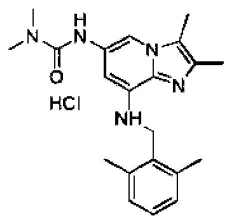

$N^8$-(2,6-Dimethylbenzyl)-2,3-dimethylimidazo[1,2-a]pyridine-6,8-diamine hydrochloride (Intermediate 1, 50.0 mg, 0.15 mmol) was dissolved in dichloromethane. 1,1'-Carbonyldiimidazole (48.0 mg, 0.3 mmol), triethylamine (0.06 mL, 0.45 mmol), and 10% dimethylamine diluted in methanol (67.0 mg, 1.5 mmol) were added and stirred at room temperature for 16 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (20 mg, 36%). 4 N hydrochloric acid (0.2 mL, 0.5 M) dissolved in 1,4-dioxane was added and stirred at room temperature for 12 hours. After the reaction was completed, the hydrochloride salt of the target compound was obtained by filtration. $^1H$ NMR (500 MHz, DMSO-$d_6$) δ 13.46 (s, 1H), 8.66 (s, 1H), 8.36 (s, 1H), 7.23-7.14 (m, 4H), 6.12 (s, 1H), 4.31-4.30 (m, 2H), 2.99 (s, 6H), 2.38-2.37 (m, 12H)

**[Example 44] Preparation of 1-(8-((4-fluoro-2,6-dimethylbenzyl)amino)-2,3-dimethylimidazo[1,2-a]pyridin-6-yl)-3-methylurea hydrochloride**

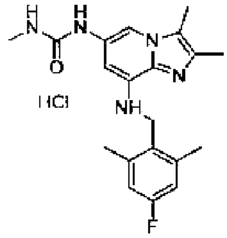

$N^8$-(4-Fluoro-2,6-dimethylbenzyl)-2,3-dimethylimidazo[1,2-a]pyridine-6,8-diamine hydrochloride (Intermediate

2, 70.0 mg, 0.20 mmol) was dissolved in dichloromethane. 1,1'-Carbonyldiimidazole (64.0 mg, 0.4 mmol), triethylamine (0.08 mL, 0.6 mmol), and 40% methylamine diluted in methanol (15.5 mg, 0.5 mmol) were added and stirred at room temperature for 16 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (10 mg, 13%). 4 N hydrochloric acid (0.1 mL, 0.2 M) dissolved in 1,4-dioxane was added and stirred at room temperature for 12 hours. After the reaction was completed, the hydrochloride salt of the target compound was obtained by filtration.

$^1$H NMR (500 MHz, DMSO-$d_6$) δ 13.74 (s, 1H), 9.16 (s, 1H), 8.32 (s, 1H), 7.01-6.99 (m, 2H), 6.77 (s, 1H), 6.44 (s, 1H), 6.41 (s, 1H), 6.26 (s, 1H), 4.26-4.25 (m, 2H), 2.68 (s, 3H), 2.38-2.37 (m, 12H)

**[Example 45] Preparation of N-(8-((4-fluoro-2,6-dimethylbenzyl)amino)-2,3-dimethylimidazo[1,2-a]pyridin-6-yl) acetamide hydrochloride**

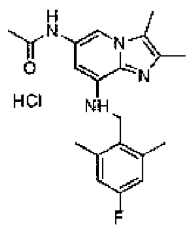

N8-(4-Fluoro-2,6-dimethylbenzyl)-2,3-dimethylimidazo[1,2-a]pyridine-6,8-diamine hydrochloride (Intermediate 2, 70.0 mg, 0.2 mmol) was dissolved in dichloromethane. Triethylamine (0.05 mL, 0.4 mmol) and acetic anhydride (0.01 mL, 0.2 mmol) were added, and the mixture was stirred at room temperature for 6 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (45 mg, 60%). 4 N hydrochloric acid (0.25 mL, 0.5 M) dissolved in 1,4-dioxane was added, and the mixture was stirred at room temperature for 12 hours. When the reaction was complete, the solution was filtered to obtain the hydrochloride salt of the target compound.

$^1$H NMR (500 MHz, DMSO-$d_6$) δ 13.67 (s, 1H), 10.38 (s, 1H), 8.47 (s, 1H), 7.02-7.00 (m, 2H), 6.83 (s, 1H), 6.20-6.16 (brs, 1H), 4.26-4.25 (m, 2H), 2.38-2.37 (m, 12H), 2.12 (s, 3H)

**[Example 46] Preparation of methyl((8-((2,6-dimethylbenzyl)amino)-2,3-dimethylimidazo[1,2-a]pyridin-6-yl)carbamoyl)glycinate hydrochloride**

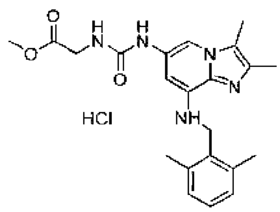

N8-(2,6-Dimethylbenzyl)-2,3-dimethylimidazo[1,2-a]pyridine-6,8-diamine hydrochloride (Intermediate 1, 50.0 mg, 0.15 mmol) was dissolved in dichloromethane. 1,1'-Carbonyldiimidazole (48.0 mg, 0.3 mmol), triethylamine (0.06 mL, 0.45 mmol), and methyl glycinate (13.0 mg, 0.15 mmol) were added and stirred at room temperature for 16 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (10 mg, 16%). 4 N hydrochloric acid (0.04 mL, 0.5 M) dissolved in 1,4-dioxane was added and stirred at room temperature for 12 hours. After the reaction was completed, the hydrochloride salt of the target compound was obtained by filtration.

$^1$H NMR (500 MHz, DMSO-$d_6$) δ 13.41 (s, 1H), 9.35 (s, 1H), 8.30 (s, 1H), 7.23-7.20 (m, 2H), 7.15-7.14 (m, 1H), 6.82-6.77 (m, 1H), 6.16 (s, 1H), 4.31-4.30 (m, 2H), 3.92-3.91 (m, 2H), 3.66 (s, 3H), 2.38-2.37 (m, 12H)

**[Example 47] Preparation of 2-(3-(8-((2,6-dimethylbenzyl)amino)-2,3-dimethylimidazo[1,2-a]pyridin-6-yl)ureido)-N-methylacetamide hydrochloride**

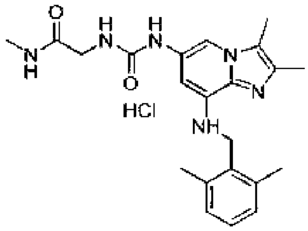

$N^8$-(2,6-Dimethylbenzyl)-2,3-dimethylimidazo[1,2-a]pyridine-6,8-diamine hydrochloride (Intermediate 1, 50.0 mg, 0.15 mmol) was dissolved in dichloromethane. 1,1'-Carbonyldiimidazole (48.0 mg, 0.3 mmol), triethylamine (0.06 mL, 0.45 mmol), and 2-amino-N-methylacetamide (13.0 mg, 0.15 mmol) were added and stirred at room temperature for 16 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (10 mg, 16%). 4 N hydrochloric acid (0.08 mL, 0.5 M) dissolved in 1,4-dioxane was added and stirred at room temperature for 12 hours. After the reaction was completed, the hydrochloride salt of the target compound was obtained by filtration.
$^1$H NMR (500 MHz, DMSO-$d_6$) δ 13.57 (s, 1H), 9.32 (s, 1H), 8.30 (s, 1H), 7.91 (s, 1H), 7.22-7.20 (m, 1H), 7.14-7.13 (m, 2H), 6.73 (s, 1H), 6.67-6.65 (m, 2H), 6.24 (s, 1H), 4.31-4.30 (m, 2H), 3.73-3.72 (m, 2H), 2.62 (s, 3H), 2.38-2.37 (m, 12H)

**[Example 48] Preparation of 2-(3-(8-((2,6-dimethylbenzyl)amino)-2,3-dimethylimidazo[1,2-a]pyridin-6-yl)ureido)-N,N-dimethylacetamide hydrochloride**

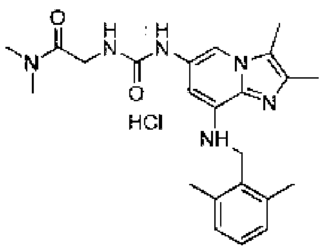

$N^8$-(2,6-Dimethylbenzyl)-2,3-dimethylimidazo[1,2-a]pyridine-6,8-diamine hydrochloride (Intermediate 1, 50.0 mg, 0.15 mmol) was dissolved in dichloromethane. 1,1'-Carbonyldiimidazole (48.0 mg, 0.3 mmol), triethylamine (0.06 mL, 0.45 mmol), and 2-amino-N,N-dimethylacetamide (15.0 mg, 0.15 mmol) were added and stirred at room temperature for 16 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (11 mg, 17%). 4 N hydrochloric acid (0.04 mL, 0.5 M) dissolved in 1,4-dioxane was added and stirred at room temperature for 12 hours. After the reaction was completed, the hydrochloride salt of the target compound was obtained by filtration.
$^1$H NMR (500 MHz, DMSO-$d_6$) δ 13.51 (s, 1H), 9.40 (s, 1H), 8.31 (s, 1H), 7.22-7.19 (m, 1H), 7.15-7.13 (m, 2H), 6.71 (s, 1H), 6.59-6.57 (m, 2H), 6.22 (s, 1H), 4.31-4.30 (m, 2H), 3.99-3.98 (m, 2H), 2.97 (s, 3H), 2.87 (s, 3H), 2.38-2.37 (m, 12H)

**[Example 49] Preparation of 3-(8-((4-fluoro-2,6-dimethylbenzyl)amino)-2,3-dimethylimidazo[1,2-a]pyridin-6-yl)-1,1-dimethylurea hydrochloride**

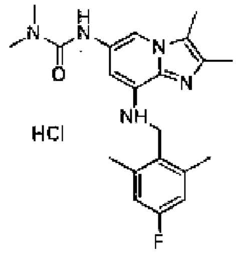

$N^8$-(4-Fluoro-2,6-dimethylbenzyl)-2,3-dimethylimidazo[1,2-a]pyridine-6,8-diamine hydrochloride (Intermediate 2, 50.0 mg, 0.14 mmol) was dissolved in dichloromethane. 1,1'-Carbonyldiimidazole (46.0 mg, 0.28 mmol), triethylamine (0.06 mL, 0.42 mmol), and 10% dimethylamine diluted in methanol (70.0 mg, 1.43 mmol) were added and stirred at room temperature for 16 hours. When the reaction was complete, dichloromethane (50 mL) and distilled water (50 mL) were added, the layers were separated, and the organic layer was recovered, dried over anhydrous magnesium sulfate, and filtered. The filtrate was concentrated under reduced pressure and purified by column chromatography to obtain the target compound (16 mg, 29%). 4 N hydrochloric acid (0.08 mL, 0.5 M) dissolved in 1,4-dioxane was added and stirred at room temperature for 12 hours. After the reaction was completed, the hydrochloride salt of the target compound was obtained by filtration.

$^1H$ NMR (500 MHz, DMSO-$d_6$) δ 13.33 (s, 1H), 8.64 (s, 1H), 8.35 (s, 1H), 7.17 (s, 1H), 7.04-7.02 (m, 2H), 6.05 (s, 1H), 4.27-4.26 (m, 2H), 2.98 (s, 6H), 2.39-2.37 (m, 12H)

**[Example 50] Preparation of 1-cyclopropyl-3-(8-((4-fluoro-2,6-dimethylbenzyl)amino)-2,3-dimethylimidazo[1,2-a]pyridin-6-yl)urea hydrochloride**

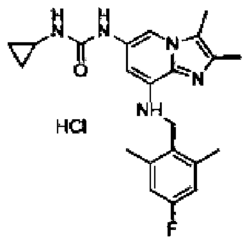

$N^8$-(4-Fluoro-2,6-dimethylbenzyl)-2,3-dimethylimidazo[1,2-a]pyridine-6,8-diamine hydrochloride (Intermediate 2, 50 mg, 0.14 mmol) and 1,1'-carbonyldiimidazole (45 mg, 0.28 mmol) were dissolved in dichloromethane (0.7 mL) and stirred at room temperature for 1 hour. Cyclopropanamine (0.009 mL, 0.14 mmol) and triethylamine (0.05 mL, 0.42 mmol) were added and stirred at room temperature for 6 hours. The reaction mixture was purified by column chromatography, dissolved in ethyl acetate, and 4 N hydrochloric acid diluted in 1,4-dioxane (0.28 mL) was added and stirred at room temperature for 2 hours. The resulting solid was filtered and dried to obtain the target compound (18 mg, 34%).

$^1H$ NMR (500 MHz, DMSO-$d_6$) δ 13.42 (s, 1H), 8.80 (s, 1H), 8.32 (s, 1H), 7.03-7.01 (m, 2H), 6.78-6.67 (m, 2H), 6.02 (s, 1H), 4.27-4.26 (m, 2H), 2.98 (s, 6H), 2.38-2.37 (m, 12H), 1.19-1.16 (m, 1H), 0.85-0.68 (m, 2H), 0.45-0.44 (m, 2H)

**[Example 51] Preparation of N-(8-((4-fluoro-2,6-dimethylbenzyl)amino)-2,3-dimethylimidazo[1,2-a]pyridin-6-yl) azetidine-1-carboxamide citrate**

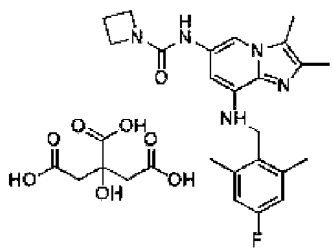

$N^8$-(4-Fluoro-2,6-dimethylbenzyl)-2,3-dimethylimidazo[1,2-a]pyridine-6,8-diamine hydrochloride (Intermediate 2, 80 mg, 0.22 mmol) and 1,1'-carbonyldiimidazole (35 mg, 0.22 mmol) were dissolved in dichloromethane (3.14 mL) and stirred at room temperature for 1 hour. Azetidine (0.01 mL, 0.22 mmol) and triethylamine (0.06 mL, 0.44 mmol) were added and stirred at room temperature for 12 hours. The reaction mixture was purified by column chromatography to obtain the target compound (10 mg, 11%). This was then dissolved in acetone, citric acid (9 mg, 0.05 mmol) was added, and stirred at room temperature for 12 hours. The resulting solid was filtered and dried to obtain the citrate salt of the target compound.
$^1$H NMR (500 MHz, DMSO-$d_6$) $\delta$ 8.40 (s, 1H), 8.08 (s, 1H), 6.99-6.97 (m, 2H), 6.67-6.59 (m, 1H), 5.33-5.32 (m, 1H), 4.24-4.23 (m, 2H), 3.99-3.97 (m, 4H), 2.69-2.57 (m, 4H), 2.38-2.35 (m, 12H)

**[Example 52] Preparation of 2-acetamido-N-(8-((2,6-dimethylbenzyl)amino)-2,3-dimethylimidazo[1,2-a]pyridin-6-yl)acetamide**

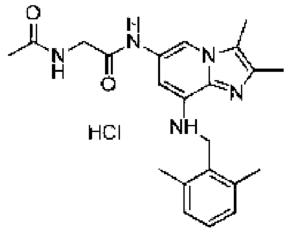

2-Amino-N-(8-((2,6-dimethylbenzyl)amino)-2,3-dimethylimidazo[1,2-a]pyridin-6-yl)acetamide (Example 10, 80 mg, 0.23 mmol) and trimethylamine (0.06 mL, 0.45 mmol) were dissolved in dimethylformamide (4 mL). Acetic anhydride (0.03 mL, 0.34 mmol) was added, and the mixture was stirred at room temperature for 2 hours. The reaction was quenched by adding saturated aqueous sodium bicarbonate, extracted with ethyl acetate, and the organic layer was dried over anhydrous magnesium sulfate, reduced pressure, and concentrated. The concentrated residue was dissolved in dichloromethane, and a 2 M hydrochloric acid solution (0.5 mL) diluted with diethyl ether was added, and the mixture was stirred at room temperature for 12 hours. The target compound (24 mg, 24%) was obtained by reducing pressure and concentrating.
$^1$H NMR (500 MHz, DMSO-$d_6$) $\delta$ 13.70 (s, 1H), 10.47 (s, 1H), 8.50 (s, 1H), 8.34-8.31 (m, 1H), 7.23-7.20 (m, 4H), 6.97 (s, 1H), 4.31-4.30 (m, 2H), 3.93-3.91 (m, 2H), 2.37 (s, 12H), 1.98 (s, 3H)

**[Example 53] Preparation of 2-(dimethylamino)-N-(8-((2,6-dimethylbenzyl)amino)-2,3-dimethylimidazo[1,2-a]pyridin-6-yl)acetamide**

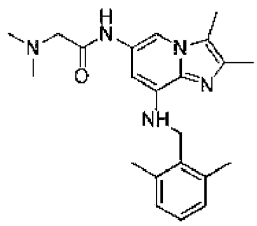

$N^8$-(2,6-Dimethylbenzyl)-2,3-dimethylimidazo[1,2-a]pyridine-6,8-diamine hydrochloride (Intermediate 1, 50 mg, 0.14 mmol) was dissolved in dichloromethane (2 mL). 2-(Dimethylamino)acetic acid (16 mg, 0.16 mmol), 1-[bis(dimethylamino)methylene]-1H-1,2,3-triazolo[4,5-b]pyridinium 3-oxide hexafluorophosphate (74 mg, 0.19 mmol), and diisopropylethylamine (0.9 mL, 0.60 mmol) were added dropwise and stirred at room temperature for 12 hours. The reaction mixture was concentrated in vacuo, and the residue was purified by column chromatography to obtain the target compound (24 mg, 42%).
$^1$H NMR (500 MHz, DMSO-$d_6$) $\delta$ 9.74-9.70 (m, 1H), 8.23-8.16 (m, 1H), 7.20-7.08 (m, 4H), 6.67-6.45 (m, 1H), 4.31-4.30 (m, 2H), 2.34 (s, 14H), 1.29-1.27 (m, 6H)

**[Example 54] Preparation of 1-butyl-3-(8-((2,6-dimethylbenzyl)amino)-2,3-dimethylimidazo[1,2-a]pyridin-6-yl) urea**

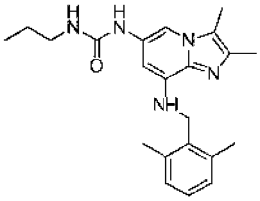

$N^8$-(2,6-Dimethylbenzyl)-2,3-dimethylimidazo[1,2-a]pyridine-6,8-diamine hydrochloride (Intermediate 1, 50 mg, 0.14 mmol) and 1,1'-carbonyldiimidazole (49 mg, 0.30 mmol) were dissolved in dichloromethane (2 mL) and stirred at room temperature for 1 hour. Triethylamine (0.06 mL, 0.45 mmol) and propylamine hydrochloride (14 mg, 0.15 mmol) were added and stirred at room temperature for 6 hours. The reaction mixture was concentrated in vacuo, and the residue was purified by column chromatography to obtain the target compound (22 mg, 37%).
$^1H$ NMR (500 MHz, DMSO-$d_6$) $\delta$ 8.23 (s, 1H), 7.98 (m, 1H), 7.16-7.07 (m, 4H), 6.16-6.14 (m, 1H), 6.07-6.01 (m, 1H), 4.30-4.29 (m, 2H), 3.05-3.04 (m, 2H), 2.35-2.22 (m, 12H), 1.47-1.46 (m, 2H), 0.89-0.86 (m, 3H)

**[Example 55] Preparation of N-(2-((8-((2,6-dimethylbenzyl)amino)-2,3-dimethylimidazo[1,2-a]pyridin-6-yl)amino)-2-oxoethyl)pivalamide**

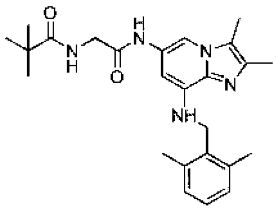

2-Amino-N-(8-((2,6-dimethylbenzyl)amino)-2,3-dimethylimidazo[1,2-a]pyridin-6-yl)acetamide (Example 10, 40 mg, 0.10 mmol) was dissolved in dichloromethane (2 mL). Parvalic acid (11 mg, 0.11 mmol), 1-[bis(dimethylamino) methylene]-1H-1,2,3-triazolo[4,5-b]pyridinium 3-oxide hexafluorophosphate (51 mg, 0.13 mmol), and diisopropylethylamine (0.07 mL, 0.41 mmol) were added dropwise and stirred at room temperature for 12 hours. The reaction mixture was concentrated in vacuo, and the residue was purified by column chromatography to obtain the target compound (32 mg, 73%).
$^1H$ NMR (500 MHz, DMSO-$d_6$) $\delta$ 9.89 (s, 1H), 8.17 (s, 1H), 7.77 (s, 1H), 7.15-7.09 (m, 3H), 6.21 (s, 1H), 5.02 (s, 1H), 4.31-4.30 (m, 2H), 3.87-3.85 (m, 2H), 2.35-2.21 (m, 12H), 1.24 (s, 9H)

**[Example 56] Preparation of N-(2-((8-((2,6-dimethylbenzyl)amino)-2,3-dimethylimidazo[1,2-a]pyridin-6-yl)amino)-2-oxoethyl)-2,2-difluoroacetamide**

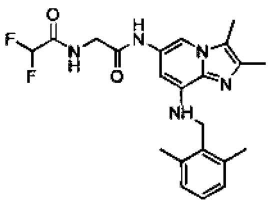

2-Amino-N-(8-((2,6-dimethylbenzyl)amino)-2,3-dimethylimidazo[1,2-a]pyridin-6-yl)acetamide (Example 10, 40 mg, 0.10 mmol) was dissolved in dichloromethane (2 mL). Difluoroacetic acid (11 mg, 0.11 mmol), 1-[bis(dimethylamino) methylene]-1H-1,2,3-triazolo[4,5-b]pyridinium 3-oxide hexafluorophosphate (51 mg, 0.13 mmol), and diisopropylethylamine (0.07 mL, 0.41 mmol) were added dropwise and stirred at room temperature for 12 hours. The reaction mixture was concentrated in vacuo, and the residue was purified by column chromatography to obtain the target compound (29 mg, 68%).
$^1$H NMR (500 MHz, DMSO-$d_6$) δ 10.05 (s, 1H), 8.97-8.95 (m, 1H), 8.16 (s, 1H), 7.15-7.08 (m, 3H), 6.22 (s, 1H), 5.03 (s, 1H), 4.31-4.30 (m, 2H), 3.97-3.96 (m, 2H), 2.35-2.28 (m, 12H), 1.83-1.79 (m, 1H)

**[Example 57] Preparation of N-(2-((8-((2,6-dimethylbenzyl)amino)-2,3-dimethylimidazo[1,2-a]pyridin-6-yl)amino)-2-oxoethyl)-2,2-difluoropropanamide**

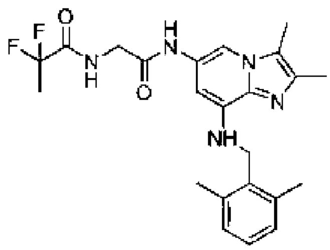

2-Amino-N-(8-((2,6-dimethylbenzyl)amino)-2,3-dimethylimidazo[1,2-a]pyridin-6-yl)acetamide (Example 10, 40 mg, 0.10 mmol) was dissolved in dichloromethane (2 mL). 2,2-Difluoropropionic acid (12 mg, 0.11 mmol), 1-[bis(dimethylamino)methylene]-1H-1,2,3-triazolo[4,5-b]pyridinium 3-oxide hexafluorophosphate (51 mg, 0.13 mmol), and diisopropylethylamine (0.07 mL, 0.41 mmol) were added dropwise and stirred at room temperature for 12 hours. The reaction mixture was concentrated in vacuo and the residue was purified by column chromatography to obtain the target compound (31 mg, 70%).
$^1$H NMR (500 MHz, DMSO-$d_6$) δ 10.06 (s, 1H), 9.15-9.12 (m, 1H), 8.16 (s, 1H), 7.16-7.09 (m, 3H), 6.44-6.24 (m, 1H), 5.06 (s, 1H), 4.31-4.30 (m, 2H), 4.01-4.00 (m, 2H), 2.69 (s, 3H), 2.35-2.28 (m, 12H)

**[Example 58] Preparation of N-(2-((8-((2,6-dimethylbenzyl)amino)-2,3-dimethylimidazo[1,2-a]pyridin-6-yl)amino)-2-oxoethyl)-3-hydroxycyclobutane-1-carboxamide**

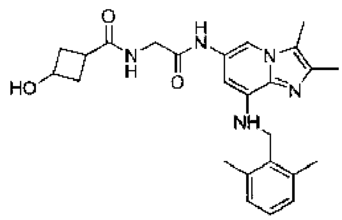

2-Amino-N-(8-((2,6-dimethylbenzyl)amino)-2,3-dimethylimidazo[1,2-a]pyridin-6-yl)acetamide (Example 10, 40 mg, 0.10 mmol) was dissolved in dichloromethane (2 mL). 3-Hydroxycyclobutanecarboxylic acid (12 mg, 0.11 mmol), 1-[bis(dimethylamino)methylene]-1H-1,2,3-triazolo[4,5-b]pyridinium 3-oxide hexafluorophosphate (51 mg, 0.13 mmol), and diisopropylethylamine (0.07 mL, 0.41 mmol) were added dropwise and stirred at room temperature for 12 hours. The reaction mixture was concentrated in vacuo, and the residue was purified by column chromatography to obtain the target compound (4 mg, 9%).
$^1$H NMR (500 MHz, DMSO-$d_6$) δ 10.06 (s, 1H), 8.25-8.09 (m, 2H), 7.17-7.10 (m, 3H), 6.42 (s, 1H), 5.11-5.09 (m, 1H), 4.30 (s, 2H), 3.92-3.90 (m, 2H), 2.30-2.28 (m, 12H), 1.99-1.97 (m, 3H), 1.40-1.35 (m, 2H), 1.29-1.20 (m, 2H)

**[Example 59] Preparation of N-(2-((8-((2,6-dimethylbenzyl)amino)-2,3-dimethylimidazo[1,2-a]pyridin-6-yl)amino)-2-oxoethyl)-3-fluorocyclobutane-1-carboxamide**

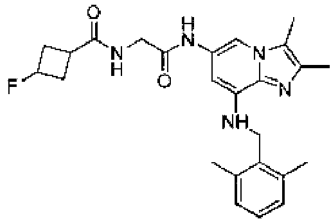

2-Amino-N-(8-((2,6-dimethylbenzyl)amino)-2,3-dimethylimidazo[1,2-a]pyridin-6-yl)acetamide (Example 10, 40 mg, 0.10 mmol) was dissolved in dichloromethane (2 mL). 3-Fluorocyclobutanecarboxylic acid (13 mg, 0.11 mmol), 1-[bis(dimethylamino)methylene]-1H-1,2,3-triazolo[4,5-b]pyridinium 3-oxide hexafluorophosphate (51 mg, 0.13 mmol), and diisopropylethylamine (0.07 mL, 0.41 mmol) were added dropwise and stirred at room temperature for 12 hours. The reaction mixture was concentrated in vacuo, and the residue was purified by column chromatography to obtain the target compound (23 mg, 51%).
$^1H$ NMR (500 MHz, DMSO-$d_6$) δ 9.95-9.93 (m, 1H), 8.28-8.27 (m, 1H), 8.15 (s, 1H), 7.15-7.08 (m, 3H), 6.23 (s, 1H), 5.04-5.01 (m, 1H), 4.30-4.29 (m, 2H), 3.91-3.90 (m, 2H), 2.28-2.21 (m, 12H), 1.26-1.24 (m, 6H)

**[Example 60] Preparation of 3-(8-((2,6-dimethylbenzyl)amino)-2,3-dimethylimidazo[1,2-a]pyridin-6-yl)imidazolidine-2,4-dione**

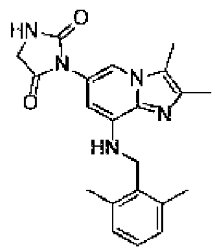

2-Amino-N-(8-((2,6-dimethylbenzyl)amino)-2,3-dimethylimidazo[1,2-a]pyridin-6-yl)acetamide (Example 10, 40 mg, 0.10 mmol) and 1,1'-carbonyldiimidazole (22 mg, 0.10 mmol) were dissolved in dichloromethane (2 mL) and stirred at room temperature for 1 hour. Triethylamine (0.06 mL, 0.45 mmol) and a 40% solution of methylamine diluted in methanol (8 mg, 0.26 mmol) were added and stirred at room temperature for 12 hours. The reaction mixture was concentrated in vacuo, and the residue was purified by column chromatography to obtain the target compound (10 mg, 26%).
$^1H$ NMR (500 MHz, DMSO-$d_6$) δ 8.33 (s, 1H), 7.62 (s, 1H), 7.15-7.13 (m, 2H), 7.09-7.07 (m, 1H), 6.21 (s, 1H), 5.08-5.07 (m, 1H), 4.30-4.29 (m, 2H), 4.09 (s, 2H), 2.34-2.32 (m, 9H), 2.25 (s, 3H)

**[Example 61] Preparation of N-(8-((2,6-dimethylbenzyl)amino)-2,3-dimethylimidazo[1,2-a]pyridin-6-yl)morpholine-4-carboxamide citrate**

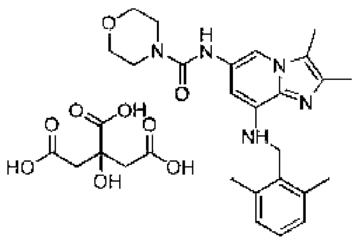

$N^8$-(2,6-Dimethylbenzyl)-2,3-dimethylimidazo[1,2-a]pyridine-6,8-diamine hydrochloride (Intermediate 1, 50 mg, 0.15 mmol) and 1,1'-carbonyldiimidazole (48 mg, 0.30 mmol) were dissolved in dichloromethane (0.7 mL) and stirred at room temperature for 1 hour. Morpholine (0.01 mL, 0.15 mmol) and triethylamine (0.06 mL, 0.45 mmol) were added and stirred at room temperature for 6 hours. The reaction mixture was purified by column chromatography to obtain the target compound (20 mg, 32%). This was then dissolved in acetone, citric acid (9 mg, 0.05 mmol) was added, and the

mixture was stirred at room temperature for 12 hours. The resulting solid was filtered and dried to obtain the citrate salt of the target compound.
$^1$H NMR (500 MHz, DMSO-$d_6$) δ 8.54 (s, 1H), 8.04 (s, 1H), 7.20-7.15 (m, 3H), 6.67-6.60 (m, 1H), 5.33-5.32 (m, 1H), 4.29-4.28 (m, 2H), 3.64-3.62 (m, 4H), 3.47-3.45 (m, 4H), 2.68-2.64 (m, 4H), 2.37-2.36 (m, 12H)

**[Example 62] Preparation of 2-(3-(8-((2,6-dimethylbenzyl)amino)-2,3-dimethylimidazo[1,2-a]pyridin-6-yl)ureido) ethyl acetate hydrochloride**

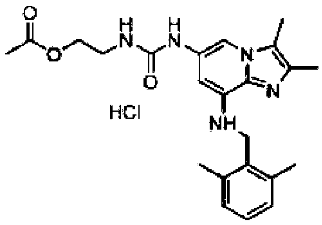

N$^8$-(2,6-Dimethylbenzyl)-2,3-dimethylimidazo[1,2-a]pyridine-6,8-diamine hydrochloride (Intermediate 1, 50 mg, 0.15 mmol) and 1,1'-carbonyldiimidazole (48 mg, 0.30 mmol) were dissolved in dichloromethane (0.7 mL) and stirred at room temperature for 1 hour. 2-Aminoethyl acetate (15 mg, 0.15 mmol) and triethylamine (0.06 mL, 0.45 mmol) were added and stirred at room temperature for 6 hours. The reaction was quenched by adding distilled water, extracted with dichloromethane, and the organic layer was dried over anhydrous magnesium sulfate, and concentrated under reduced pressure. The residue was purified by column chromatography, dissolved in ethyl acetate, and 4 N hydrochloric acid (0.3 mL) dissolved in 1,4-dioxane was added, followed by stirring at room temperature for 2 hours. The resulting solid was filtered and dried to obtain the target compound (10 mg, 15%).
$^1$H NMR (500 MHz, DMSO-$d_6$) δ 9.08 (s, 1H), 8.34-8.31 (m, 1H), 7.23-7.14 (m, 3H), 6.76-6.60 (m, 2H), 6.14 (s, 1H), 4.36-4.35 (m, 2H), 4.05-4.01 (m, 2H), 2.38-2.36 (m, 12H), 2.04-1.99 (m, 3H), 1.24-1.17 (m, 2H)

**[Example 63] Preparation of (S)-2-amino-N-(8-((4-fluoro-2,6-dimethylbenzyl)amino)-2,3-dimethylimidazo[1,2-a] pyridin-6-yl)propanamide hydrochloride**

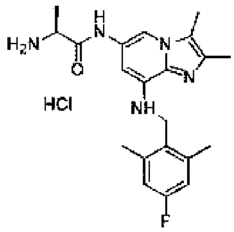

<u>Step 1: Preparation of tert-butyl (S)-(1-((8-((4-fluoro-2,6-dimethylbenzyl)amino)-2,3-dimethylimidazo[1,2-a]pyridin-6-yl) amino)-1-oxopropan-2-yl)carbamate</u>

N$^8$-(4-fluoro-2,6-dimethylbenzyl)-2,3-dimethylimidazo[1,2-a]pyridine-6,8-diamine hydrochloride (Intermediate 2, 50 mg, 0.14 mmol), tert-butyl (S)-(1-amino-1-oxopropan-2-yl)carbamate (29 mg, 0.15 mmol), 1-[bis(dimethylamino) methylene]-1H-1,2,3-triazolo[4,5-B]pyridinium 3-oxide hexafluorophosphate (69 mg, 0.18 mmol), and diisopropylethylamine (0.09 mL, 0.56 mmol) were dissolved in dichloromethane (0.7 mL) and stirred at room temperature for 12 hours. The reaction was terminated by adding distilled water, extracted with dichloromethane, and the organic layer was dried over anhydrous magnesium sulfate, then concentrated under reduced pressure. The residue was purified by column chromatography to obtain the target compound (28 mg, 44%).

<u>Step 2: Preparation of (S)-2-amino-N-(8-((4-fluoro-2,6-dimethylbenzyl)amino)-2,3-dimethylimidazo[1,2-a]pyridin-6-yl) propanamide hydrochloride</u>

Tert-butyl (R)-2-((8-((2,6-dimethylbenzyl)amino)-2,3-dimethylimidazo[1,2-a]pyridin-6-yl)carbamoyl)-2-methylazetidine-1-carboxylate (26 mg, 0.053 mmol) prepared in Step 1 was dissolved in ethyl acetate (0.25 mL), and 4 N hydrochloric acid (0.1 mL) diluted in 1,4-dioxane was added. The mixture was stirred at 50°C for 12 hours. The resulting solid was filtered and dried to obtain the target compound (10 mg, 45%).

$^1H$ NMR (500 MHz, DMSO-$d_6$) δ 11.32 (s, 1H), 8.44-8.37 (m, 4H), 7.02-7.00 (m, 3H), 4.28-4.27 (m, 2H), 4.05-4.01 (m, 1H), 2.41-2.40 (m, 12H), 1.53-1.51 (m, 3H)

**[Example 64] Preparation of 2-amino-N-(8-((4-fluoro-2,6-dimethylbenzyl)amino)-2,3-dimethylimidazo[1,2-a]pyridin-6-yl)acetamide hydrochloride**

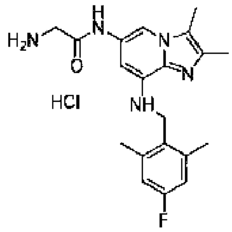

Step 1: Preparation of tert-butyl (2-((8-((4-fluoro-2,6-dimethylbenzyl)amino)-2,3-dimethylimidazo[1,2-a]pyridin-6-yl)amino)-2-oxoethyl)carbamate

$N^8$-(4-fluoro-2,6-dimethylbenzyl)-2,3-dimethylimidazo[1,2-a]pyridine-6,8-diamine hydrochloride (Intermediate 2, 50 mg, 0.14 mmol), (tert-butoxycarbonyl)glycine (30 mg, 0.17 mmol), 1-[bis(dimethylamino)methylene]-1H-1,2,3-triazolo[4,5-B]pyridinium 3-oxide hexafluorophosphate (69 mg, 0.18 mmol), and diisopropylethylamine (0.07 mL, 0.42 mmol) were dissolved in dichloromethane (0.7 mL) and stirred at room temperature for 12 hours. The reaction was terminated by adding distilled water, extracted with dichloromethane, and the organic layer was dried over anhydrous magnesium sulfate, then concentrated under reduced pressure. The residue was purified by column chromatography to obtain the target compound (28 mg, 44%).

Step 2: Preparation of 2-Amino-N-(8-((4-fluoro-2,6-dimethylbenzyl)amino)-2,3-dimethylimidazo[1,2-a]pyridin-6-yl)acetamide hydrochloride

Tert-butyl (2-((8-((4-fluoro-2,6-dimethylbenzyl)amino)-2,3-dimethylimidazo[1,2-a]pyridin-6-yl)amino)-2-oxoethyl)carbamate (28 mg, 0.06 mmol) prepared in Step 1 was dissolved in ethyl acetate (0.31 mL). 4 N hydrochloric acid (0.31 mL) diluted in 1,4-dioxane was added, and the mixture was stirred at 50°C for 16 hours. The resulting solid was filtered and dried to obtain the target compound (16 mg, 68%).
$^1H$ NMR (500 MHz, DMSO-$d_6$) δ 11.28 (s, 1H), 8.42-8.28 (m, 4H), 7.01-6.94 (m, 3H), 4.27 (s, 2H), 3.89 (s, 2H), 2.41-2.39 (m, 12H)

**[Example 65] Preparation of (S)-N-(8-((4-fluoro-2,6-dimethylbenzyl)amino)-2,3-dimethylimidazo[1,2-a]pyridin-6-yl)azetidine-2-carboxamide hydrochloride**

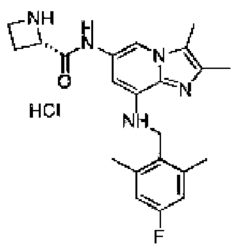

$N^8$-(4-Fluoro-2,6-dimethylbenzyl)-2,3-dimethylimidazo[1,2-a]pyridine-6,8-diamine hydrochloride (Intermediate 2, 50 mg, 0.14 mmol), (S)-1-(tert-butoxycarbonyl)azetidine-2-carboxylic acid (31 mg, 0.15 mmol), 1-[bis(dimethylamino)methylene]-1H-1,2,3-triazolo[4,5-B]pyridinium 3-oxide hexafluorophosphate (69 mg, 0.18 mmol), and diisopropylethylamine (0.09 mL, 0.56 mmol) were dissolved in dichloromethane (0.7 mL), and the mixture was stirred at room temperature for 12 hours. The reaction was quenched by adding distilled water, extracted with dichloromethane, and the organic layer was dried over anhydrous magnesium sulfate, then concentrated under reduced pressure. The residue was purified by column chromatography, dissolved in ethyl acetate, and 4 N hydrochloric acid (0.3 mL) diluted in 1,4-dioxane was added. The mixture was stirred at 50°C for 12 hours. The resulting solid was filtered and dried to obtain the target compound (26 mg, 38%).

$^1$H NMR (500 MHz, DMSO-$d_6$) δ 11.57-11.28 (m, 1H), 8.58-8.36 (m, 3H), 7.08-6.99 (m, 3H), 5.21 (s, 1H), 4.27-4.24 (m, 2H), 3.82-3.79 (m, 2H), 2.44-2.41 (m, 12H), 1.30-1.26 (m, 2H)

**[Example 66] Preparation of N-(8-((2,6-dimethylbenzyl)amino)-2,3-dimethylimidazo[1,2-a]pyridin-6-yl)-2-azaspiro[3.3]heptane-2-carboxamide citrate**

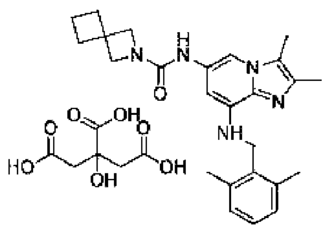

N$^8$-(2,6-Dimethylbenzyl)-2,3-dimethylimidazo[1,2-a]pyridine-6,8-diamine hydrochloride (Intermediate 1, 60 mg, 0.18 mmol) and 1,1'-carbonyldiimidazole (58 mg, 0.36 mmol) were dissolved in dichloromethane (2.57 mL) and stirred at room temperature for 1 hour. 2-Azaspiro[3.3]heptane (17 mg, 0.18 mmol) and triethylamine (0.07 mL, 0.54 mmol) were added and stirred at room temperature for 6 hours. The reaction mixture was purified by column chromatography, dissolved in acetone, and citric acid (34 mg, 0.18 mmol) was added and stirred at room temperature for 1 hour. The resulting solid was filtered and dried to obtain the target compound (33 mg, 30%).

$^1$H NMR (500 MHz, DMSO-$d_6$) δ 8.44 (s, 1H), 8.08 (s, 1H), 7.19-7.12 (m, 3H), 6.62 (s, 1H), 5.32-5.23 (m, 1H), 4.28-4.27 (m, 2H), 3.94 (s, 4H), 2.69-2.60 (m, 4H), 2.35-2.30 (m, 12H), 2.17-2.15 (m, 4H), 1.79-1.76 (m, 2H)

**[Example 67] Preparation of N-(8-((2,6-dimethylbenzyl)amino)-2,3-dimethylimidazo[1,2-a]pyridin-6-yl)isoindoline-2-carboxamide**

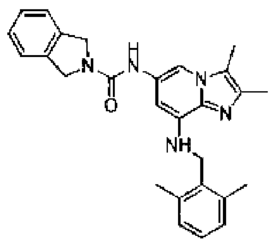

N$^8$-(2,6-Dimethylbenzyl)-2,3-dimethylimidazo[1,2-a]pyridine-6,8-diamine hydrochloride (Intermediate 1, 40 mg, 0.12 mmol) and 1,1'-carbonyldiimidazole (39 mg, 0.24 mmol) were dissolved in dichloromethane (2 mL) and stirred at room temperature for 1 hour. Triethylamine (0.05 mL, 0.36 mmol) and isoindole (14 mg, 0.12 mmol) were added and stirred at room temperature for 12 hours. The reaction mixture was concentrated in vacuo, and the residue was purified by column chromatography to obtain the target compound (11 mg, 21%).

$^1$H NMR (500 MHz, DMSO-$d_6$) δ 10.21 (s, 1H), 8.23 (s, 1H), 8.03 (s, 1H), 7.39-7.38 (m, 4H), 7.17-7.15 (m, 1H), 7.11-7.09 (m, 3H), 4.79 (s, 4H), 4.32-4.31 (m, 2H), 2.36-2.29 (m, 12H)

**[Example 68] Preparation of N-(8-((2,6-dimethylbenzyl)amino)-2,3-dimethylimidazo[1,2-a]pyridin-6-yl)-1,3-dihydro-2H-pyrrolo[3,4-c]pyridine-2-carboxamide**

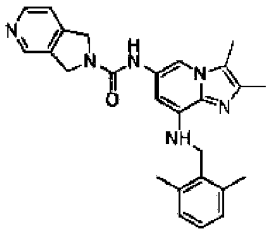

$N^8$-(2,6-Dimethylbenzyl)-2,3-dimethylimidazo[1,2-a]pyridine-6,8-diamine hydrochloride (Intermediate 1, 40 mg, 0.12 mmol) and 1,1'-carbonyldiimidazole (39 mg, 0.24 mmol) were dissolved in dichloromethane (2 mL) and stirred at room temperature for 1 hour. Triethylamine (0.05 mL, 0.36 mmol) and 2,3-dihydro-1H-pyrrolo[3,4-c]pyridine (15 mg, 0.12 mmol) were added and stirred at room temperature for 12 hours. The reaction mixture was concentrated in vacuo, and the residue was purified by column chromatography to obtain the target compound (10 mg, 19%).
$^1$H NMR (500 MHz, DMSO-$d_6$) δ 8.63-8.62 (m, 2H), 8.52-8.51 (m, 2H), 7.46-7.45 (m, 1H), 7.17-7.09 (m, 5H), 4.84-4.82 (m, 4H), 4.32-4.31 (m, 2H), 2.37-2.36 (m, 12H)

**[Example 69] Preparation of N-(8-((2,6-dimethylbenzyl)amino)-2,3-dimethylimidazo[1,2-a]pyridin-6-yl)-3-oxoazetidine-1-carboxamide citrate**

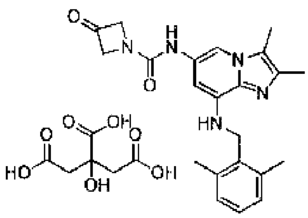

$N^8$-(2,6-Dimethylbenzyl)-2,3-dimethylimidazo[1,2-a]pyridine-6,8-diamine hydrochloride (Intermediate 1, 60 mg, 0.18 mmol) and 1,1'-carbonyldiimidazole (58 mg, 0.36 mmol) were dissolved in dichloromethane (0.9 mL) and stirred at room temperature for 1 hour. Azetidine-3-one hydrochloride (19 mg, 0.18 mmol) and triethylamine (0.07 mL, 0.54 mmol) were added and stirred at room temperature for 6 hours. The reaction mixture was purified by column chromatography, dissolved in acetone, and citric acid (34 mg, 0.18 mmol) was added and stirred at room temperature for 1 hour. The resulting solid was filtered and dried to obtain the target compound (30 mg, 43%).
$^1$H NMR (500 MHz, DMSO-$d_6$) δ 8.93 (s, 1H), 8.11 (s, 1H), 7.19-7.10 (m, 4H), 6.66-6.49 (m, 1H), 4.82 (s, 4H), 4.30-4.29 (m, 2H), 2.70-2.64 (m, 4H), 2.35-2.31 (m, 12H)

**[Example 70] Preparation of ((8-((2,6-dimethylbenzyl)amino)-2,3-dimethylimidazo[1,2-a]pyridin-6-yl)carbamoyl)glycine hydrochloride**

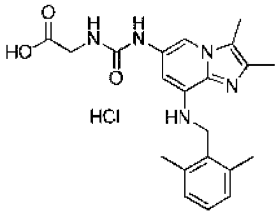

Step 1: Preparation of methyl((8-((2,6-dimethylbenzyl)amino)-2,3-dimethylimidazo[1,2-a]pyridin-6-yl)carbamoyl)glycinate

$N^8$-(2,6-dimethylbenzyl)-2,3-dimethylimidazo[1,2-a]pyridine-6,8-diamine hydrochloride (Intermediate 1, 100 mg, 0.30 mmol) and 1,1'-carbonyldiimidazole (98 mg, 0.61 mmol) were dissolved in dichloromethane (1.5 mL) and stirred at room temperature for 1 hour. Methyl glycinate (27 mg, 0.30 mmol) and triethylamine (0.13 mL, 0.91 mmol) were

added and stirred at room temperature for 12 hours. The reaction was terminated by adding distilled water, extracted with dichloromethane, and the organic layer was dried over anhydrous magnesium sulfate, then concentrated under reduced pressure. The residue was purified by column chromatography to obtain the target compound (32 mg, 26%).

Step 2: Preparation of ((8-((2,6-dimethylbenzyl)amino)-2,3-dimethylimidazo[1,2-a]pyridin-6-yl)carbamoyl)glycine hydrochloride

Methyl((8-((2,6-dimethylbenzyl)amino)-2,3-dimethylimidazo[1,2-a]pyridin-6-yl)carbamoyl)glycinate (32 mg, 0.079 mmol) prepared in Step 1 was dissolved in tetrahydrofuran (0.5 mL), methanol (0.5 mL), and distilled water (0.5 mL). Potassium hydroxide (22 mg, 0.40 mmol) was added, and the mixture was stirred at room temperature for 12 hours. The reaction mixture was acidified with 4 N aqueous hydrochloric acid, and the resulting solid was filtered and dried to obtain the target compound (16 mg, 46%).
$^1$H NMR (500 MHz, DMSO-$d_6$) δ 12.69-12.51 (brs, 1H), 8.67 (s, 1H), 7.96 (s, 1H), 7.21-7.09 (m, 3H), 6.41-6.40 (m, 1H), 6.09 (s, 1H), 4.31-4.30 (m, 2H), 3.80-3.79 (m, 2H), 2.35-2.27 (m, 12H)

**[Example 71] Preparation of 3-(3-(8-((2,6-dimethylbenzyl)amino)-2,3-dimethylimidazo[1,2-a]pyridin-6-yl)ureido) propionic acid citrate**

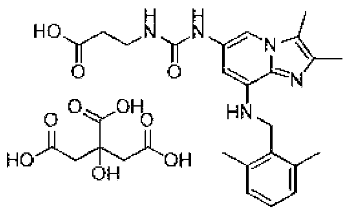

Step 1: Preparation of methyl 3-(3-(8-((2,6-dimethylbenzyl)amino)-2,3-dimethylimidazo[1,2-a]pyridin-6-yl)ureido)propanoate

$N^8$-(2,6-dimethylbenzyl)-2,3-dimethylimidazo[1,2-a]pyridine-6,8-diamine hydrochloride (Intermediate 1, 60 mg, 0.18 mmol) and 1,1'-carbonyldiimidazole (58 mg, 0.36 mmol) were dissolved in dichloromethane (2.5 mL) and stirred at room temperature for 1 hour. Methyl 3-aminopropanoate (18 mg, 0.18 mmol) and triethylamine (0.07 mL, 0.54 mmol) were added and stirred at room temperature for 6 hours. The reaction mixture was purified by column chromatography to obtain the target compound (18 mg, 26%).

Step 2: Preparation of 3-(3-(8-((2,6-dimethylbenzyl)amino)-2,3-dimethylimidazo[1,2-a]pyridin-6-yl)ureido)propionic acid citrate

Methyl 3-(3-(8-((2,6-dimethylbenzyl)amino)-2,3-dimethylimidazo[1,2-a]pyridin-6-yl)ureido)propanoate (30 mg, 0.07 mmol) was dissolved in tetrahydrofuran (0.3 mL), methanol (0.3 mL), and distilled water (0.3 mL). Potassium hydroxide (19 mg, 0.35 mmol) was added, and the mixture was stirred at room temperature for 12 hours. The solvent was removed, and the resulting solid was filtered and dried using dichloromethane to obtain the target compound (16 mg, 55%). This was then dissolved in acetone, citric acid (7 mg, 0.04 mmol) was added, and the mixture was stirred at room temperature for 12 hours. The resulting solid was filtered and dried to obtain the citrate salt of the target compound.
$^1$H NMR (500 MHz, DMSO-$d_6$) δ 12.31-12.03 (brs, 1H), 7.95-7.94 (m, 1H), 7.15-7.04 (m, 4H), 6.67-6.45 (m, 1H), 6.08-6.04 (m, 1H), 4.88-4.87 (m, 1H), 4.29-4.28 (m, 2H), 2.46-2.40 (m, 4H), 2.37-2.34 (m, 12H)

**[Example 72] Preparation of 3-(8-((2,6-dimethylbenzyl)amino)-2,3-dimethylimidazo[1,2-a]pyridin-6-yl)-1,3,5-triazepane-2,4-dione**

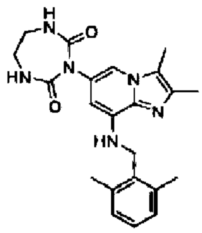

1-(2-Aminoethyl)-3-(8-((2,6-dimethylbenzyl)amino)-2,3-dimethylimidazo[1,2-a]pyridin-6-yl)urea hydrochloride (Example 99, 40 mg, 0.10 mmol) and 1,1'-carbonyldiimidazole (31 mg, 0.19 mmol) were dissolved in dichloromethane (2 mL) and stirred at room temperature for 1 hour. Triethylamine (0.04 mL, 0.29 mmol) and 40% methylamine solution (7 mg, 0.24 mmol) were added and stirred at room temperature for 12 hours. The reaction mixture was concentrated in vacuo, and the residue was purified by column chromatography to obtain the target compound (11 mg, 28%).
$^1$H NMR (500 MHz, DMSO-$d_6$) δ 10.31-10.21 (brs, 1H), 8.04-8.02 (m, 1H), 7.90 (s, 1H), 7.21-7.05 (m, 5H), 4.35-4.34 (m, 2H), 3.88-3.85 (m, 2H), 3.38-3.37 (m, 2H), 2.30-2.29 (m, 12H)

**[Example 73] Preparation of N-(2-(3-(8-((2,6-dimethylbenzyl)amino)-2,3-dimethylimidazo[1,2-a]pyridin-6-yl)ureido)ethyl)acetamide**

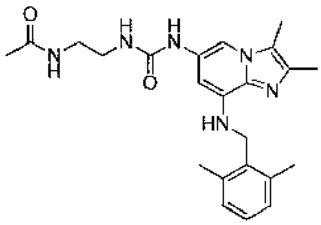

1-(2-Aminoethyl)-3-(8-((2,6-dimethylbenzyl)amino)-2,3-dimethylimidazo[1,2-a]pyridin-6-yl)urea hydrochloride (Example 99, 40 mg, 0.10 mmol) and triethylamine (0.03 mL, 0.19 mmol) were dissolved in dimethylformamide (2 mL). Acetic anhydride (0.01 mL, 0.14 mmol) was added and stirred at room temperature for 12 hours. The reaction mixture was concentrated in vacuo, and the residue was purified by column chromatography to obtain the target compound (13 mg, 32%).
$^1$H NMR (500 MHz, DMSO-$d_6$) δ 8.45 (s, 1H), 7.97-7.94 (m, 2H), 7.20-7.03 (m, 5H), 6.21-6.11 (m, 1H), 4.29 (s, 2H), 3.28-3.27 (m, 4H), 2.36-2.35 (m, 12H), 1.98 (s, 3H)

**[Example 74] Preparation of 1-(8-((2,6-dimethylbenzyl)amino)-2,3-dimethylimidazo[1,2-a]pyridin-6-yl)-3-((tetrahydrofuran-3-yl)methyl)urea hydrochloride**

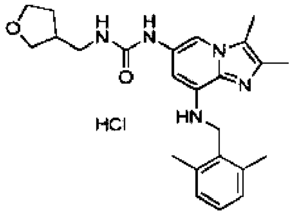

N$^8$-(2,6-Dimethylbenzyl)-2,3-dimethylimidazo[1,2-a]pyridine-6,8-diamine hydrochloride (Intermediate 1, 60 mg, 0.18 mmol) and 1,1'-carbonyldiimidazole (58 mg, 0.36 mmol) were dissolved in dichloromethane (2.5 mL) and stirred at room temperature for 1 hour. (Tetrahydrofuran-3-yl)methanamine (18 mg, 0.18 mmol) and triethylamine (0.07 mL, 0.54 mmol) were added and stirred at room temperature for 6 hours. The reaction was quenched by adding distilled water, extracted with dichloromethane, and the organic layer was dried over anhydrous magnesium sulfate, and concentrated under reduced pressure. The residue was purified by column chromatography, dissolved in ethyl acetate, and 4 N hydrochloric acid (0.3 mL) diluted in 1,4-dioxane was added, followed by stirring at room temperature for 6 hours.

The resulting solid was filtered and dried to obtain the target compound (14 mg, 18%).
$^1H$ NMR (500 MHz, DMSO-$d_6$) δ 13.48 (s, 1H), 8.83 (s, 1H), 8.17 (s, 1H), 7.18-7.11 (m, 3H), 6.59-6.49 (m, 2H), 4.30-4.29 (m, 2H), 3.76-3.74 (m, 3H), 3.44-3.41 (m, 1H), 3.13-3.10 (m, 2H), 2.38-2.34 (m, 12H), 1.99-1.95 (m, 1H)

**[Example 75] Preparation of (R)-N-(8-((2,6-dimethylbenzyl)amino)-2,3-dimethylimidazo[1,2-a]pyridin-6-yl)-2-methylazetidine-2-carboxamide hydrochloride**

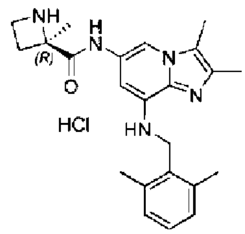

Step 1: Preparation of tert-butyl (R)-2-((8-((2,6-dimethylbenzyl)amino)-2,3-dimethylimidazo[1,2-a]pyridin-6-yl)carbamoyl)-2-methylazetidine-1-carboxylate

$N^8$-(2,6-dimethylbenzyl)-2,3-dimethylimidazo[1,2-a]pyridine-6,8-diamine hydrochloride (Intermediate 1, 50 mg, 0.15 mmol), (2R)-1-[(tert-butoxy)carbonyl]-2-methylazetidine-2-carboxylic acid (36 mg, 0.17 mmol), 1-[bis(dimethylamino)methylene]-1H-1,2,3-triazolo[4,5-B]pyridinium 3-oxide hexafluorophosphate (75 mg, 0.20 mmol), and diisopropylethylamine (0.11 mL, 0.61 mmol) were dissolved in dichloromethane (0.7 mL), and stirred at room temperature for 5 hours. The reaction was quenched by adding distilled water, extracted with dichloromethane, and the organic layer was dried over anhydrous magnesium sulfate, then concentrated under reduced pressure. The residue was purified by column chromatography, dissolved in ethyl acetate, and hexane was added to precipitate a solid, obtaining the target compound (27 mg, 37%).

Step 2: Preparation of (R)-N-(8-((2,6-dimethylbenzyl)amino)-2,3-dimethylimidazo[1,2-a]pyridin-6-yl)-2-methylazetidine-2-carboxamide hydrochloride

Tert-butyl (R)-2-((8-((2,6-dimethylbenzyl)amino)-2,3-dimethylimidazo[1,2-a]pyridin-6-yl)carbamoyl)-2-methylazetidine-1-carboxylate (27 mg, 0.056 mmol) prepared in Step 1 was dissolved in ethyl acetate (0.28 mL), and 2 M hydrochloric acid (0.11 mL) diluted in diethyl ether was added. The mixture was stirred at 50°C for 12 hours. The resulting solid was filtered and dried to obtain the target compound (19 mg, 78%).
$^1H$ NMR (500 MHz, DMSO-$d_6$) δ 14.09 (s, 1H), 11.26 (s, 1H), 9.68 (s, 1H), 9.13 (s, 1H), 8.50 (s, 1H), 7.30-7.13 (m, 4H), 6.53 (s, 1H), 4.31 (s, 2H), 4.00-3.71 (m, 2H), 2.88-2.86 (m, 1H), 2.36-2.34 (m, 12H), 1.99 (s, 3H)

**[Example 76] Preparation of (R)-2-(azetidin-2-yl)-N-(8-((2,6-dimethylbenzyl)amino)-2,3-dimethylimidazo[1,2-a]pyridin-6-yl)acetamide hydrochloride**

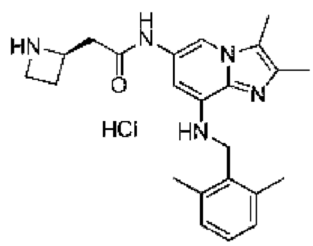

Step 1: Preparation of tert-butyl (R)-2-(2-((8-((2,6-dimethylbenzyl)amino)-2,3-dimethylimidazo[1,2-a]pyridin-6-yl)amino)-2-oxoethyl)azetidine-1-carboxylate

$N^8$-(2,6-dimethylbenzyl)-2,3-dimethylimidazo[1,2-a]pyridine-6,8-diamine hydrochloride (Intermediate 1, 50 mg,

0.15 mmol), (R)-2-(1-(tert-butoxycarbonyl)azetidin-2-yl)acetic acid (36 mg, 0.17 mmol), 1-[bis(dimethylamino)methylene]-1H-1,2,3-triazolo[4,5-B]pyridinium 3-oxide hexafluorophosphate (75 mg, 0.20 mmol), and diisopropylethylamine (0.11 mL, 0.61 mmol) were dissolved in dichloromethane (0.7 mL), and stirred at room temperature for 12 hours. The reaction was quenched by adding distilled water, extracted with dichloromethane, and the organic layer was dried over anhydrous magnesium sulfate, then concentrated under reduced pressure. The residue was purified by column chromatography, dissolved in ethyl acetate, and hexane was added to precipitate a solid to obtain the target compound (20 mg, 27%).

Step 2: Preparation of (R)-2-(azetidin-2-yl)-N-(8-((2,6-dimethylbenzyl)amino)-2,3-dimethylimidazo[1,2-a]pyridin-6-yl) acetamide hydrochloride

Tert-butyl (R)-2-(2-((8-((2,6-dimethylbenzyl)amino)-2,3-dimethylimidazo[1,2-a]pyridin-6-yl)amino)-2-oxoethyl) azetidine-1-carboxylate (20 mg, 0.041 mmol) prepared in Step 1 was dissolved in ethyl acetate (0.21 mL), and 2 M hydrochloric acid (0.08 mL) diluted in diethyl ether was added, followed by stirring at 50°C for 12 hours. The resulting solid was filtered and dried to obtain the target compound (14 mg, 81%).
$^1$H NMR (500 MHz, DMSO-$d_6$) δ 13.81 (s, 1H), 10.89-10.84 (m, 1H), 8.97-8.96 (m, 1H), 8.54-8.47 (m, 1H), 8.19 (s, 1H), 7.22-7.13 (m, 4H), 4.30 (s, 2H), 3.83-3.80 (m, 2H), 3.23-3.20 (m, 2H), 2.89-2.87 (m, 2H), 2.36-2.33 (m, 12H)

**[Example 77] Preparation of N-(8-((2,6-dimethylbenzyl)amino)-2,3-dimethylimidazo[1,2-a]pyridin-6-yl)-3-phenylazetidine-1-carboxamide citrate**

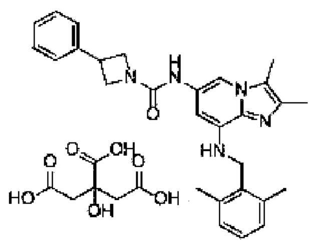

N$^8$-(2,6-Dimethylbenzyl)-2,3-dimethylimidazo[1,2-a]pyridine-6,8-diamine hydrochloride (Intermediate 1, 60 mg, 0.18 mmol) and 1,1'-carbonyldiimidazole (58 mg, 0.36 mmol) were dissolved in dichloromethane (0.9 mL) and stirred at room temperature for 1 hour. 3-Phenylazetidine (24 mg, 0.18 mmol) and triethylamine (0.07 mL, 0.54 mmol) were added and stirred at room temperature for 6 hours. The reaction mixture was purified by column chromatography, dissolved in acetone, and citric acid (34 mg, 0.18 mmol) was added and stirred at room temperature for 3 hours. The resulting solid was filtered and dried to obtain the target compound (29 mg, 25%).
$^1$H NMR (500 MHz, DMSO-$d_6$) δ 8.59 (s, 1H), 8.12 (s, 1H), 7.41-7.37 (m, 4H), 7.30-7.26 (m, 1H), 7.19-7.11 (m, 3H), 6.64 (s, 1H), 4.43-4.39 (m, 2H), 4.29-4.28 (m, 2H), 3.99-3.98 (m, 2H), 3.88-3.87 (m, 1H), 2.69-2.66 (m, 4H), 2.35-2.31 (m, 12H)

**[Example 78] Preparation of (S)-1-(2,3-dihydro-1H-inden-1-yl)-3-(8-((2,6-dimethylbenzyl)amino)-2,3-dimethylimidazo[1,2-a]pyridin-6-yl)urea hydrochloride**

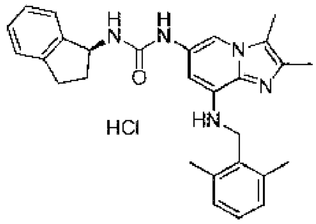

N$^8$-(2,6-Dimethylbenzyl)-2,3-dimethylimidazo[1,2-a]pyridine-6,8-diamine hydrochloride (intermediate 1, 60 mg, 0.18 mmol) and 1,1'-carbonyldiimidazole (58 mg, 0.36 mmol) were dissolved in dichloromethane (2.57 mL) and stirred at room temperature for 1 hour. (S)-2,3-dihydro-1H-inden-1-amine (23 mg, 0.18 mmol) and triethylamine (0.07 mL, 0.54 mmol) were added and stirred at room temperature for 6 hours. The reaction mixture was purified by column chromatography, dissolved in ethyl acetate, and 2 M hydrochloric acid diluted in diethyl ether (0.36 mL) was added and stirred at room temperature for 6 hours. The resulting solid was filtered and dried to obtain the target compound (16 mg, 22%).
$^1$H NMR (500 MHz, DMSO-d$_6$) δ 13.50 (s, 1H), 8.92 (s, 1H), 8.30 (s, 1H), 7.30-7.12 (m, 7H), 6.88 (s, 1H), 6.65-6.64 (m, 1H), 5.22-5.21 (m, 1H), 4.31-4.30 (m, 2H), 2.98-2.92 (m, 2H), 2.45-2.44 (m, 12H), 1.99 (s, 1H)

**[Example 79] Preparation of (S)-N2-(8-((2,6-dimethylbenzyl)amino)-2,3-dimethylimidazo[1,2-a]pyridin-6-yl)-N1-methylazetidine-1,2-dicarboxamide**

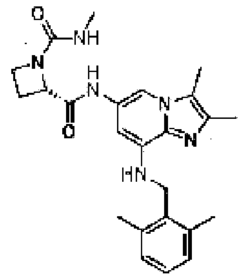

(S)-N-(8-((2,6-dimethylbenzyl)amino)-2,3-dimethylimidazo[1,2-a]pyridin-6-yl)azetidine-2-carboxamide (Example 65, 40 mg, 0.12 mmol) and 1,1'-carbonyldiimidazole (31 mg, 0.19 mmol) were dissolved in dichloromethane (2 mL) and stirred at room temperature for 1 hour. Triethylamine (0.04 mL, 0.29 mmol) and 40% methylamine solution (8 mg, 0.24 mmol) were added and stirred at room temperature for 12 hours. The reaction mixture was concentrated in vacuo, and the residue was purified by column chromatography to obtain the target compound (12 mg, 28%).
$^1$H NMR (500 MHz, DMSO-d$_6$) δ 8.01-7.95 (m, 1H), 7.18-7.14 (m, 4H), 4.61-4.58 (m, 1H), 4.32-4.28 (m, 2H), 3.95-3.92 (m, 1H), 3.18-3.16 (m, 1H), 2.66 (s, 3H), 2.35 (s, 6H), 2.31 (s, 3H), 2.28 (s, 3H), 2.16-2.13 (m, 2H)

**[Example 80] Preparation of (R)-1-(2,3-dihydro-1H-inden-1-yl)-3-(8-((2,6-dimethylbenzyl)amino)-2,3-dimethylimidazo[1,2-a]pyridin-6-yl)urea hydrochloride**

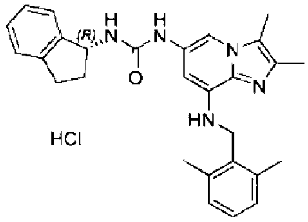

N$^8$-(2,6-Dimethylbenzyl)-2,3-dimethylimidazo[1,2-a]pyridine-6,8-diamine hydrochloride (Intermediate 1, 60 mg, 0.18 mmol) and 1,1'-carbonyldiimidazole (58 mg, 0.36 mmol) were dissolved in dichloromethane (0.9 mL) and stirred at room temperature for 1 hour. (R)-2,3-dihydro-1H-inden-1-amine (23 mg, 0.18 mmol) and triethylamine (0.07 mL, 0.54 mmol) were added and stirred at room temperature for 6 hours. The reaction mixture was purified by column chromatography, dissolved in ethyl acetate, and 2 M hydrochloric acid diluted in diethyl ether (0.36 mL) was added and stirred at room temperature for 10 hours. The resulting solid was filtered and dried to obtain the target compound (10 mg, 3%).
$^1$H NMR (500 MHz, DMSO-d$_6$) δ 8.99 (s, 1H), 8.37 (s, 1H), 7.30-7.28 (m, 2H), 7.26-7.23(m, 4H), 7.23-7.21 (m, 2H), 6.94-6.92 (d, 1H), 6.77 (s, 1H), 6.19 (s, 1H), 5.23-5.20 (m, 1H), 4.32-4.31 (d, 2H), 2.41-2.37 (m, 12H)

**[Example 81] Preparation of N-(8-((2,6-dimethylbenzyl)amino)-2,3-dimethylimidazo [1,2-a] pyridin-6-yl)-2-(2-oxoazetidin-1-yl)acetamide**

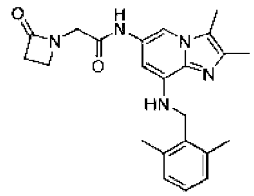

2-Amino-N-(8-((2,6-dimethylbenzyl)amino)-2,3-dimethylimidazo[1,2-a]pyridin-6-yl)acetamide (Example 10, 50 mg, 0.13 mmol) and triethylamine (0.05 mL, 0.39 mmol) were dissolved in tetrahydrofuran (2 mL). 3-Bromopropionyl chloride (24 mg, 0.14 mmol) was added and stirred at room temperature for 12 hours. The reaction mixture was concentrated in vacuo, and the residue was purified by column chromatography to obtain the target compound (17 mg, 32%).
$^1$H NMR (500 MHz, DMSO-$d_6$) δ 10.02 (s, 1H), 8.51 (s, 1H), 8.19 (s, 1H), 7.17-7.15 (m, 1H), 7.14-7.08 (m, 2H), 6.38-6.36 (m, 1H), 6.35-6.33 (m, 1H), 6.15-6.12 (d, 1H), 5.66-5.64 (d, 1H), 4.31-4.30 (d, 1H), 4.01-4.00 (d, 1H), 2.35 (s, 6H), 2.29 (s, 3H), 2.22 (s, 3H)

**[Example 82] Preparation of N-(8-((2,6-dimethylbenzyl)amino)-2,3-dimethylimidazo[1,2-a]pyridin-6-yl)-4-phenylpiperidine-1-carboxamide**

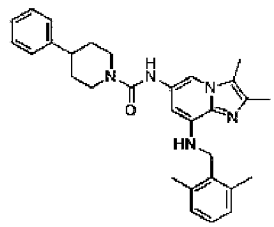

N$^8$-(2,6-Dimethylbenzyl)-2,3-dimethylimidazo[1,2-a]pyridine-6,8-diamine hydrochloride (Intermediate 1, 40 mg, 0.12 mmol) and 1,1'-carbonyldiimidazole (39 mg, 0.24 mmol) were dissolved in dichloromethane (2 mL) and stirred at room temperature for 1 hour. Triethylamine (0.05 mL, 0.36 mmol) and 4-methylpiperidine (19 mg, 0.12 mmol) were added and stirred at room temperature for 12 hours. The reaction mixture was concentrated in vacuo, and the residue was purified by column chromatography to obtain the target compound (12 mg, 21%).
$^1$H NMR (500 MHz, DMSO-$d_6$) δ 8.39 (s, 1H), 7.96 (s, 1H), 7.32-7.29 (m, 5H), 7.27-7.10 (m, 4H), 6.46 (s, 1H), 4.81 (s, 1H), 4.33-4.29 (m, 4H), 2.93-2.88 (t, 1H), 2.77-2.75 (m, 1H), 2.35 (s, 6H), 2.28(s, 3H), 2.22 (s, 3H), 1.84-1.82 (d, 2H), 1.61-1.59 (m, 2H)

**[Example 83] Preparation of N-(8-((2,6-dimethylbenzyl l) amino)-2,3-dimethylimidazo[1,2-a]pyridin-6-yl)-3-phenylpiperidine-1-carboxamide**

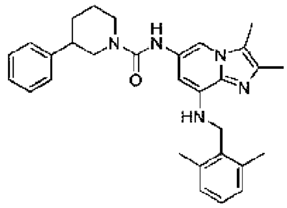

N$^8$-(2,6-Dimethylbenzyl)-2,3-dimethylimidazo[1,2-a]pyridine-6,8-diamine hydrochloride (Intermediate 1, 40 mg, 0.12 mmol) and 1,1'-carbonyldiimidazole (39 mg, 0.24 mmol) were dissolved in dichloromethane (2 mL) and stirred at room temperature for 1 hour. Triethylamine (0.05 mL, 0.36 mmol) and 3-phenylpyrrolidine (18 mg, 0.12 mmol) were added and stirred at room temperature for 12 hours. The reaction mixture was concentrated in vacuo, and the residue was purified by column chromatography to obtain the target compound (28 mg, 48%).
$^1$H NMR (500 MHz, DMSO-$d_6$) δ 8.37 (s, 1H), 7.95 (s, 1H), 7.35-7.33 (m, 5H), 7.26-7.25 (m, 1H), 7.15-7.14 (m, 1H), 7.10-7.08 (m, 2H), 6.43 (s, 1H), 4.78 (s, 1H), 4.27-4.23 (m, 4H), 2.93-2.84 (m, 2H), 2.71-2.69 (m, 1H), 2.34 (s, 6H), 2.27(s,

3H), 2.20 (s, 3H), 1.94-1.93 (m, 2H), 1.77-1.74 (m, 2H), 1.70-1.68 (m, 1H)

**[Example 84] Preparation of N-(8-((2,6-dimethylbenzyl)amino)-2,3-dimethylimidazo[1,2-a]pyridin-6-yl)-3-phenylpyrrolidine-1-carboxamide**

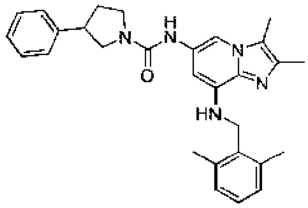

$N^8$-(2,6-Dimethylbenzyl)-2,3-dimethylimidazo[1,2-a]pyridine-6,8-diamine hydrochloride (Intermediate 1, 40 mg, 0.12 mmol) and 1,1'-carbonyldiimidazole (39 mg, 0.24 mmol) were dissolved in dichloromethane (2 mL) and stirred at room temperature for 1 hour. Triethylamine (0.05 mL, 0.36 mmol) and 3-phenylpyrrolidine (18 mg, 0.12 mmol) were added and stirred at room temperature for 12 hours. The reaction mixture was concentrated in vacuo, and the residue was purified by column chromatography to obtain the target compound (4 mg, 7%).
$^1$H NMR (500 MHz, DMSO-$d_6$) δ 8.05-7.99 (m, 2H), 7.36-7.35 (m, 5H), 7.27-7.26 (m, 1H), 7.17-7.16 (m, 1H), 7.10-7.09 (m, 2H), 6.48 (s, 1H), 4.78 (s, 1H), 4.29-4.28 ( m, 2H), 3.93-3.89 (t, 1H), 3.67-3.65 (m, 1H), 3.47-3.45 (m, 2H), 2.35 (s, 6H), 2.28, (s, 3H), 2.20 (s, 3H), 1.48-146 (m, 2H)

**[Example 85] Preparation of (R)-N-(8-((2,6-dimethylbenzyl)amino)-2,3-dimethylimidazo[1,2-a]pyridin-6-yl)piperazine-2-carboxamide hydrochloride**

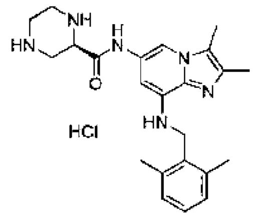

Step 1: Preparation of di-tert-butyl (R)-2-((8-((2,6-dimethylbenzyl)amino)-2,3-dimethylimidazo[1,2-a]pyridin-6-yl)carbamoyl)piperazine-1,4-dicarboxylate

$N^8$-(2,6-dimethylbenzyl)-2,3-dimethylimidazo[1,2-a]pyridine-6,8-diamine hydrochloride (Intermediate 1, 50 mg, 0.15 mmol), (R)-1,4-bis(tert-butoxycarbonyl)piperazine-2-carboxylic acid (55 mg, 0.17 mmol), 1-[bis(dimethylamino)methylene]-1H-1,2,3-triazolo[4,5-B]pyridinium 3-oxide hexafluorophosphate (75 mg, 0.20 mmol), and diisopropylethylamine (0.11 mL, 0.61 mmol) were dissolved in dichloromethane (0.7 mL), and stirred at room temperature for 12 hours. The reaction was quenched by adding distilled water, extracted with dichloromethane, and the organic layer was dried over anhydrous magnesium sulfate, then concentrated under reduced pressure. The residue was purified by column chromatography to obtain the target compound (91 mg, 99%).

Step 2: Preparation of (R)-N-(8-((2,6-dimethylbenzyl)amino)-2,3-dimethylimidazo[1,2-a]pyridin-6-yl)piperazine-2-carboxamide hydrochloride

Di-tert-butyl (R)-2-((8-((2,6-dimethylbenzyl)amino)-2,3-dimethylimidazo[1,2-a]pyridin-6-yl)carbamoyl)piperazine-1,4-dicarboxylate (91 mg, 0.15 mmol) prepared in Step 1 was dissolved in ethyl acetate (0.7 mL), 2 M hydrochloric acid diluted in diethyl ether (0.30 mL) was added, and the mixture was stirred at 50°C for 12 hours. The resulting solid was filtered and dried to obtain the target compound (63 mg, 95%).
$^1$H NMR (500 MHz, DMSO-$d_6$) δ 8.44 (s, 1H), 7.22-7.21 (m, 1H), 7.16-7.15 (m, 3H), 6.46 (s, 1H), 4.32-4.31 (m, 2H), 2.69 (s, 2H), 2.46-2.43 (m, 6H), 2.40-2.37 (m, 6H), 2.01-1.99 (m, 2H), 1.48-1.46 (m, 2H)

**[Example 86] Preparation of N-(8-((4-fluoro-2,6-dimethylbenzyl)amino)-2,3-dimethylimidazo[1,2-a]pyridin-6-yl)-2-hydroxyacetamide citrate**

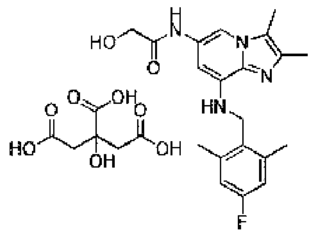

Step 1: Preparation of 2-((8-((4-fluoro-2,6-dimethylbenzyl)amino)-2,3-dimethylimidazo[1,2-a]pyridin-6-yl)amino)-2-oxoethyl acetate

$N^8$-(4-fluoro-2,6-dimethylbenzyl)-2,3-dimethylimidazo[1,2-a]pyridine-6,8-diamine hydrochloride (Intermediate 2, 80 mg, 0.22 mmol) and triethylamine (0.092 mL, 0.66 mmol) were dissolved in dichloromethane (3.14 mL) and stirred at room temperature for 10 minutes. 2-Chloro-2-oxoethyl acetate (0.032 mL, 0.29 mmol) was slowly added dropwise and stirred at room temperature for 12 hours. The reaction mixture was purified by column chromatography to obtain the target compound (18 mg, 20%).

Step 2: Preparation of N-(8-((4-fluoro-2,6-dimethylbenzyl)amino)-2,3-dimethylimidazo[1,2-a]pyridin-6-yl)-2-hydroxyacetamide citrate

2-((8-((4-fluoro-2,6-dimethylbenzyl)amino)-2,3-dimethylimidazo[1,2-a]pyridin-6-yl)amino)-2-oxoethyl acetate (50 mg, 0.12 mmol) prepared in Step 1 was dissolved in tetrahydrofuran (1 mL), methanol (1 mL), and distilled water (1 mL). Potassium hydroxide (33 mg, 0.60 mmol) was added, and the mixture was stirred at room temperature for 12 hours. Distilled water was added to quench the reaction, and the mixture was extracted with ethyl acetate. The organic layer was dried over anhydrous magnesium sulfate, and concentreated under reduced pressure. The residue was purified by column chromatography to obtain the target compound (20 mg, 45%). This was then dissolved in acetone, citric acid (10 mg, 0.05 mmol) was added, and the mixture was stirred at room temperature for 12 hours. The resulting solid was filtered and dried to obtain the citrate salt of the target compound.
$^1$H NMR (500 MHz, DMSO-$d_6$) $\delta$ 9.67 (s, 1H), 8.33 (s, 1H), 6.98-6.96 (d, 2H), 6.74-6.67 (m, 1H), 5.80-5.77 (m, 1H), 5.33-5.31 (m, 1H), 4.28-4.27 (d, 2H), 4.03-4.03 (d, 2H), 2.71-2.59 (m, 6H), 2.36-2.26 (m, 12H)

**[Example 87] Preparation of 1-(2-aminoethyl)-3-(8-((2,6-dimethylbenzyl)amino)-2,3-dimethylimidazo[1,2-a]pyridin-6-yl)-1-methylurea hydrochloride**

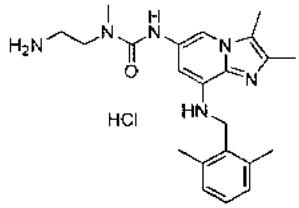

Step 1: Preparation of tert-butyl (2-(3-(8-((2,6-dimethylbenzyl)amino)-2,3-dimethylimidazo[1,2-a]pyridin-6-yl)ureido)ethyl)carbamate

$N^8$-(2,6-dimethylbenzyl)-2,3-dimethylimidazo[1,2-a]pyridine-6,8-diamine hydrochloride (Intermediate 1, 60.0 mg, 0.18 mmol) was dissolved in dichloromethane. 1,1'-Carbonyldiimidazole (58.0 mg, 0.36 mmol), triethylamine (0.07 mL, 0.54 mmol), and tert-butyl (2-(methylamino)ethyl)carbamate (30.0 mg, 0.18 mmol) were added, and the mixture was stirred at room temperature for 16 hours. When the reaction was complete, dichloromethane (50 mL) and distilled water (50 mL) were added, the layers were separated, the organic layer was recovered, dried over anhydrous

magnesium sulfate, and filtered. The filtrate was concentrated under reduced pressure and purified by column chromatography to obtain the target compound (22 mg, 23%).

Step 2: Preparation of 1-(2-aminoethyl)-3-(8-((2,6-dimethylbenzyl)amino)-2,3-dimethylimidazo[1,2-a]pyridin-6-yl)-1-methylurea hydrochloride

Tert-butyl (2-(3-(8-((2,6-dimethylbenzyl)amino)-2,3-dimethylimidazo[1,2-a]pyridin-6-yl)ureido)ethyl)carbamate (15.0 mg, 0.03 mmol) prepared in Step 1 was dissolved in ethyl acetate. 4 N hydrochloric acid (0.6 mL, 0.5 M) dissolved in 1,4-dioxane was added and stirred at room temperature for 12 hours. After completion of the reaction, the residue was filtered to obtain the target compound (12 mg, 78%).
$^1$H NMR (500 MHz, DMSO-d$_6$) $\delta$ 8.83 (s, 1H), 8.39 (s, 1H), 8.01 (s, 3H), 7.41 (s, 1H), 7.21-7.14 (m, 3H), 6.26 (s, 1H), 4.31-4.30 (d, 2H), 3.63-3.61 (t, 1H), 3.07 (s, 3H), 3.03-3.02 (m, 2H), 2.39-2.37 (m, 12H)

**[Example 88] Preparation of 1-(8-((2,6-dimethylbenzyl)amino)-2,3-dimethylimidazo[1,2-a]pyridin-6-yl)-3-(2-hydroxyethyl)urea citrate**

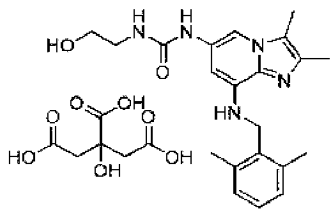

Step 1: Preparation of 2-(3-(8-((2,6-dimethylbenzyl)amino)-2,3-dimethylimidazo[1,2-a]pyridin-6-yl)ureido)ethyl acetate

N$^8$-(2,6-dimethylbenzyl)-2,3-dimethylimidazo[1,2-a]pyridine-6,8-diamine hydrochloride (Intermediate 1, 80 mg, 0.24 mmol) and 1,1'-carbonyldiimidazole (77 mg, 0.48 mmol) were dissolved in dichloromethane (3.4 mL) and stirred at room temperature for 1 hour. 2-Aminoethyl acetate hydrochloride (33 mg, 0.24 mmol) and triethylamine (0.1 mL, 0.72 mmol) were added and stirred at room temperature for 6 hours. The reaction mixture was purified by column chromatography to obtain the target compound (33 mg, 30%).

Step 2: Preparation of 1-(8-((2,6-dimethylbenzyl)amino)-2,3-dimethylimidazo[1,2-a]pyridin-6-yl)-3-(2-hydroxyethyl) urea citrate

2-(3-(8-((2,6-dimethylbenzyl)amino)-2,3-dimethylimidazo[1,2-a]pyridin-6-yl)ureido)ethyl acetate (100 mg, 0.23 mmol) prepared in Step 1 was dissolved in tetrahydrofuran (1.15 mL), methanol (1.15 mL), and distilled water (1.15 mL). Potassium hydroxide (66 mg, 1.18 mmol) was added, and the mixture was stirred at room temperature for 12 hours. The solvent was removed, distilled water was added, and the reaction was quenched. The mixture was then extracted with ethyl acetate. The organic layer was dried over anhydrous magnesium sulfate, concentrated under reduced pressure, and the target compound (70 mg, 80%) was obtained. This was then dissolved in acetone, citric acid (35 mg, 0.18 mmol) was added, and the mixture was stirred at room temperature for 12 hours. The resulting solid was filtered and dried to obtain the citrate salt of the target compound.
$^1$H NMR (500 MHz, DMSO-d$_6$) $\delta$ 8.06 (s, 1H), 7.21-7.11 (m, 3H), 6.28 (s, 1H), 4.77-4.75 (m, 1H), 4.31-4.30 (d, 2H), 3.47-3.46 (m, 2H), 3.18-3.17 (m, 2H), 2.69-2.64 (m, 4H), 2.36-2.26 (m, 12H), 2.01-2.00 (m, 2H)

**[Example 89] Preparation of (R)-N-(8-((2,6-dimethylbenzyl)amino)-2,3-dimethylimidazo[1,2-a]pyridin-6-yl)pyrrolidine-3-carboxamide hydrochloride**

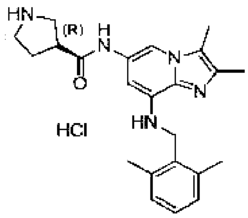

Step 1: Preparation of tert-butyl (R)-3-((8-((2,6-dimethylbenzyl)amino)-2,3-dimethylimidazo[1,2-a]pyridin-6-yl)carbamoyl)pyrrolidine-1-carboxylate

$N^8$-(2,6-dimethylbenzyl)-2,3-dimethylimidazo[1,2-a]pyridine-6,8-diamine hydrochloride (Intermediate 1, 50 mg, 0.15 mmol), (R)-1-Boc-3-pyrrolidinecarboxylic acid (36 mg, 0.17 mmol), 1-[bis(dimethylamino)methylene]-1H-1,2,3-triazolo[4,5-B]pyridinium 3-oxide hexafluorophosphate (75 mg, 0.20 mmol), and diisopropylethylamine (0.11 mL, 0.61 mmol) were dissolved in dichloromethane (0.7 mL) and stirred at room temperature for 12 hours. The reaction was quenched by adding distilled water, extracted with dichloromethane, and the organic layer was dried over anhydrous magnesium sulfate, then concentrated under reduced pressure. The residue was purified by column chromatography to obtain the target compound (73 mg, 99%).

Step 2: Preparation of (R)-N-(8-((2,6-dimethylbenzyl)amino)-2,3-dimethylimidazo[1,2-a]pyridin-6-yl)pyrrolidine-3-carboxamide hydrochloride

Tert-butyl (R)-3-((8-((2,6-dimethylbenzyl)amino)-2,3-dimethylimidazo[1,2-a]pyridin-6-yl)carbamoyl)pyrrolidine-1-carboxylate (73 mg, 0.15 mmol) prepared in Step 1 was dissolved in ethyl acetate (0.7 mL). 2 M hydrochloric acid (0.30 mL) diluted in diethyl ether was added, and the mixture was stirred at 50°C for 12 hours. The resulting solid was filtered and dried to obtain the target compound (55 mg, 85%).
$^1H$ NMR (500 MHz, DMSO-$d_6$) δ 14.06 (s, 1H), 11.09 (s, 1H), 9.43-9.27 (m, 2H), 8.52 (sm 1H), 7.22-7.13 (m, 4H), 6.51 (s, 1H), 4.31-4.3 (d, 1H), 3.28-3.27 (m, 2H), 2.40-2.37 (m, 12H), 2.11-2.08 (m, 2H)

**[Example 90] Preparation of (R)-N-(8-((2,6-dimethylbenzyl)amino)-2,3-dimethylimidazo[1,2-a]pyridin-6-yl)piperidine-2-carboxamide hydrochloride**

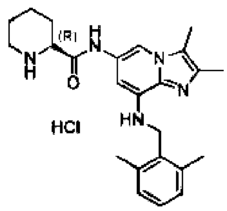

Step 1: Preparation of tert-butyl (R)-2-((8-((2,6-dimethylbenzyl)amino)-2,3-dimethylimidazo[1,2-a]pyridin-6-yl)carbamoyl)piperidine-1-carboxylate

$N^8$-(2,6-dimethylbenzyl)-2,3-dimethylimidazo[1,2-a]pyridine-6,8-diamine hydrochloride (Intermediate 1, 50 mg, 0.15 mmol), (R)-(+)-N-Boc-2-piperidinecarboxylic acid (38 mg, 0.17 mmol), 1-[bis(dimethylamino)methylene]-1H-1,2,3-triazolo[4,5-B]pyridinium 3-oxide hexafluorophosphate (75 mg, 0.20 mmol), and diisopropylethylamine (0.11 mL, 0.61 mmol) were dissolved in dichloromethane (0.7 mL) and stirred at room temperature for 12 hours. The reaction was quenched by adding distilled water, extracted with dichloromethane, and the organic layer was dried over anhydrous magnesium sulfate, then concentrated under reduced pressure. The residue was purified by column chromatography to obtain the target compound (75 mg, 98%).

Step 2: Preparation of (R)-N-(8-((2,6-dimethylbenzyl)amino)-2,3-dimethylimidazo[1,2-a]pyridin-6-yl)piperidine-2-carboxamide hydrochloride

Tert-butyl (R)-2-((8-((2,6-dimethylbenzyl)amino)-2,3-dimethylimidazo[1,2-a]pyridin-6-yl)carbamoyl)piperidine-1-carboxylate (75 mg, 0.15 mmol) prepared in Step 1 was dissolved in ethyl acetate (0.7 mL). 2 M hydrochloric

acid (0.30 mL) diluted in diethyl ether was added, and the mixture was stirred at 50°C for 12 hours. The resulting solid was filtered and dried to obtain the target compound (55 mg, 83%).
$^1$H NMR (500 MHz, DMSO-$d_6$) δ 13.86 (s, 1H), 11.28 (s, 1H), 9.31-9.29 (m, 1H), 8.89-8.87 (m, 1H), 8.44 (s, 1H), 7.23-7.16 (m, 4H), 6.44 (s, 1H), 4.31-4.30 (d, 2H), 4.05-4.01 (m, 1H), 3.06-3.02 (m, 2H), 2.42-2.37 (m, 14H), 2.01-2.00 (m, 2H), 1.88-1.76 (m, 2H), 1.69-1.59 (m, 4H), 1.47-1.45 (m, 2H)

**[Example 91] Preparation of (R)-N-(8-((2,6-dimethylbenzyl)amino)-2,3-dimethylimidazo[1,2-a]pyridin-6-yl)-2-(pyrrolidin-2-yl)acetamide hydrochloride**

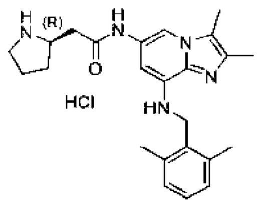

Step 1: Preparation of tert-butyl (R)-2-(2-((8-((2,6-dimethylbenzyl)amino)-2,3-dimethylimidazo[1,2-a]pyridin-6-yl)amino)-2-oxoethyl)pyrrolidine-1-carboxylate

N$^8$-(2,6-dimethylbenzyl)-2,3-dimethylimidazo[1,2-a]pyridine-6,8-diamine hydrochloride (Intermediate 1, 50 mg, 0.15 mmol), (R)-2-(1-(tert-butoxycarbonyl)pyrrolidin-2-yl)acetic acid (38 mg, 0.17 mmol), 1-[bis(dimethylamino)methylene]-1H-1,2,3-triazolo[4,5-B]pyridinium 3-oxide hexafluorophosphate (75 mg, 0.20 mmol), and diisopropylethylamine (0.11 mL, 0.61 mmol) were dissolved in dichloromethane (0.7 mL) and stirred at room temperature for 12 hours. The reaction was quenched by adding distilled water, extracted with dichloromethane, and the organic layer was dried over anhydrous magnesium sulfate, then concentrated under reduced pressure. The residue was purified by column chromatography to obtain the target compound (76 mg, 99%).

Step 2: Preparation of (R)-N-(8-((2,6-dimethylbenzyl)amino)-2,3-dimethylimidazo[1,2-a]pyridin-6-yl)-2-(pyrrolidin-2-yl) acetamide hydrochloride

Tert-butyl (R)-2-(2-((8-((2,6-dimethylbenzyl)amino)-2,3-dimethylimidazo[1,2-a]pyridin-6-yl)amino)-2-oxoethyl) pyrrolidine-1-carboxylate (76 mg, 0.15 mmol) prepared in Step 1 was dissolved in ethyl acetate (0.7 mL), and 2 M hydrochloric acid (0.30 mL) diluted in diethyl ether was added. The mixture was stirred at 50°C for 12 hours. The resulting solid was filtered and dried to obtain the target compound (54 mg, 82%).
$^1$H NMR (500 MHz, DMSO-$d_6$) δ 14.12 (s, 1H), 11.01 (s, 1H), 9.30-9.20 (m, 2H), 8.52 (s, 1H), 7.21-7.07 (m, 4H), 6.55 (s, 1H), 4.31 (s, 1H), 3.82-3.80 (m, 1H), 3.19-3.18 (m, 2H), 3.03-2.99 (m, 2H), 2.40-2.37 (m, 12H), 2.18-2.17 (m, 1H), 1.99-1.87 (m, 2H), 1.66-1.64 (m, 1H)

**[Example 92] Preparation of N$^8$-(2,6-dimethylbenzyl)-N$^6$-isopropyl-2,3-dimethylimidazo [1,2-a] pyridine-6,8-diamine**

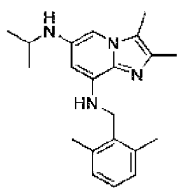

N$^8$-(2,6-Dimethylbenzyl)-2,3-dimethylimidazo[1,2-a]pyridine-6,8-diamine hydrochloride (Intermediate 1, 50 mg, 0.15 mmol), acetone (0.016 mL, 0.22 mmol), and acetic acid (0.012 mL, 0.22 mmol) were dissolved in tetrahydrofuran (2.16 mL), and the mixture was stirred at room temperature for 2 hours. Sodium triacetoxyborohydride (46 mg, 0.22 mmol) was slowly added, and the mixture was stirred at room temperature for 12 hours. The reaction was quenched by adding aqueous sodium bicarbonate solution, extracted with ethyl acetate, and the organic layer was dried over anhydrous

magnesium sulfate, concentrated under reduced pressure, and the target compound (8 mg, 16%) was obtained.
$^1$H NMR (500 MHz, DMSO-$d_6$) δ 8.47-8.45 (d, 1H), 7.15-7.13 (m, 2H), 7.10-7.09 (m, 2H), 6.65 (s, 1H), 5.50-5.49 (m, 1H), 4.48-4.47 (d, 2H), 2.76 (s, 3H), 2.55 (s, 3H), 2.27-2.01 (m, 12H), 2.00-1.98 (m, 1H)

**[Example 93] Preparation of (S)-N-(8-((2,6-dimethylbenzyl)amino)-2,3-dimethylimidazo[1,2-a]pyridin-6-yl)-2-(pyrrolidin-2-yl)acetamide hydrochloride**

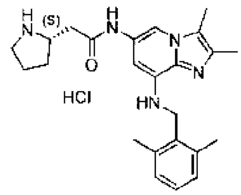

Step 1: Preparation of tert-butyl (S)-2-(2-((8-((2,6-dimethylbenzyl)amino)-2,3-dimethylimidazo[1,2-a]pyridin-6-yl)amino)-2-oxoethyl)pyrrolidine-1-carboxylate

N$^8$-(2,6-dimethylbenzyl)-2,3-dimethylimidazo[1,2-a]pyridine-6,8-diamine hydrochloride (Intermediate 1, 50 mg, 0.15 mmol), (S)-2-(1-Boc-2-pyrrolidinyl)acetic acid (38 mg, 0.17 mmol), 1-[bis(dimethylamino)methylene]-1H-1,2,3-triazolo[4,5-B]pyridinium 3-oxide hexafluorophosphate (75 mg, 0.20 mmol), and diisopropylethylamine (0.11 mL, 0.61 mmol) were dissolved in dichloromethane (0.7 mL) and stirred at room temperature for 12 hours. The reaction was quenched by adding distilled water, extracted with dichloromethane, and the organic layer was dried over anhydrous magnesium sulfate, then concentrated under reduced pressure. The residue was purified by column chromatography to obtain the target compound (75 mg, 98%).

Step 2: Preparation of (S)-N-(8-((2,6-dimethylbenzyl)amino)-2,3-dimethylimidazo[1,2-a]pyridin-6-yl)-2-(pyrrolidin-2-yl)acetamide hydrochloride

Tert-butyl (S)-2-(2-((8-((2,6-dimethylbenzyl)amino)-2,3-dimethylimidazo[1,2-a]pyridin-6-yl)amino)-2-oxoethyl)pyrrolidine-1-carboxylate (75 mg, 0.15 mmol) prepared in Step 1 was dissolved in ethyl acetate (0.7 mL), and 2 M hydrochloric acid (0.30 mL) diluted in diethyl ether was added. The mixture was stirred at 50°C for 12 hours. The resulting solid was filtered and dried to obtain the target compound (52 mg, 79%).
$^1$H NMR (500 MHz, DMSO-$d_6$) δ 13.90 (s, 1H), 10.90 (s, 1H), 9.20-9.03 (m, 2H), 8.50 (s, 1H), 7.23-7.21 (m, 1H), 7.15-7.14 (m, 2H), 7.00 (s, 1H), 4.31-4.30 (d, 2H), 3.83-3.80 (m, 2H), 2.40-2.37 (m, 12H), 2.18-2.16 (m, 1H), 1.99-90 (m, 2H), 1.66-1.63 (m, 1H)

**[Example 94] Preparation of (S)-N-(8-((2,6-dimethylbenzyl)amino)-2,3-dimethylimidazo[1,2-a]pyridin-6-yl)piperazine-2-carboxamide hydrochloride**

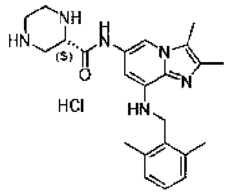

Step 1: Preparation of di-tert-butyl (S)-2-((8-((2,6-dimethylbenzyl)amino)-2,3-dimethylimidazo[1,2-a]pyridin-6-yl)carbamoyl)piperazine-1,4-dicarboxylate

N$^8$-(2,6-dimethylbenzyl)-2,3-dimethylimidazo[1,2-a]pyridine-6,8-diamine hydrochloride (Intermediate 1, 50 mg, 0.15 mmol), (S)-1,4-bis(tert-butoxycarbonyl)piperazine-2-carboxylic acid (55 mg, 0.17 mmol), 1-[bis(dimethylamino)methylene]-1H-1,2,3-triazolo[4,5-B]pyridinium 3-oxide hexafluorophosphate (75 mg, 0.20 mmol), and diisopropylethylamine (0.11 mL, 0.61 mmol) were dissolved in dichloromethane (0.7 mL), and stirred at room temperature for 12 hours.

The reaction was quenched by adding distilled water, extracted with dichloromethane, and the organic layer was dried over anhydrous magnesium sulfate, then concentrated under reduced pressure. The residue was purified by column chromatography to obtain the target compound (91 mg, 99%).

Step 2: Preparation of (S)-N-(8-((2,6-dimethylbenzyl)amino)-2,3-dimethylimidazo[1,2-a]pyridin-6-yl)piperazine-2-carboxamide hydrochloride

Di-tert-butyl (S)-2-((8-((2,6-dimethylbenzyl)amino)-2,3-dimethylimidazo[1,2-a]pyridin-6-yl)carbamoyl)piperazine-1,4-dicarboxylate (91 mg, 0.15 mmol) prepared in Step 1 was dissolved in ethyl acetate (0.7 mL), 2 M hydrochloric acid diluted in diethyl ether (0.30 mL) was added, and the mixture was stirred at 50°C for 12 hours. The resulting solid was filtered and dried to obtain the target compound (61 mg, 92%).
$^1$H NMR (500 MHz, DMSO-$d_6$) δ 8.44 (s, 1H), 7.23-7.14 (m, 4H), 6.46 (s, 1H), 4.45-4.42 (m, 1H), 4.32-4.31 (d, 2H), 2.69 (s, 3H), 2.43-2.42 (m, 12H), 2.01-1.99 (m, 2H), 1.48-14.6 (m, 2H)

**[Example 95] Preparation of N-(8-((2,6-dimethylbenzyl)amino)-2,3-dimethylimidazo[1,2-a]pyridin-6-yl)-2-(2-hydroxyacetamido)acetamide**

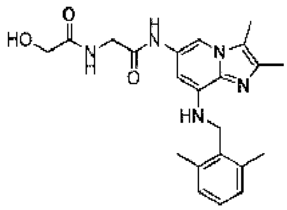

2-Amino-N-(8-((2,6-dimethylbenzyl)amino)-2,3-dimethylimidazo[1,2-a]pyridin-6-yl)acetamide (Example 10, 40 mg, 0.12 mmol) was dissolved in tetrahydrofuran (1 mL), methanol (1 mL), and distilled water (1 mL). Potassium hydroxide (11 mg, 0.19 mmol) was added, and the mixture was stirred at room temperature for 12 hours. The reaction was quenched by adding 1 N aqueous hydrochloric acid solution, and the pH of the mixture was maintained at 2. After extraction with ethyl acetate, the organic layer was dried over anhydrous magnesium sulfate and concentrated under reduced pressure. The concentrated residue was dried to obtain the target compound (9 mg, 58%).
$^1$H NMR (500 MHz, DMSO-$d_6$) δ 9.96 (s, 1H), 8.16 (s, 1H), 8.03-8.01 (m, 1H),7.17-7.14 (m, 1H), 7.12-7.09 (m, 3H), 6.26 (s, 1H), 5.01 (s, 1H), 4.31-4.30 (d, 2H), 3.97-3.96 (d, 2H), 3.79 (s, 2H), 2.35 (s, 6H), 2.28-2.21 (m, 6H)

**[Example 96] Preparation of (S)-N-(8-((2,6-dimethylbenzyl)amino)-2,3-dimethylimidazo[1,2-a]pyridin-6-yl)-2-hydroxypropanamide citrate**

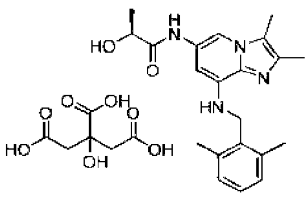

Step 1: Preparation of (S)-1-((8-((2,6-dimethylbenzyl)amino)-2,3-dimethylimidazo[1,2-a]pyridin-6-yl)amino)-1-oxopropan-2-yl acetate

$N^8$-(2,6-dimethylbenzyl)-2,3-dimethylimidazo[1,2-a]pyridine-6,8-diamine hydrochloride (Intermediate 1, 80 mg, 0.24 mmol), (S)-2-acetoxypropionic acid (35 mg, 0.26 mmol), 1-[bis(dimethylamino)methylene]-1H-1,2,3-triazolo[4,5-B]pyridinium 3-oxide hexafluorophosphate (118 mg, 0.31 mmol), and diisopropylethylamine (0.12 mL, 0.72 mmol) was dissolved in dichloromethane (3.42 mL) and stirred at room temperature for 11 hours. The reaction was quenched by adding distilled water, extracted with dichloromethane, and the organic layer was dried over anhydrous magnesium sulfate, then concentrated under reduced pressure. The residue was purified by column chromatography to obtain the target compound (39 mg, 40%).

Step 2: Preparation of (S)-N-(8-((2,6-dimethylbenzyl)amino)-2,3-dimethylimidazo[1,2-a]pyridin-6-yl)-2-hydroxypropanamide citrate

(S)-1-((8-((2,6-dimethylbenzyl)amino)-2,3-dimethylimidazo[1,2-a]pyridin-6-yl)amino)-1-oxopropan-2-yl acetate (39 mg, 0.09 mmol) prepared in Step 1 was dissolved in tetrahydrofuran (0.45 mL), methanol (0.45 mL), and distilled water (0.45 mL). Potassium hydroxide (26 mg, 0.47 mmol) was added, and the mixture was stirred at room temperature for 16 hours. The solvent was removed, distilled water was added, and the reaction was quenched. The mixture was then extracted with ethyl acetate. The organic layer was dried over anhydrous magnesium sulfate, then concentrated under reduced pressure to obtain the target compound (20 mg, 61%). This was then dissolved in acetone, citric acid (10 mg, 0.054 mmol) was added, and the mixture was stirred at room temperature for 12 hours. The resulting solid was filtered and dried to obtain the citrate salt of the target compound.
$^1$H NMR (500 MHz, DMSO-$d_6$) δ 9.67 (s, 1H), 8.38 (s, 1H), 7.21-7.19 (m, 1H), 7.17-7.16 (m, 2H), 6.80-6.67 (m, 1H), 5.87-5.86 (m, 1H), 5.34-5.32 (m, 1H), 4.31-4.30 (d, 2H), 4.20-4.19 (m, 1H), 2.71-2.58 (m, 4H), 2.35-2.26 (m, 12H), 1.35-1.33 (d, 3H)

**[Example 97] Preparation of (R)-N-(8-((2,6-dimethylbenzyl)amino)-2,3-dimethylimidazo[1,2-a]pyridin-6-yl)-2-hydroxypropanamide citrate**

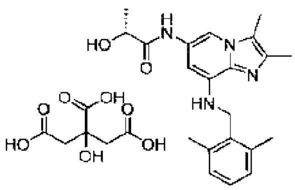

Step 1: Preparation of (R)-1-((8-((2,6-dimethylbenzyl)amino)-2,3-dimethylimidazo[1,2-a]pyridin-6-yl)amino)-1-oxopropan-2-yl acetate

N[8]-(2,6-Dimethylbenzyl)-2,3-dimethylimidazo[1,2-a]pyridine-6,8-diamine hydrochloride (intermediate 1, 80 mg, 0.24 mmol), (R)-2-acetoxypropionic acid (35 mg, 0.26 mmol), 1-[bis(dimethylamino)methylene]-1H-1,2,3-triazolo[4,5-B]pyridinium 3-oxide hexafluorophosphate (118 mg, 0.31 mmol), and diisopropylethylamine (0.12 mL, 0.72 mmol) were dissolved in dichloromethane (3.42 mL) and stirred at room temperature for 16 hours. The reaction was quenched by adding distilled water, extracted with dichloromethane, and the organic layer was dried over anhydrous magnesium sulfate, then concentrated under reduced pressure. The residue was purified by column chromatography to obtain the target compound (40 mg, 40%).

Step 2: Preparation of (R)-N-(8-((2,6-dimethylbenzyl)amino)-2,3-dimethylimidazo[1,2-a]pyridin-6-yl)-2-hydroxypropanamide citrate

(R)-1-((8-((2,6-dimethylbenzyl)amino)-2,3-dimethylimidazo[1,2-a]pyridin-6-yl)amino)-1-oxopropan-2-yl acetate (40 mg, 0.09 mmol) prepared in Step 1 was dissolved in tetrahydrofuran (0.9 mL), methanol (0.9 mL), and distilled water (0.9 mL). Potassium hydroxide (26 mg, 0.47 mmol) was added, and the mixture was stirred at room temperature for 16 hours. The solvent was removed, distilled water was added, and the reaction was quenched. The mixture was then extracted with ethyl acetate. The organic layer was dried over anhydrous magnesium sulfate, concentrated under reduced pressure, dissolved in dichloromethane, and diethyl ether was added to precipitate a solid to obtain the target compound (20 mg, 60%). This was then dissolved in acetone, citric acid (10 mg, 0.054 mmol) was added, and the mixture was stirred at room temperature for 16 hours. The resulting solid was filtered and dried to obtain the citrate salt of the target compound.
$^1$H NMR (500 MHz, DMSO-$d_6$) δ 9.68 (s, 1H), 8.39 (s, 1H), 7.21-7.12 (m, 3H), 6.81-6.74 (m, 1H), 5.87-5.86 (d, 2H), 4.20-4.19 (m, 1H), 2.71-2.62 (m, 4H), 2.35-2.27 (m, 12H), 2.09-2.07 (m, 2H), 1.35-1.33 (d, 3H)

**[Example 98] Preparation of N-(8-((2,6-dimethylbenzyl)amino)-2,3-dimethylimidazo[1,2-a]pyridin-6-yl)-2-(methylamino)acetamide hydrochloride**

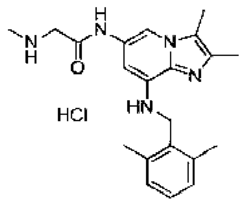

Step 1: Preparation of tert-butyl (2-((8-((2,6-dimethylbenzyl)amino)-2,3-dimethylimidazo[1,2-a]pyridin-6-yl)amino)-2-oxoethyl)(methyl)carbamate

$N^8$-(2,6-dimethylbenzyl)-2,3-dimethylimidazo[1,2-a]pyridine-6,8-diamine hydrochloride (intermediate 1, 50 mg, 0.15 mmol), N-(tert-butoxycarbonyl)-N-methylglycine (31 mg, 0.17 mmol), 1-[bis(dimethylamino)methylene]-1H-1,2,3-triazolo[4,5-B]pyridinium 3-oxide hexafluorophosphate (75 mg, 0.20 mmol), and diisopropylethylamine (0.11 mL, 0.61 mmol) were dissolved in dichloromethane (0.7 mL) and stirred at room temperature for 12 hours. The reaction was quenched by adding distilled water, extracted with dichloromethane, and the organic layer was dried over anhydrous magnesium sulfate, then concentrated under reduced pressure. The residue was purified by column chromatography to obtain the target compound (70 mg, 99%).

Step 2: Preparation of N-(8-((2,6-dimethylbenzyl)amino)-2,3-dimethylimidazo[1,2-alpyridin-6-yl)-2-(methylamino)acetamide hydrochloride

Tert-butyl (2-((8-((2,6-dimethylbenzyl)amino)-2,3-dimethylimidazo[1,2-a]pyridin-6-yl)amino)-2-oxoethyl)(methyl)carbamate (70 mg, 0.15 mmol) prepared in Step 1 was dissolved in ethyl acetate (0.7 mL). 2 M hydrochloric acid (0.30 mL) diluted in diethyl ether was added, and the mixture was stirred at 50°C for 12 hours. The resulting solid was filtered and dried to obtain the target compound (42 mg, 69%).
$^1$H NMR (500 MHz, DMSO-$d_6$) δ 11.42 (s, 1H), 9.16 (s, 1H), 8.41 (s, 1H), 7.22-7.18 (m, 1H), 7.13-7.11 (m, 2H), 6.99 (s, 1H), 6.53-6.52 (m, 1H), 4.31-4.30 (d, 2H), 4.04-4.03 (m, 2H), 2.66 (s, 2H), 2.41-2.37 (m, 12H)

**[Example 99] Preparation of 3-amino-N-(8-((2,6-dimethylbenzyl)amino)-2,3-dimethylimidazo[1,2-a]pyridin-6-yl)propanamide hydrochloride**

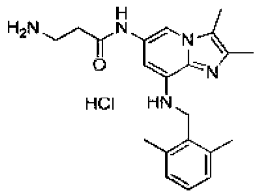

Step 1: Preparation of tert-butyl (3-((8-((2,6-dimethylbenzyl)amino)-2,3-dimethylimidazo[1,2-a]pyridin-6-yl)amino)-3-oxopropyl)carbamate

$N^8$-(2,6-dimethylbenzyl)-2,3-dimethylimidazo[1,2-a]pyridine-6,8-diamine hydrochloride (Intermediate 1, 50 mg, 0.15 mmol), Boc-beta-alanine (31 mg, 0.17 mmol), 1-[bis(dimethylamino)methylene]-1H-1,2,3-triazolo[4,5-B]pyridinium 3-oxide hexafluorophosphate (75 mg, 0.20 mmol), and diisopropylethylamine (0.11 mL, 0.61 mmol) were dissolved in dichloromethane (0.7 mL), and the mixture was stirred at room temperature for 12 hours. The reaction was quenched by adding distilled water, extracted with dichloromethane, and the organic layer was dried over anhydrous magnesium sulfate, then concentrated under reduced pressure. The residue was purified by column chromatography to obtain the target compound (70 mg, 99%).

Step 2: Preparation of 3-amino-N-(8-((2,6-dimethylbenzyl)amino)-2,3-dimethylimidazo[1,2-a]pyridin-6-yl)propanamide hydrochloride

Tert-butyl (3-((8-((2,6-dimethylbenzyl)amino)-2,3-dimethylimidazo[1,2-a]pyridin-6-yl)amino)-3-oxopropyl)carbamate (70 mg, 0.15 mmol) prepared in Step 1 was dissolved in ethyl acetate (0.7 mL). 2 M hydrochloric acid (0.30 mL) diluted in diethyl ether was added, and the mixture was stirred at 50°C for 12 hours. The resulting solid was filtered and

dried to obtain the target compound (40 mg, 67%).
$^1$H NMR (500 MHz, DMSO-$d_6$) δ 10.88 (s, 1H), 8.49 (s, 1H), 8.01 (s, 1H), 7.21-7.13 (m, 3H), 7.01 (s, 1H), 6.46 (s, 1H), 4.31-4.30 (d, 2H), 3.12-3.09 (m, 2H), 2.86-2.83 (m, 2H), 2.40-2.37 (m, 12H)

**[Example 100] Preparation of N-(8-((4-fluoro-2,6-dimethylbenzyl)amino)-2,3-dimethylimidazo[1,2-a]pyridin-6-yl)-2-methoxyacetamide citrate**

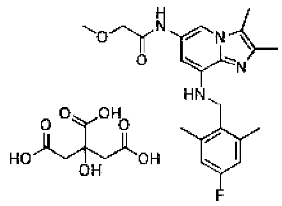

N$^8$-(4-Fluoro-2,6-dimethylbenzyl)-2,3-dimethylimidazo[1,2-a]pyridine-6,8-diamine hydrochloride (Intermediate 2, 80 mg, 0.22 mmol) and triethylamine (0.092 mL, 0.66 mmol) were dissolved in dichloromethane (3.14 mL) and stirred at room temperature for 5 minutes. 2-Methoxyacetyl chloride (0.023 mL, 0.25 mmol) was slowly added dropwise and stirred at room temperature for 16 hours. The reaction was quenched by adding distilled water, extracted with dichloromethane, and the organic layer was dried over anhydrous magnesium sulfate, and concentrated under reduced pressure. The residue was purified by column chromatography to obtain the target compound (20 mg, 23%). After this, it was dissolved in acetone, citric acid (10 mg, 0.05 mmol) was added, and the mixture was stirred at room temperature for 12 hours. The resulting solid was filtered and dried to obtain the citrate salt of the target compound.
$^1$H NMR (500 MHz, DMSO-$d_6$) δ 9.76 (s, 1H), 8.28 (s, 1H), 7.21 (s, 1H), 6.98-6.96 (d, 2H), 6.67 (s, 1H), 5.34-5.33 (m, 1H), 4.27-4.26 (d, 2H), 4.03 (s, 2H), 3.41 (s, 3H), 2.72-2.60 (m, 4H), 2.32-2.26 (m, 12H)

**[Example 101] Preparation of N-(8-((4-fluoro-2,6-dimethylbenzyl)amino)-2,3-dimethylimidazo[1,2-a]pyridin-6-yl)-3-hydroxypropanamide citrate**

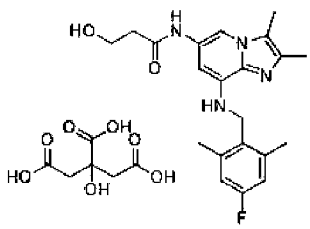

Step 1: Preparation of 3-((8-((4-fluoro-2,6-dimethylbenzyl)amino)-2,3-dimethylimidazo[1,2-a]pyridin-6-yl)amino)-3-oxopropyl acetate

N$^8$-(4-fluoro-2,6-dimethylbenzyl)-2,3-dimethylimidazo[1,2-a]pyridine-6,8-diamine hydrochloride (Intermediate 2, 80 mg, 0.22 mmol), 3-acetoxypyropionic acid (31 mg, 0.24 mmol), 1-[bis(dimethylamino)methylene]-1H-1,2,3-triazolo [4,5-B]pyridinium 3-oxide hexafluorophosphate (108 mg, 0.28 mmol), and diisopropylethylamine (0.15 mL, 0.88 mmol) were dissolved in dichloromethane (3.1 mL) and stirred at room temperature for 12 hours. The reaction was quenched by adding distilled water, extracted with dichloromethane, and the organic layer was dried over anhydrous magnesium sulfate, then concentrated under reduced pressure. The residue was purified by column chromatography to obtain the target compound (20 mg, 22%).

Step 2: Preparation of N-(8-((4-fluoro-2,6-dimethylbenzyl)amino)-2,3-dimethylimidazo[1,2-a]pyridin-6-yl)-3-hydroxypropanamide citrate

3-((8-((4-fluoro-2,6-dimethylbenzyl)amino)-2,3-dimethylimidazo[1,2-a]pyridin-6-yl)amino)-3-oxopropyl acetate (20 mg, 0.046 mmol) prepared in Step 1 was dissolved in tetrahydrofuran (0.22 mL), methanol (0.22 mL), and distilled

water (0.22 mL). Potassium hydroxide (13 mg, 0.23 mmol) was added, and the mixture was stirred at room temperature for 16 hours. The solvent was removed, distilled water was added, and the reaction was quenched. The mixture was extracted with dichloromethane. The organic layer was dried over anhydrous magnesium sulfate, concentrated under reduced pressure, and the residue was purified by column chromatography to obtain the target compound (10 mg, 56%). This was dissolved in acetone, citric acid (5 mg, 0.025 mmol) was added, and the mixture was stirred at room temperature for 12 hours. The resulting solid was filtered and dried to obtain the citrate salt of the target compound.
$^1$H NMR (500 MHz, DMSO-$d_6$) δ 9.90 (s, 1H), 8.28 (s, 1H), 6.97-6.95 (d, 2H), 6.32 (s, 1H), 5.20 (s, 1H), 4.26-4.25 (d, 2H), 3.73-3.72 (m, 2H), 2.64-2.58 (m, 4H), 2.36-2.23 (m, 12H)

**[Example 102] Preparation of N-(8-((2,6-dimethylbenzyl)amino)-2,3-dimethylimidazo[1,2-a]pyridin-6-yl)-3-hydroxypropanamide citrate**

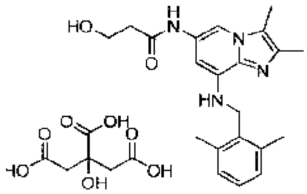

Step 1: Preparation of 3-((8-((2,6-dimethylbenzyl)amino)-2,3-dimethylimidazo[1,2-a]pyridin-6-yl)amino)-3-oxopropyl acetate

N$^8$-(2,6-dimethylbenzyl)-2,3-dimethylimidazo[1,2-a]pyridine-6,8-diamine hydrochloride (Intermediate 1, 1.0 g, 3.03 mmol), 3-acetoxypyropionic acid (0.33 mL, 3.33 mmol), 1-[bis(dimethylamino)methylene]-1H-1,2,3-triazolo[4,5-B] pyridinium 3-oxide hexafluorophosphate (1.49 g, 3.93 mmol), and diisopropylethylamine (1.5 mL, 9.09 mmol) were dissolved in dichloromethane (30 mL), and stirred at room temperature for 16 hours. Distilled water was added to quench the reaction, extraction was performed with dichloromethane, and the organic layer was dried over anhydrous magnesium sulfate, then concentrated under reduced pressure. The residue was purified by column chromatography, dissolved in dichloromethane, and diethyl ether was added to precipitate a solid, obtaining the target compound (0.6 g, 49%).

Step 2: Preparation of N-(8-((2,6-dimethylbenzyl)amino)-2,3-dimethylimidazo[1,2-a]pyridin-6-yl)-3-hydroxypropanamide citrate

3-((8-((2,6-dimethylbenzyl)amino)-2,3-dimethylimidazo[1,2-a]pyridin-6-yl)amino)-3-oxopropyl acetate (600 mg, 1.47 mmol) prepared in Step 1 was dissolved in tetrahydrofuran (4.9 mL), methanol (4.9 mL), and distilled water (4.9 mL). Potassium hydroxide (411 mg, 7.35 mmol) was added, and the mixture was stirred at room temperature for 16 hours. The solvent was removed, distilled water was added, and the reaction was quenched. The mixture was extracted with ethyl acetate. The organic layer was dried over anhydrous magnesium sulfate, concentrated under reduced pressure, and the mixture was stirred for 16 hours. The residue was precipitated by adding dichloromethane to obtain a solid, obtaining the target compound (238 mg, 44%). This was then dissolved in acetone, citric acid (124 mg, 0.65 mmol) was added, and the mixture was stirred at room temperature for 16 hours. The resulting solid was filtered and dried to obtain the citrate salt of the target compound.
$^1$H NMR (500 MHz, DMSO-$d_6$) δ 9.93 (s, 1H), 8.31 (s, 1H), 7.18-7.11 (m, 3H), 6.37 (s, 1H), 5.33-5.32 (m, 1H), 4.72-4.71 (d, 2H), 3.74-3.73 (m, 2H), 2.68-2.56 (m, 4H), 2.35-2.24 (m, 12H)

**[Example 103] Preparation of N-(8-((2,6-dimethylbenzyl)amino)-2,3-dimethylimidazo[1,2-a]pyridin-6-yl)piperazine-1-carboxamide hydrochloride**

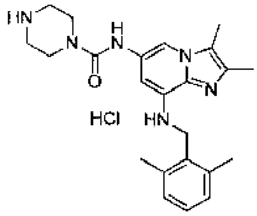

$N^8$-(2,6-Dimethylbenzyl)-2,3-dimethylimidazo[1,2-a]pyridine-6,8-diamine hydrochloride (Intermediate 1, 70 mg, 0.21 mmol) and 1,1'-carbonyldiimidazole (68 mg, 0.42 mmol) were dissolved in dichloromethane (1.05 mL) and stirred at room temperature for 1 hour. Tert-Butyl piperazine-1-carboxylate (39 mg, 0.21 mmol) and triethylamine (0.08 mL, 0.63 mmol) were added and stirred at room temperature for 6 hours. The reaction was quenched by adding distilled water, extracted with dichloromethane, and the organic layer was dried over anhydrous magnesium sulfate, and concentrated under reduced pressure. The residue was purified by column chromatography, dissolved in ethyl acetate, and 4 N hydrochloric acid (0.42 mL) diluted in 1,4-dioxane was added. The mixture was stirred at room temperature for 6 hours. The resulting solid was filtered and dried to obtain the target compound (19 mg, 20%).
$^1$H NMR (500 MHz, DMSO-$d_6$) δ 9.37-9.33 (m, 2H), 8.33 (s, 1H), 7.28-13 (m, 4H), 6.34 (s, 1H), 4.31-4.30 (d, 2H), 3.80-3.78 (m, 4H), 3.17-3.16 (m, 4H), 2.39-2.37 (m, 12H)

**[Example 104] Preparation of N-(8-((2,6-dimethylbenzyl)amino)-2,3-dimethylimidazo[1,2-a]pyridin-6-yl)benzamide citrate**

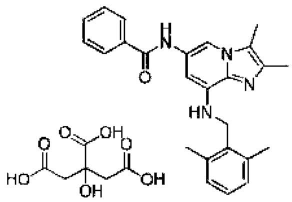

$N^8$-(2,6-Dimethylbenzyl)-2,3-dimethylimidazo[1,2-a]pyridine-6,8-diamine hydrochloride (Intermediate 1, 80 mg, 0.24 mmol), benzoic acid (29 mg, 0.24 mmol), 1-[bis(dimethylamino)methylene]-1H-1,2,3-triazolo[4,5-B]pyridinium 3-oxide hexafluorophosphate (118 mg, 0.31 mmol), and diisopropylethylamine (0.12 mL, 0.72 mmol) were dissolved in dichloromethane (3.4 mL) and stirred at room temperature for 12 hours. Distilled water was added to quench the reaction, and the mixture was extracted with dichloromethane. The organic layer was dried over anhydrous magnesium sulfate, and concentrated under reduced pressure. The residue was purified by column chromatography, dissolved in acetone, and citric acid (11 mg, 0.06 mmol) was added. The mixture was stirred at room temperature for 12 hours. The resulting solid was filtered and dried to obtain the target compound (20 mg, 14%).
$^1$H NMR (500 MHz, DMSO-$d_6$) δ 10.41 (s, 1H), 8.56 (s, 1H), 8.03-8.01 (d, 2H), 7.66-7.57 (m, 3H), 7.22-7.13 (m, 4H), 4.35-4.34 (d, 2H), 2.75-2.63 (m, 4H), 2.40-2.34 (m, 12H)

**[Example 105] Preparation of (S)-N-(8-((2,6-dimethylbenzyl)amino)-2,3-dimethylimidazo[1,2-a]pyridin-6-yl)pyrrolidine-3-carboxamide hydrochloride**

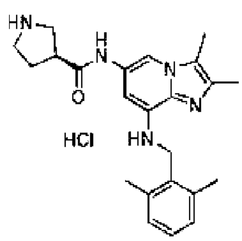

Step 1: Preparation of tert-butyl (S)-3-((8-((2,6-dimethylbenzyl)amino)-2,3-dimethylimidazo[1,2-a]pyridin-6-yl)carbamoyl)pyrrolidine-1-carboxylate

$N^8$-(2,6-dimethylbenzyl)-2,3-dimethylimidazo[1,2-a]pyridine-6,8-diamine hydrochloride (Intermediate 1, 50 mg, 0.15 mmol), (S)-1-Boc-3-pyrrolidinecarboxylic acid (36 mg, 0.17 mmol), 1-[bis(dimethylamino)methylene]-1H-1,2,3-triazolo[4,5-B]pyridinium 3-oxide hexafluorophosphate (75 mg, 0.20 mmol), and diisopropylethylamine (0.11 mL, 0.61 mmol) were dissolved in dichloromethane (0.7 mL) and stirred at room temperature for 12 hours. The reaction was quenched by adding distilled water, extracted with dichloromethane, and the organic layer was dried over anhydrous magnesium sulfate, then concentrated under reduced pressure. The residue was purified by column chromatography to obtain the target compound (72 mg, 96%).

Step 2: Preparation of (S)-N-(8-((2,6-dimethylbenzyl)amino)-2,3-dimethylimidazo[1,2-a]pyridin-6-yl)pyrrolidine-3-carboxamide hydrochloride

Tert-butyl (S)-3-((8-((2,6-dimethylbenzyl)amino)-2,3-dimethylimidazo[1,2-a]pyridin-6-yl)carbamoyl)pyrrolidine-1-carboxylate (72 mg, 0.15 mmol) prepared in Step 1 was dissolved in ethyl acetate (0.73 mL), 2 M hydrochloric acid (0.29 mL) diluted in diethyl ether was added, and the mixture was stirred at 50°C for 12 hours. The resulting solid was filtered and dried to obtain the target compound (52 mg, 83%).
$^1$H NMR (500 MHz, DMSO-$d_6$) δ 10.91-10.74 (m, 1H), 9.19-9.11 (m, 2H), 8.50-8.42 (m, 1H), 7.22-7.14 (m, 4H), 5.34-5.32 (m, 1H), 4.31-4.30 (d, 2H), 2.43-2.40 (m, 12H), 2.38-2.37 (m, 2H), 2.09-2.01 (m, 4H)

**[Example 106] Preparation of (S)-N-(8-((2,6-dimethylbenzyl)amino)-2,3-dimethylimidazo[1,2-a]pyridin-6-yl)piperidine-2-carboxamide hydrochloride**

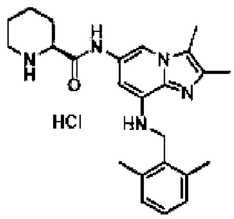

Step 1: Preparation of tert-butyl (S)-2-((8-((2,6-dimethylbenzyl)amino)-2,3-dimethylimidazo[1,2-a]pyridin-6-yl)carbamoyl)piperidine-1-carboxylate

$N^8$-(2,6-dimethylbenzyl)-2,3-dimethylimidazo[1,2-a]pyridine-6,8-diamine hydrochloride (Intermediate 1, 50 mg, 0.15 mmol), (S)-1-Boc-piperidine-2-carboxylic acid (38 mg, 0.17 mmol), 1-[bis(dimethylamino)methylene]-1H-1,2,3-triazolo[4,5-B]pyridinium 3-oxide hexafluorophosphate (75 mg, 0.20 mmol), and diisopropylethylamine (0.11 mL, 0.61 mmol) were dissolved in dichloromethane (0.7 mL) and stirred at room temperature for 12 hours. The reaction was quenched by adding distilled water, extracted with dichloromethane, and the organic layer was dried over anhydrous magnesium sulfate, then concentrated under reduced pressure. The residue was purified by column chromatography to obtain the target compound (75 mg, 98%).

Step 2: Preparation of (S)-N-(8-((2,6-dimethylbenzyl)amino)-2,3-dimethylimidazo[1,2-a]pyridin-6-yl)piperidine-2-carboxamide hydrochloride

Tert-butyl (S)-2-((8-((2,6-dimethylbenzyl)amino)-2,3-dimethylimidazo[1,2-a]pyridin-6-yl)carbamoyl)piperidine-1-carboxylate (75 mg, 0.15 mmol) prepared in Step 1 was dissolved in ethyl acetate (0.7 mL). 2 M hydrochloric acid (0.30 mL) diluted in diethyl ether was added, and the mixture was stirred at 50°C for 12 hours. The resulting solid was filtered and dried to obtain the target compound (59 mg, 89%).
$^1$H NMR (500 MHz, DMSO-$d_6$) δ 11.32 (s, 1H), 9.35-9.33 (m, 1H), 8.89-8.87 (m, 1H), 8.44 (s, 1H), 7.23-7.14 (m, 3H), 7.04 (s, 1H), 6.45 (s, 1H), 4.31-4.30 (d, 2H), 4.03-4.02 (m, 1H), 3.07-3.05 (m, 2H), 2.42-2.31 (m, 14H), 1.69-1.64 (m, 4H)

**[Example 107] Preparation of (E)-4-(3-((8-((2,6-dimethylbenzyl)amino)-2,3-dimethylimidazo[1,2-a]pyridin-6-yl) amino)-3-oxoprop-1-en-1-yl)phenyl acetate**

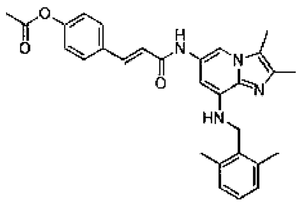

$N^8$-(2,6-Dimethylbenzyl)-2,3-dimethylimidazo[1,2-a]pyridine-6,8-diamine hydrochloride (Intermediate 1, 80 mg, 0.24 mmol), (E)-3-(4-acetoxyphenyl)acrylic acid (54 mg, 0.26 mmol), 1-ethyl-3-(3-dimethylaminopropyl)carbodiimide (68 mg, 0.36 mmol), 1-hydroxybenzotriazole hydrate (48 mg, 0.36 mmol), and triethylamine (0.1 mL, 0.72 mmol) were dissolved in dichloromethane (1.2 mL) and stirred at room temperature for 12 hours. Distilled water was added to quench the reaction, and extraction was performed with dichloromethane. The organic layer was dried over anhydrous magnesium sulfate and concentrated under reduced pressure. The residue was purified by column chromatography to obtain the target compound (22 mg, 19%).
$^1H$ NMR (500 MHz, DMSO-$d_6$) $\delta$ 9.95 (s, 1H), 7.17-7.06 (m, 7H), 6.09 (s, 1H), 5.19-5.18 (m, 1H), 5.11-5.10 (m, 1h), 4.32-4.31 (d, 2H), 2.37-2.24 (m, 12H), 2.11 (s, 3H)

**[Example 108] Preparation of (E)-4-(3-((8-((2,6-dimethylbenzyl)amino)-2,3-dimethylimidazo[1,2-a]pyridin-6-yl) amino)-3-oxoprop-1-en-1-yl)-2-methoxyphenyl acetate**

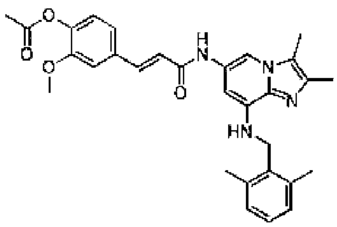

$N^8$-(2,6-Dimethylbenzyl)-2,3-dimethylimidazo[1,2-a]pyridine-6,8-diamine hydrochloride (Intermediate 1, 80 mg, 0.24 mmol), (E)-3-(4-ethoxy-3-methoxyphenyl)acrylic acid (61 mg, 0.26 mmol), 1-ethyl-3-(3-dimethylaminopropyl) carbodiimide (59 mg, 0.31 mmol), 1-hydroxybenzotriazole hydrate (42 mg, 0.31 mmol), and diisopropylethylamine (0.2 mL, 1.20 mmol) were dissolved in dichloromethane (1.2 mL) and stirred at room temperature for 12 hours. Distilled water was added to quench the reaction, and the mixture was extracted with dichloromethane. The organic layer was dried over anhydrous magnesium sulfate and concentrated under reduced pressure. The residue was purified by column chromatography to obtain the target compound (30 mg, 24%).
$^1H$ NMR (500 MHz, DMSO-$d_6$) $\delta$ 9.94 (s, 1H), 7.17-7.06 (m, 4H), 6.96-6.94 (d, 1H), 6.35-6.34 (d, 1H), 6.08 (s, 1H), 5.18-5.17 (d, 1H), 5.11-5.10 (m, 1H), 4.33-4.32 (d, 2H), 3.86-3.84 (m, 1H), 3.75 (s, 3H), 2.37-2.35 (m, 9H), 2.23 (s, 3H), 2.11 (s, 3H)

**[Example 109] Preparation of (S)-N-(8-((2,6-dimethylbenzyl)amino)-2,3-dimethylimidazo[1,2-a]pyridin-6-yl)aziridine-2-carboxamide hydrochloride**

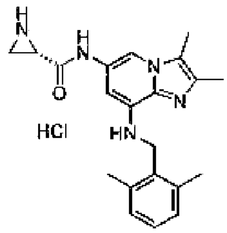

Step 1: Preparation of tert-butyl (S)-2-((8-((2,6-dimethylbenzyl)amino)-2,3-dimethylimidazo[1,2-a]pyridin-6-yl)carbamoyl)aziridine-1-carboxylate

$N^8$-(2,6-dimethylbenzyl)-2,3-dimethylimidazo[1,2-a]pyridine-6,8-diamine hydrochloride (Intermediate 1, 80 mg, 0.24 mmol), (S)-1-(tert-butoxycarbonyl)aziridine-2-carboxylic acid (50 mg, 0.26 mmol), 1-[bis(dimethylamino)methylene]-1H-1,2,3-triazolo[4,5-B]pyridinium 3-oxide hexafluorophosphate (118 mg, 0.31 mmol), and diisopropylethylamine (0.12 mL, 0.72 mmol) were dissolved in dichloromethane (3.42 mL) and stirred at room temperature for 12 hours. The reaction was quenched by adding distilled water, extracted with dichloromethane, and the organic layer was dried over anhydrous magnesium sulfate, then concentrated under reduced pressure. The residue was purified by column chromatography to obtain the target compound (31 mg, 28%).

Step 2: Preparation of (S)-N-(8-((2,6-dimethylbenzyl)amino)-2,3-dimethylimidazo[1,2-a]pyridin-6-yl)aziridine-2-carboxamide hydrochloride

Tert-butyl (S)-2-((8-((2,6-dimethylbenzyl)amino)-2,3-dimethylimidazo[1,2-a]pyridin-6-yl)carbamoyl)aziridine-1-carboxylate (30 mg, 0.06 mmol) prepared in Step 1 was dissolved in ethyl acetate (0.3 mL). 2 N hydrochloric acid (0.12 mL) diluted in diethyl ether was added. The mixture was stirred at room temperature for 16 hours. The resulting solid was filtered and dried to obtain the target compound (7 mg, 29%).
$^1$H NMR (500 MHz, DMSO-$d_6$) δ 8.34-8.33 (m, 2H), 7.21-7.10 (m, 4H), 4.32-4.30 (m, 2H), 4.29-4.26 (m, 1H), 4.24-4.21 (m, 1H), 2.38-2.34 (m, 12H)

**[Example 110] Preparation of (S)-N-(8-((2,6-dimethylbenzyl)amino)-2,3-dimethylimidazo[1,2-a]pyridin-6-yl)-1-(2-hydroxyacetyl)pyrrolidine-3-carboxamide citrate**

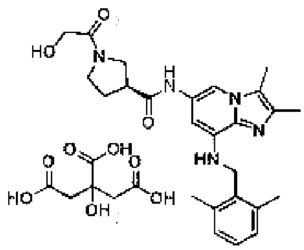

Step 1: Preparation of (S)-2-(3-((8-((2,6-dimethylbenzyl)amino)-2,3-dimethylimidazo[1,2-a]pyridin-6-yl)carbamoyl)pyrrolidin-1-yl)-2-oxoethyl acetate

(S)-N-(8-((2,6-dimethylbenzyl)amino)-2,3-dimethylimidazo[1,2-a]pyridin-6-yl)pyrrolidine-3-carboxamide hydrochloride (Example 105, 40 mg, 0.093 mmol) and triethylamine (39 μL, 0.28 mmol) were dissolved in dichloromethane (0.47 mL), and the mixture was stirred at room temperature for 5 minutes. 2-Chloro-2-oxoethyl acetate (12 μL, 0.11 mmol) was slowly added dropwise and stirred at room temperature for 4 hours. The reaction was quenched by adding distilled water, extracted with dichloromethane, and the organic layer was dried over anhydrous magnesium sulfate, then concentrated under reduced pressure. The residue was purified by column chromatography to obtain the target compound (24 mg, 51%).

Step 2: Preparation of (S)-N-(8-((2,6-dimethylbenzyl)amino)-2,3-dimethylimidazo[1,2-a]pyridin-6-yl)-1-(2-hydroxyacetyl)pyrrolidine-3-carboxamide citrate

(S)-2-(3-((8-((2,6-dimethylbenzyl)amino)-2,3-dimethylimidazo[1,2-a]pyridin-6-yl)carbamoyl)pyrrolidin-1-yl)-2-oxoethyl acetate (24 mg, 0.048 mmol) prepared in Step 1 was dissolved in tetrahydrofuran (0.3 mL), methanol (0.3 mL), and distilled water (0.3 mL), and then potassium hydroxide (13 mg, 0.24 mmol) was added and stirred at room temperature for 5 hours. The solvent was removed, distilled water was added to stop the reaction, and extraction was performed with ethyl acetate. The organic layer was dried over anhydrous magnesium sulfate, concentrated under reduced pressure, and the target compound (21 mg, 97%) was obtained. This was then dissolved in acetone, citric acid (9 mg, 0.047 mmol) was added, and the mixture was stirred at room temperature for 12 hours. The resulting solid was filtered and dried to obtain the citrate salt of the target compound.
$^1$H NMR (500 MHz, DMSO-$d_6$) δ 10.09-10.08 (m, 1H), 8.25 (s, 1H), 7.18-7.11 (m, 3H), 6.31-6.26 (m, 1H), 5.08 (s, 1H), 4.59-4.58 (m, 1H), 4.31-4.30 (d, 2H), 4.02 (s, 1H), 3.68-3.67 (m, 1H), 3.65-3.63 (m, 2H), 2.64-2.53 (m, 4H), 2.35-2.22 (m, 12H)

**[Example 111] Preparation of (R)-1-((8-((4-fluoro-2,6-dimethylbenzyl)amino)-2,3-dimethylimidazo[1,2-a]pyridin-6-yl)amino)-1-oxopropan-2-yl acetate**

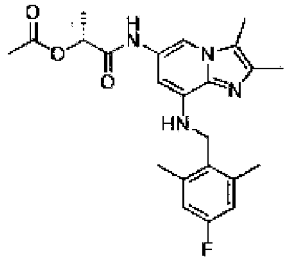

N$^8$-(4-Fluoro-2,6-dimethylbenzyl)-2,3-dimethylimidazo[1,2-a]pyridine-6,8-diamine hydrochloride (Intermediate 2, 80 mg, 0.22 mmol), (R)-2-acetoxypyropionic acid (32 mg, 0.24 mmol), 1-[bis(dimethylamino)methylene]-1H-1,2,3-triazolo[4,5-B]pyridinium 3-oxide hexafluorophosphate (130 mg, 0.34 mmol), and diisopropylethylamine (0.16 mL, 0.88 mmol) were dissolved in dichloromethane (3.14 mL) and stirred at room temperature for 12 hours. Distilled water was added to quench the reaction, and the mixture was extracted with dichloromethane. The organic layer was dried over anhydrous magnesium sulfate and concentrated under reduced pressure. The residue was purified by column chromatography to obtain the target compound (45 mg, 48%).
$^1$H NMR (500 MHz, DMSO-$d_6$) δ 10.33 (s, 1H), 8.37 (s, 1H), 7.02-7.00 (d, 2H), 6.71 (s, 1H), 5.10-5.09 (m, 1H), 4.28-4.27 (d, 2H), 3.64-3.62 (m, 1H), 3.16-3.15 (m, 1H), 2.70 (s, 3H), 2.37-2.33 (m, 12H), 2.11 (s, 3H)

**[Example 112] Preparation of (S)-1-((8-((4-fluoro-2,6-dimethylbenzyl)amino)-2,3-dimethylimidazo[1,2-a]pyridin-6-yl)amino)-1-oxopropan-2-yl acetate**

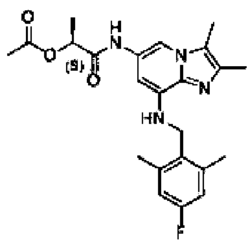

N$^8$-(4-Fluoro-2,6-dimethylbenzyl)-2,3-dimethylimidazo[1,2-a]pyridine-6,8-diamine hydrochloride (Intermediate 2, 80 mg, 0.22 mmol), (S)-2-acetoxypyropionic acid (29 mg, 0.22 mmol), 1-[bis(dimethylamino)methylene]-1H-1,2,3-triazolo[4,5-B]pyridinium 3-oxide hexafluorophosphate (110 mg, 0.29 mmol), and diisopropylethylamine (0.15 mL, 0.88 mmol) were dissolved in dichloromethane (3.14 mL) and stirred at room temperature for 16 hours. Distilled water was added to quench the reaction, and the mixture was extracted with dichloromethane. The organic layer was dried over

anhydrous magnesium sulfate and concentrated under reduced pressure. The residue was purified by column chromatography to obtain the target compound (45 mg, 48%).
$^1$H NMR (500 MHz, DMSO-$d_6$) δ 10.31 (s, 1H), 8.39 (s, 1H), 7.21 (s, 1H), 7.00-6.98 (d, 2H), 6.67 (s, 1H), 5.10-5.07 (m, 1H), 4.28-4.27 (m, 2H), 2.70 (s, 3H), 2.37-2.34 (m, 12H), 2.11 (s, 3H)

**[Example 113] Preparation of 1-((8-((4-fluoro-2,6-dimethylbenzyl)amino)-2,3-dimethylimidazo[1,2-a]pyridin-6-yl)carbamoyl)cyclopropyl acetate**

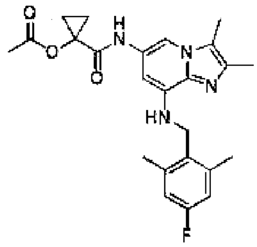

N$^8$-(4-Fluoro-2,6-dimethylbenzyl)-2,3-dimethylimidazo[1,2-a]pyridine-6,8-diamine hydrochloride (Intermediate 2, 80 mg, 0.22 mmol), 1-acetoxycyclopropane-1-carboxylic acid (31 mg, 0.22 mmol), 1-[bis(dimethylamino)methylene]-1H-1,2,3-triazolo[4,5-B]pyridinium 3-oxide hexafluorophosphate (108 mg, 0.28 mmol), and diisopropylethylamine (0.15 mL, 0.88 mmol) were dissolved in dichloromethane (3.14 mL) and stirred at room temperature for 16 hours. Distilled water was added to quench the reaction, and the mixture was extracted with dichloromethane. The organic layer was dried over anhydrous magnesium sulfate and concentrated under reduced pressure. The residue was purified by column chromatography to obtain the target compound (40 mg, 42%).
$^1$H NMR (500 MHz, DMSO-$d_6$) δ 9.77 (s, 1H), 8.24 (s, 1H), 7.21 (s, 1H), 6.99-6.97 (d, 2H), 6.67 (s, 1H), 5.33-5.32 (m, 2H), 4.27-4.26 (d, 2H), 2.69 (s, 2H), 2.33-2.28 (m, 12H), 2.15 (s, 3H), 2.01-1.98 (m, 2H)

**[Example 114] Preparation of (S)-N-(8-((4-fluoro-2,6-dimethylbenzyl)amino)-2,3-dimethylimidazo[1,2-a]pyridin-6-yl)-2-hydroxypropanamide**

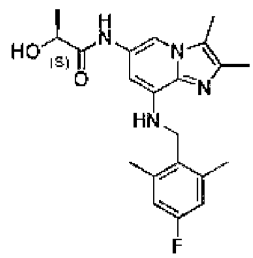

(S)-1-((8-((4-fluoro-2,6-dimethylbenzyl)amino)-2,3-dimethylimidazo[1,2-a]pyridin-6-yl)amino)-1-oxopropan-2-yl acetate (Example 112, 20 mg, 0.04 mmol) was dissolved in tetrahydrofuran (0.4 mL), methanol (0.4 mL), and distilled water (0.4 mL), potassium hydroxide (13 mg, 0.23 mmol) was added, and the mixture was stirred at room temperature for 16 hours. The solvent was removed, distilled water was added to quench the reaction, and the mixture was extracted with ethyl acetate. The organic layer was dried over anhydrous magnesium sulfate and concentrated under reduced pressure. The residue was purified by column chromatography to obtain the target compound (10 mg, 65%).
$^1$H NMR (500 MHz, DMSO-$d_6$) δ 9.52 (s, 1H), 8.28 (s, 1H), 6.96-6.94 (d, 2H), 5.82-5.81 (m, 1H), 4.27-4.26 (d, 2H), 4.18-4.16 (m, 1H), 2.35 (s, 6H), 2.29 (s, 3H), 2.21 (s, 3H)1.34-1.33 (d, 3H)

**[Example 115] Preparation of N-(8-((4-fluoro-2,6-dimethylbenzyl)amino)-2,3-dimethylimidazo[1,2-a]pyridin-6-yl)-1-hydroxycyclopropane-1-carboxamide**

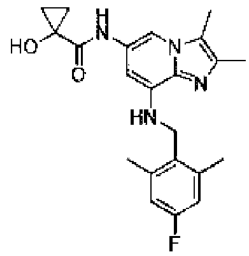

1-((8-((4-Fluoro-2,6-dimethylbenzyl)amino)-2,3-dimethylimidazo[1,2-a]pyridin-6-yl)carbamoyl)cyclopropyl acetate (Example 113, 30 mg, 0.068 mmol) was dissolved in tetrahydrofuran (0.68 mL), methanol (0.68 mL), and distilled water (0.68 mL), potassium hydroxide (19 mg, 0.34 mmol) was added, and the mixture was stirred at room temperature for 16 hours. The solvent was removed, distilled water was added to quench the reaction, and the mixture was extracted with ethyl acetate. The organic layer was dried over anhydrous magnesium sulfate and concentrated under reduced pressure. The residue was purified by column chromatography, dissolved in ethyl acetate, and hexane was added to precipitate a solid to obtain the target compound (16 mg, 59%).
$^1$H NMR (500 MHz, DMSO-$d_6$) δ 9.65 (s, 1H), 8.26 (s, 1H), 6.96-6.94 (d, 2H), 6.70 (s, 1H), 6.59 (s, 1H), 5.02 (s, 1H), 4.28-4.27 (d, 2H), 2.35 (s, 6H), 2.78 (s, 3H), 2.22 (s, 3H), 1.18-1.17 (m, 2H), 0.99-0.98 (m, 2H)

**[Example 116] Preparation of 3-(8-((4-fluoro-2,6-dimethylbenzyl)amino)-2,3-dimethylimidazolidine[1,2-a]pyridin-6-yl)-1-methylimidazolidine-2,4-dione**

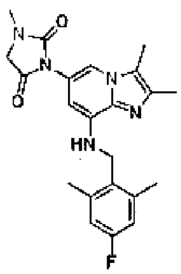

N$^8$-(4-Fluoro-2,6-dimethylbenzyl)-2,3-dimethylimidazo[1,2-a]pyridine-6,8-diamine hydrochloride (Intermediate 2, 60 mg, 0.17 mmol) and 1,1'-carbonyldiimidazole (55 mg, 0.34 mmol) were dissolved in dichloromethane (2 mL) and stirred at room temperature for 1 hour. Methylglycine hydrochloride (21 mg, 0.17 mmol) and triethylamine (0.09 mL, 0.68 mmol) were added and stirred at room temperature for 12 hours. The reaction was quenched by adding distilled water, extracted with dichloromethane, and the organic layer was dried over anhydrous magnesium sulfate and concentrated under reduced pressure. The residue was purified by column chromatography to obtain the target compound (18 mg, 25%).
$^1$H NMR (500 MHz, DMSO-$d_6$) δ 7.61 (s, 1H), 6.95-6.93 (d, 2H), 6.20 (s, 1H), 5.23-5.21 (m, 1H), 4.26-4.25 (d, 2H), 4.14 (s, 2H), 2.95 (sm 3H), 2.35-2.26 (m, 12H)

**[Example 117] Preparation of (R)-N-(8-((4-fluoro-2,6-dimethylbenzyl)amino)-2,3-dimethylimidazo[1,2-a]pyridin-6-yl)-2-hydroxypropanamide**

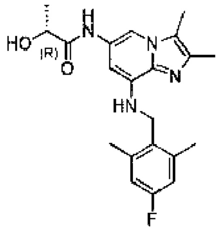

(R)-1-((8-((4-fluoro-2,6-dimethylbenzyl)amino)-2,3-dimethylimidazo[1,2-a]pyridin-6-yl)amino)-1-oxopropan-2-yl acetate (Example 111, 20 mg, 0.04 mmol) was dissolved in tetrahydrofuran (0.4 mL), methanol (0.4 mL), and distilled water (0.4 mL), potassium hydroxide (13 mg, 0.23 mmol) was added, and the mixture was stirred at room temperature for 16 hours. The solvent was removed, distilled water was added to stop the reaction, and the mixture was extracted with ethyl acetate. The organic layer was dried over anhydrous magnesium sulfate and concentrated under reduced pressure. The residue was purified by column chromatography to obtain the target compound (10 mg, 65%).
$^1$H NMR (500 MHz, DMSO-$d_6$) δ 9.51 (s, 1H), 8.27 (s, 1H), 6.96-6.94 (d, 2H), 6.56 (s, 1H), 5.81-5.80 (d, 1H), 5.04 (s, 1H), 4.27-4.26 (d, 2H), 4.18-4.16 (m, 1H), 2.35 (s, 6H), 2.29 (s, 3H), 2.22 (s, 3H), 1.34-1.32 (d, 3H)

**[Example 118] Preparation of (S)-N-(8-((2,6-dimethylbenzyl)amino)-2,3-dimethylimidazo[1,2-a]pyridin-6-yl)piperidine-3-carboxamide hydrochloride**

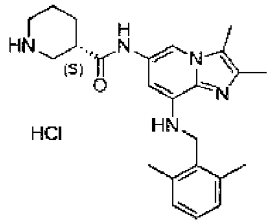

Step 1: Preparation of tert-butyl (S)-3-((8-((2,6-dimethylbenzyl)amino)-2,3-dimethylimidazo[1,2-a]pyridin-6-yl)carbamoyl)piperidine-1-carboxylate

N[8]-(2,6-dimethylbenzyl)-2,3-dimethylimidazo[1,2-a]pyridine-6,8-diamine hydrochloride (Intermediate 1, 50 mg, 0.15 mmol), (S)-1-Boc-piperidine-3-carboxylic acid (38 mg, 0.17 mmol), 1-[bis(dimethylamino)methylene]-1H-1,2,3-triazolo[4,5-B]pyridinium 3-oxide hexafluorophosphate (75 mg, 0.20 mmol), and diisopropylethylamine (0.11 mL, 0.61 mmol) were dissolved in dichloromethane (0.7 mL) and stirred at room temperature for 12 hours. The reaction was quenched by adding distilled water, extracted with dichloromethane, and the organic layer was dried over anhydrous magnesium sulfate, then concentrated under reduced pressure. The residue was purified by column chromatography to obtain the target compound (75 mg, 98%).

Step 2: Preparation of (S)-N-(8-((2,6-dimethylbenzyl)amino)-2,3-dimethylimidazo[1,2-a]pyridin-6-yl)piperidine-3-carboxamide hydrochloride

Tert-butyl (S)-3-((8-((2,6-dimethylbenzyl)amino)-2,3-dimethylimidazo[1,2-a]pyridin-6-yl)carbamoyl)piperidine-1-carboxylate (75 mg, 0.15 mmol) prepared in Step 1 was dissolved in ethyl acetate (0.7 mL). 2 M hydrochloric acid (0.30 mL) diluted in diethyl ether was added, and the mixture was stirred at 50°C for 12 hours. The resulting solid was filtered and dried to obtain the target compound (60 mg, 91%).
$^1$H NMR (500 MHz, DMSO-$d_6$) δ 10.78 (s, 1H), 8.52 (s, 1H), 7.22-7.13 (m, 3H), 6.56 (s, 1H), 4.31-4.30 (d, 2H), 3.13-3.10 (m, 4H), 2.40-2.34 (m, 12H), 1.90-1.58 (m, 4H)

**[Example 119] Preparation of N-(8-((2,6-dimethylbenzyl)amino)-2,3-dimethylimidazo[1,2-a]pyridin-6-yl)-3-(2-hydroxyacetamido)propanamide**

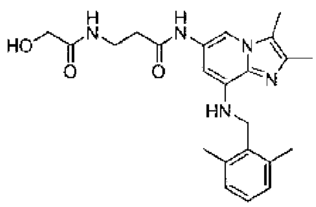

Step 1: Preparation of 2-((3-((8-((2,6-dimethylbenzyl)amino)-2,3-dimethylimidazo[1,2-a]pyridin-6-yl)amino)-3-oxopropyl)amino)-2-oxoethylacetate

3-Amino-N-(8-((2,6-dimethylbenzyl)amino)-2,3-dimethylimidazo[1,2-a]pyridin-6-yl)propanamide hydrochloride (Example 99, 28 mg, 0.069 mmol) and triethylamine (29 μL, 0.21 mmol) were dissolved in dichloromethane (0.35 mL) and stirred at room temperature for 5 minutes. 2-Chloro-2-oxoethyl acetate (9 μL, 0.083 mmol) was slowly added dropwise and stirred at room temperature for 12 hours. The reaction was quenched by adding distilled water, extracted with dichloromethane, and the organic layer was dried over anhydrous magnesium sulfate, then concentrated under reduced pressure. The residue was purified by column chromatography to obtain the target compound (23 mg, 72%).

Step 2: Preparation of N-(8-((2,6-dimethylbenzyl)amino)-2,3-dimethylimidazo[1,2-a]pyridin-6-yl)-3-(2-hydroxyacetamido)propanamide

2-((3-((8-((2,6-dimethylbenzyl)amino)-2,3-dimethylimidazo[1,2-a]pyridin-6-yl)amino)-3-oxopropyl)amino)-2-oxoethyl acetate (23 mg, 0.050 mmol) prepared in Step 1 was dissolved in tetrahydrofuran (0.3 mL), methanol (0.3 mL), and distilled water (0.3 mL). Potassium hydroxide (14 mg, 0.25 mmol) was added, and the mixture was stirred at room temperature for 2 hours. The solvent was removed, distilled water was added, and the reaction was quenched. The mixture was then extracted with ethyl acetate. The organic layer was dried over anhydrous magnesium sulfate, then concentrated under reduced pressure to obtain the target compound (15 mg, 71%).
$^1$H NMR (500 MHz, DMSO-$d_6$) δ 9.93 (s, 1H), 8.22 (s, 1H), 7.84-7.82 (m, 1H), 7.17-7.09 (m, 3H), 6.24 (s, 1H), 5.54 (s, 1H), 4.98-4.97 (m, 1H), 4.30-4.29 (d, 2H), 3.81-3.80 (m, 2H), 3.61-3.60 (m, 1H), 3.42-3.41 (m, 2H), 2.56-2.55 (m, 2H), 2.35-2.21 (m, 12H)

**[Example 120] Preparation of (S)-N-(8-((2,6-dimethylbenzyl)amino)-2,3-dimethylimidazo[1,2-a]pyridin-6-yl)-1-(2-hydroxyacetyl)piperidine-3-carboxamide**

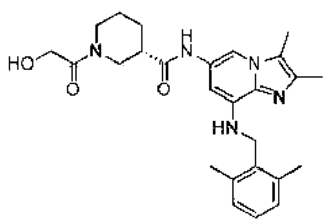

Step 1: Preparation of (S)-2-(3-((8-((2,6-dimethylbenzyl)amino)-2,3-dimethylimidazo[1,2-a]pyridin-6-yl)carbamoyl)piperidin-1-yl)-2-oxoethyl acetate

(S)-N-(8-((2,6-dimethylbenzyl)amino)-2,3-dimethylimidazo[1,2-a]pyridin-6-yl)piperidine-3-carboxamide hydrochloride (Example 118, 43 mg, 0.097 mmol) and triethylamine (41 μL, 0.29 mmol) were dissolved in dichloromethane (0.49 mL), and the mixture was stirred at room temperature for 5 minutes. 2-Chloro-2-oxoethyl acetate (13 μL, 0.12 mmol) was slowly added dropwise and stirred at room temperature for 5 hours. The reaction was quenched by adding distilled water, extracted with dichloromethane, and the organic layer was dried over anhydrous magnesium sulfate, then concentrated under reduced pressure. The residue was purified by column chromatography to obtain the target compound (22 mg, 45%).

Step 2: Preparation of (S)-N-(8-((2,6-dimethylbenzyl)amino)-2,3-dimethylimidazo[1,2-a]pyridin-6-yl)-1-(2-hydroxyacetyl)piperidine-3-carboxamide

(S)-2-(3-((8-((2,6-dimethylbenzyl)amino)-2,3-dimethylimidazo[1,2-a]pyridin-6-yl)carbamoyl)piperidin-1-yl)-2-oxoethyl acetate (22 mg, 0.044 mmol) prepared in Step 1 was dissolved in tetrahydrofuran (0.3 mL), methanol (0.3 mL), and distilled water (0.3 mL), and then potassium hydroxide (12 mg, 0.22 mmol) was added and stirred at room temperature for 3 hours. After removing the solvent and adding distilled water to quench the reaction, extraction was performed with ethyl acetate. The organic layer was dried over anhydrous magnesium sulfate, concentrated under reduced pressure, and the target compound (16 mg, 80%) was obtained.
$^1$H NMR (500 MHz, DMSO-$d_6$) δ 9.98-9.97 (m, 1H), 8.24 (s, 1H), 7.17-7.09 (m, 3H), 6.88 (s, 1H), 4.97-4.96 (m, 1H), 4.55-4.44 (m, 1H), 4.31-4.30 (d, 2H), 4.16-4.01 (m, 2H), 3.78-3.68 (m, 1H), 2.33-2.19 (m, 12H), 2.10-2.08 (m, 2H),

1.75-1.71 (m, 2H)

[Comparative Example 1]

The following compound was prepared.

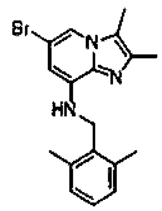

[Comparative Example 2]

The following compound was prepared.

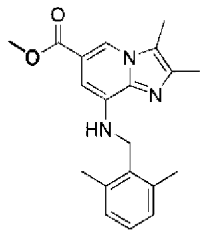

**Experimental Example 1: Measurement of Inhibitory Activity on Proton Pump ($H^+$,$K^+$-ATPase) Activity**

The inhibitory activity of the compounds prepared in Preliminary Examples 1 and 2 and Examples 1 to 120 on proton pump activity was measured as follows.

Specifically, gastric vesicles were isolated from porcine stomachs, and the protein content of the vesicles was quantified using a BSA kit (Bio-Rad, 5000205). All reactions were performed in 96-well plates with a reaction volume of 80 mL containing 100 ng of vesicles. 10 mM valinomycin and the test compound were added to reaction buffer (50 mM Tris-HEPES, 5 mM $MgCl_2$, 8 mM KCl, pH 6.5) containing porcine gastric vesicles at various concentrations, and the mixture was incubated at 37°C for 30 minutes. DMSO or test compounds at different concentrations were added after diluting them to a final DMSO concentration of 0.1%. 0.2 mM adenosine triphosphate (ATP) was then added to the reaction buffer and incubated at 37°C for 20 minutes. The reaction was stopped with 0.2% Tween 20, followed by the addition of malachite green (Sigma Aldrich, MAK307) and incubation at room temperature for 30 minutes. The optical density (OD) value was measured at 620 nm using a microplate reader (Spectramax M5e, Molecular devices). The percent (%) inhibition was derived from the OD values of the control group and various concentrations of the test compounds. The $IC_{50}$ of the test compounds was analyzed using nonlinear regression using the GraphPad Prism8 program using the % inhibition value at each concentration. The results are shown in Table 1 below.

$$(AA < 50\ nM,\ 50\ nM \leq A < 100\ nM,\ 100\ nM \leq B < 500\ nM,\ 500\ nM \leq C)$$

[Table 1]

| **No.** | **$IC_{50}$ (nM)** | **No.** | **$IC_{50}$ (nM)** | **No.** | **$IC_{50}$ (nM)** |
|---|---|---|---|---|---|
| **Example 1** | B | **Example 43** | AA | **Example 85** | B |
| **Example 2** | B | **Example 44** | AA | **Example 86** | AA |
| **Example 3** | C | **Example 45** | AA | **Example 87** | AA |
| **Example 4** | AA | **Example 46** | A | **Example 88** | AA |

(continued)

| No. | IC$_{50}$ (nM) | No. | IC$_{50}$ (nM) | No. | IC$_{50}$ (nM) |
|---|---|---|---|---|---|
| **Example 5** | B | **Example 47** | A | **Example 89** | A |
| **Example 6** | A | **Example 48** | A | **Example 90** | A |
| **Example 7** | B | **Example 49** | AA | **Example 91** | A |
| **Example 8** | C | **Example 50** | AA | **Example 92** | C |
| **Example 9** | C | **Example 51** | AA | **Example 93** | AA |
| **Example 10** | AA | **Example 52** | B | **Example 94** | AA |
| **Example 11** | B | **Example 53** | B | **Example 95** | AA |
| **Example 12** | B | **Example 54** | B | **Example 96** | B |
| **Example 13** | A | **Example 55** | C | **Example 97** | A |
| **Example 14** | B | **Example 56** | C | **Example 98** | A |
| **Example 15** | AA | **Example 57** | B | **Example 99** | A |
| **Example 16** | B | **Example 58** | B | **Example 100** | AA |
| **Example 17** | B | **Example 59** | B | **Example 101** | AA |
| **Example 18** | C | **Example 60** | AA | **Example 102** | AA |
| **Example 19** | A | **Example 61** | A | **Example 103** | B |
| **Example 20** | A | **Example 62** | B | **Example 104** | B |
| **Example 21** | AA | **Example 63** | A | **Example 105** | B |
| **Example 22** | A | **Example 64** | AA | **Example 106** | B |
| **Example 23** | A | **Example 65** | A | **Example 107** | C |
| **Example 24** | AA | **Example 66** | B | **Example 108** | C |
| **Example 25** | B | **Example 67** | A | **Example 109** | B |
| **Example 26** | AA | **Example 68** | A | **Example 110** | B |
| **Example 27** | A | **Example 69** | AA | **Example 111** | B |
| **Example 28** | C | **Example 70** | B | **Example 112** | C |
| **Example 29** | C | **Example 71** | B | **Example 113** | B |
| **Example 30** | B | **Example 72** | B | **Example 114** | B |
| **Example 31** | B | **Example 73** | B | **Example 115** | AA |
| **Example 32** | A | **Example 74** | A | **Example 116** | A |
| **Example 33** | AA | **Example 75** | B | **Example 117** | AA |
| **Example 34** | C | **Example 76** | A | **Example 118** | B |
| **Example 35** | A | **Example 77** | AA | **Example 119** | A |
| **Example 36** | A | **Example 78** | B | **Example 120** | B |
| **Example 37** | A | **Example 79** | B | **Preliminary Example 1** | A |
| **Example 38** | C | **Example 80** | C | **Preliminary Example 2** | A |
| **Example 39** | C | **Example 81** | A | **Comparative Example 1** | B |
| **Example 40** | B | **Example 82** | B | **Comparative Example 2** | C |
| **Example 41** | C | **Example 83** | B | **Tegoprazan** | C |
| **Example 42** | B | **Example 84** | B | | |

As shown in Table 1, the compounds prepared in Preliminary Examples 1 and 2 and Examples 1 to 120 were all

confirmed to have inhibitory activity against proton pump activity. They were confirmed to have inhibitory activity against proton pump activity that was equivalent to or greater than that of Tegoprazan, a previously known representative proton pump inhibitor. On the other hand, the compounds prepared in Comparative Examples 1 and 2 were confirmed to have weak inhibitory activity against proton pump activity.

Among the compounds prepared in Preliminary Examples 1 and 2 and Examples 1 to 120, the compounds prepared in Examples 4, 10, 15, 21, 24, 26, 33, 43-45, 49-51, 60, 64, 77, 86-88, 93-95, 100-102, 115, and 117 were found to have excellent inhibitory properties against proton pump activity.

## Experimental Example 2: Measurement of Inhibitory Activity on Basal Gastric Acid Secretion in Pylorus-Ligated Rats

The Shay's rat model was used to evaluate the inhibitory activity of the compounds prepared in Examples 4, 15, 21, 26, 45, 60, 86, 100, 101, and 102 on basal gastric acid secretion.

Specifically, male Sprague Dawley (SD) rats (weighing 180-220 g) were fasted for 24 hours with only water, and then randomly assigned to groups (n=4-5) after measuring body weight. The negative control group was orally administered 10% dimethylsulfoxide (DMSO)/10% Cremophor EL/80% D.W. at 2 mL/kg, and the test group was orally administered the test compound at 2 mg/kg/2 mL. One hour after administration, the rats' abdominal cavity was incised under isoflurane anesthesia, the stomach was exposed, and the pylorus was ligated. Three hours after ligation, the test animals were sacrificed and the stomach contents were collected by laparotomy again. The contents were centrifuged (3,000 rpm, 10 min), and only the supernatant was separated to obtain gastric juice. Afterwards, gastric fluid volume measurement, gastric fluid pH measurement, NaOH titration, and total gastric acid secretion calculation (gastric fluid volume $\times$ 0.1 N NaOH titer $\times$ 100) were sequentially performed. The final % inhibitory activity of the test compound was calculated according to the following formula. The results are shown in Table 2 below.

% inhibitory activity of test compound = [(total gastric acid secretion of negative control group - total gastric acid secretion of test compound-treated group) / total gastric acid secretion of negative control group] $\times$ 100

$$(A \geq 90\%, 90\% > B \geq 80\%, 80\% > C \geq 70\%)$$

[Table 2]

| No. | GERD (rat/mpk) | No. | GERD (rat/mpk) |
|---|---|---|---|
| **Example 4** | A | **Example 60** | A |
| **Example 15** | A | **Example 86** | A |
| **Example 21** | A | **Example 100** | A |
| **Example 26** | A | **Example 101** | C |
| **Example 45** | A | **Example 102** | A |

As shown in Table 2, the compounds prepared in Examples 4, 15, 21, 26, 45, 60, 86, 100, 101, and 102 were all confirmed to have inhibitory activity against basal gastric acid secretion in pyloric-ligated rats. Meanwhile, Example 101 has considerable structural similarity to Example 102, but its inhibitory activity is seen to be somewhat reduced due to the substituted fluorine group.

The above description of the present invention is for illustrative purposes only, and those skilled in the art will readily appreciate that the present invention can be readily modified into other specific forms without altering the technical spirit or essential characteristics of the present invention. Therefore, the embodiments described above should be understood to be illustrative in all respects and not restrictive.

## Claims

1. An imidazopyridine compound represented by the following chemical formula 1 or a pharmaceutically acceptable salt thereof:

[Chemical Formula 1]

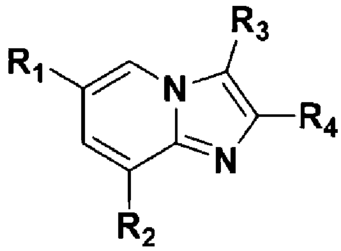

in chemical formula 1,

$R_1$ is

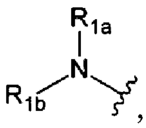, 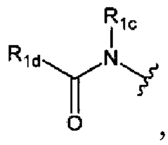, 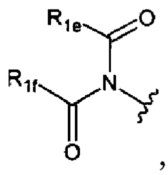, 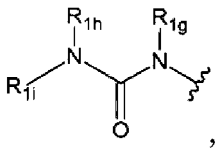, or 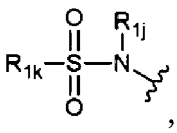,

wherein, $R_{1a}$ to $R_{1k}$ are independently hydrogen, cyano, substituted or unsubstituted C1 to C30 alkyl, substituted or unsubstituted C1 to C30 heteroalkyl, substituted or unsubstituted C2 to C30 alkenyl, substituted or unsubstituted C2 to C30 alkynyl, substituted or unsubstituted C3 to C30 cycloalkyl, substituted or unsubstituted C6 to C30 aryl, substituted or unsubstituted C7 to C30 arylalkyl, substituted or unsubstituted C2 to C30 heteroaryl, or substituted or unsubstituted C3 to C30 heteroarylalkyl, or adjacent groups among $R_{1a}$ to $R_{1k}$ are connected to each other to form a substituted or unsubstituted aliphatic ring type structure or a substituted or unsubstituted aromatic ring type structure;

$R_2$ is $-NR_{2a}R_{2b}$, wherein $R_{2a}$ or $R_{2b}$ is independently hydrogen, cyano, substituted or unsubstituted C1 to C30 alkyl, substituted or unsubstituted C2 to C30 alkenyl, substituted or unsubstituted C2 to C30 alkynyl, substituted or unsubstituted C3 to C30 cycloalkyl, substituted or unsubstituted C6 to C30 aryl, substituted or unsubstituted C7 to C30 arylalkyl, substituted or unsubstituted C2 to C30 heteroaryl, or substituted or unsubstituted C3 to C30 heteroarylalkyl; and

$R_3$ or $R_4$ is independently hydrogen, cyano, substituted or unsubstituted C1 to C30 alkyl, substituted or unsubstituted C2 to C30 alkenyl, substituted or unsubstituted C2 to C30 alkynyl, substituted or unsubstituted C3 to C30 cycloalkyl, substituted or unsubstituted C6 to C30 aryl, substituted or unsubstituted C7 to C30 arylalkyl, substituted or unsubstituted C2 to C30 heteroaryl, or substituted or unsubstituted C3 to C30 heteroarylalkyl.

2. The imidazopyridine compound of Claim 1 or a pharmaceutically acceptable salt thereof, wherein

$R_1$ is

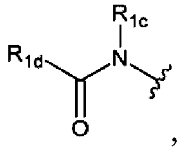, 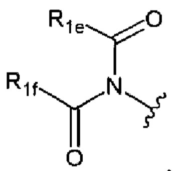, or 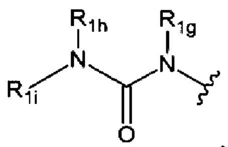,

wherein, $R_{1c}$ to $R_{1i}$ are independently hydrogen, substituted or unsubstituted C1 to C12 alkyl, substituted or unsubstituted C1 to C12 heteroalkyl, substituted or unsubstituted C3 to C12 cycloalkyl, substituted or unsubstituted C6 to C12 aryl, substituted or unsubstituted C7 to C12 arylalkyl, substituted or unsubstituted C2 to C12 heteroaryl, or substituted or unsubstituted C3 to C12 heteroarylalkyl, or adjacent groups among $R_{1c}$ to $R_{1i}$ are connected to each other to form a substituted or unsubstituted 12-membered or less aliphatic ring structure or a substituted or unsubstituted 12-membered or less aromatic ring structure.

**3.** The imidazopyridine compound of Claim 1 or a pharmaceutically acceptable salt thereof, wherein $R_2$ is $-NR_{2a}R_{2b}$, wherein $R_{2a}$ is hydrogen and $R_{2b}$ is substituted or unsubstituted C7 to C12 arylalkyl.

**4.** The imidazopyridine compound of Claim 1 or a pharmaceutically acceptable salt thereof, wherein $R_3$ or $R_4$ is independently hydrogen, or substituted or unsubstituted C1 to C12 alkyl.

**5.** The imidazopyridine compound of Claim 1 or a pharmaceutically acceptable salt thereof, wherein the compound is at least one selected from the group of consisting of:

(1) N-(8-((2,6-dimethylbenzyl)amino)-2,3-dimethylimidazo[1,2-a]pyridin-6-yl)cyclopentanecarboxamide;
(2) N-(8-((2,6-dimethylbenzyl)amino)-2,3-dimethylimidazo[1,2-a]pyridin-6-yl)cyclopropanecarboxamide;
(3) N-(8-((2,6-dimethylbenzyl)amino)-2,3-dimethylimidazo[1,2-a]pyridin-6-yl)cyclohexanecarboxamide;
(4) N-(8-((2,6-dimethylbenzyl)amino)-2,3-dimethylimidazo[1,2-a]pyridin-6-yl)acetamide;
(5) N-(8-((2,6-dimethylbenzyl)amino)-2,3-dimethylimidazo[1,2-a]pyridin-6-yl)cyclobutanecarboxamide;
(6) N-(8-((2,6-dimethylbenzyl)amino)-2,3-dimethylimidazo[1,2-a]pyridin-6-yl)azetidine-1-carboxamide;
(7) N-(8-((2,6-dimethylbenzyl)amino)-2,3-dimethylimidazo[1,2-a]pyridin-6-yl)isobutyramide;
(8) N-(8-((2,6-dimethylbenzyl)amino)-2,3-dimethylimidazo[1,2-a]pyridin-6-yl)pivalamide;
(9) N-(8-((2,6-dimethylbenzyl)amino)-2,3-dimethylimidazo[1,2-a]pyridin-6-yl)-2,2-difluoroacetamide;
(10) 2-amino-N-(8-((2,6-dimethylbenzyl)amino)-2,3-dimethylimidazo[1,2-a]pyridin-6-yl)acetamide;
(11) N-(8-((2,6-dimethylbenzyl)amino)-2,3-dimethylimidazo[1,2-a]pyridin-6-yl)pyrrolidine-1-carboxamide;
(12) N-(8-((2,6-dimethylbenzyl)amino)-2,3-dimethylimidazo[1,2-a]pyridin-6-yl)bicyclo[1.1.1]pentane-1-carboxamide;
(13) N-(8-((2,6-dimethylbenzyl)amino)-2,3-dimethylimidazo[1,2-a]pyridin-6-yl)propionamide;
(14) N-(8-((2,6-dimethylbenzyl)amino)-2,3-dimethylimidazo[1,2-a]pyridin-6-yl)oxazole-2-carboxamide;
(15) N-(8-((2,6-dimethylbenzyl)amino)-2,3-dimethylimidazo[1,2-a]pyridin-6-yl)-2-methoxyacetamide;
(16) N-(8-((2,6-dimethylbenzyl)amino)-2,3-dimethylimidazo[1,2-a]pyridin-6-yl)-2,2-difluoropropanamide;
(17) N-(8-((2,6-dimethylbenzyl)amino)-2,3-dimethylimidazo[1,2-a]pyridin-6-yl)-3-fluorocyclobutane-1-carboxamide;
(18) N-(8-((2,6-dimethylbenzyl)amino)-2,3-dimethylimidazo[1,2-a]pyridin-6-yl)cyclobutanesulfonamide;
(19) (S)-2-amino-N-(8-((2,6-dimethylbenzyl)amino)-2,3-dimethylimidazo[1,2-a]pyridin-6-yl)propanamide;
(20) (R)-2-amino-N-(8-((2,6-dimethylbenzyl)amino)-2,3-dimethylimidazo[1,2-a]pyridin-6-yl)propanamide;
(21) N-(8-((4-fluoro-2,6-dimethylbenzyl)amino)-2,3-dimethylimidazo[1,2-a]pyridin-6-yl)acetamide;
(22) 1-(8-((2,6-dimethylbenzyl)amino)-2,3-dimethylimidazo[1,2-a]pyridin-6-yl)-3-ethylurea;
(23) 1-cyclobutyl-3-(8-((2,6-dimethylbenzyl)amino)-2,3-dimethylimidazo[1,2-a]pyridin-6-yl)urea;
(24) 1-cyclopropyl-3-(8-((2,6-dimethylbenzyl)amino)-2,3-dimethylimidazo[1,2-a]pyridin-6-yl)urea;
(25) 2-((8-((2,6-dimethylbenzyl)amino)-2,3-dimethylimidazo[1,2-a]pyridin-6-yl)amino)-2-oxoethyl acetate;
(26) N-(8-((2,6-dimethylbenzyl)amino)-2,3-dimethylimidazo[1,2-a]pyridin-6-yl)-2-hydroxyacetamide;
(27) 2-amino-N-(8-((2,6-dimethylbenzyl)amino)-2,3-dimethylimidazo[1,2-a]pyridin-6-yl)-2-methylpropanamide;
(28) 1-(8-((2,6-dimethylbenzyl)amino)-2,3-dimethylimidazo[1,2-a]pyridin-6-yl)azetidin-2-one;
(29) 1-(bicyclo[1.1.1]pentan-1-yl)-3-(8-((2,6-dimethylbenzyl)amino)-2,3-dimethylimidazo[1,2-a]pyridin-6-yl)urea;
(30) 1-(cyclopropylmethyl)-3-(8-((2,6-dimethylbenzyl)amino)-2,3-dimethylimidazo[1,2-a]pyridin-6-yl)urea;
(31) 1-(8-((2,6-dimethylbenzyl)amino)-2,3-dimethylimidazo[1,2-a]pyridin-6-yl)-3-isopropylurea;
(32) 3-(8-((2,6-dimethylbenzyl)amino)-2,3-dimethylimidazo[1,2-a]pyridin-6-yl)-1-ethyl-1-methylurea;
(33) 3-(8-((2,6-dimethylbenzyl)amino)-2,3-dimethylimidazo[1,2-a]pyridin-6-yl)-1-ethyl-1-methylurea;
(34) 3-((8-((2,6-dimethylbenzyl)amino)-2,3-dimethylimidazo[1,2-a]pyridin-6-yl)amino)-3-oxopropanoate;
(35) (S)-N-(8-((2,6-dimethylbenzyl)amino)-2,3-dimethylimidazo[1,2-a]pyridin-6-yl)azetidine-2-carboxamide;
(36) (R)-N-(8-((2,6-dimethylbenzyl)amino)-2,3-dimethylimidazo[1,2-a]pyridin-6-yl)azetidine-2-carboxamide;
(37) 1-(2-aminoethyl)-3-(8-((2,6-dimethylbenzyl)amino)-2,3-dimethylimidazo[1,2-a]pyridin-6-yl)urea;
(38) 4-((8-((2,6-dimethylbenzyl)amino)-2,3-dimethylimidazo[1,2-a]pyridin-6-yl)amino)-4-oxobutanoic acid;
(39) 3-(3-((8-((2,6-dimethylbenzyl)amino)-2,3-dimethylimidazo[1,2-a]pyridin-6-yl)amino)-3-oxopropyl)propanoic acid;
(40) ethyl 4-((8-((2,6-dimethylbenzyl)amino)-2,3-dimethylimidazo[1,2-a]pyridin-6-yl)amino)-4-oxobutanoate;

(41) tert-butyl 3-(3-((8-((2,6-dimethylbenzyl)amino)-2,3-dimethylimidazo[1,2-a]pyridin-6-yl)amino)-3-oxopropyl)propanoate;
(42) tert-butyl (2-(3-(8-((2,6-dimethylbenzyl)amino)-2,3-dimethylimidazo[1,2-a]pyridin-6-yl)ureido)ethyl)carbamate;
(43) 3-(8-((2,6-dimethylbenzyl)amino)-2,3-dimethylimidazo[1,2-a]pyridin-6-yl)-1,1-dimethylurea;
(44) 1-(8-((4-fluoro-2,6-dimethylbenzyl)amino)-2,3-dimethylimidazo[1,2-a]pyridin-6-yl)-3-methylurea;
(45) N-(8-((4-fluoro-2,6-dimethylbenzyl)amino)-2,3-dimethylimidazo[1,2-a]pyridin-6-yl)acetamide;
(46) methyl ((8-((2,6-dimethylbenzyl)amino)-2,3-dimethylimidazo[1,2-a]pyridin-6-yl)carbamoyl)glycinate;
(47) 2-(3-(8-((2,6-dimethylbenzyl)amino)-2,3-dimethylimidazo[1,2-a]pyridin-6-yl)ureido)-N-methylacetamide;
(48) 2-(3-(8-((2,6-dimethylbenzyl)amino)-2,3-dimethylimidazo[1,2-a]pyridin-6-yl)ureido)-N,N-dimethylacetamide;
(49) 3-(8-((4-fluoro-2,6-dimethylbenzyl)amino)-2,3-dimethylimidazo[1,2-a]pyridin-6-yl)-1,1-dimethylurea;
(50) 1-cyclopropyl-3-(8-((4-fluoro-2,6-dimethylbenzyl)amino)-2,3-dimethylimidazo[1,2-a]pyridin-6-yl)urea;
(51) N-(8-((4-fluoro-2,6-dimethylbenzyl)amino)-2,3-dimethylimidazo[1,2-a]pyridin-6-yl)azetidine-1-carboxamide;
(52) 2-acetamido-N-(8-((2,6-dimethylbenzyl)amino)-2,3-dimethylimidazo[1,2-a]pyridin-6-yl)acetamide;
(53) 2-(dimethylamino)-N-(8-((2,6-dimethylbenzyl)amino)-2,3-dimethylimidazo[1,2-a]pyridin-6-yl)acetamide;
(54) 1-butyl-3-(8-((2,6-dimethylbenzyl)amino)-2,3-dimethylimidazo[1,2-a]pyridin-6-yl)urea;
(55) N-(2-((8-((2,6-dimethylbenzyl)amino)-2,3-dimethylimidazo[1,2-a]pyridin-6-yl)amino)-2-oxoethyl)pivalamide;
(56) N-(2-((8-((2,6-dimethylbenzyl)amino)-2,3-dimethylimidazo[1,2-a]pyridin-6-yl)amino)-2-oxoethyl)-2,2-difluoroacetamide;
(57) N-(2-((8-((2,6-dimethylbenzyl)amino)-2,3-dimethylimidazo[1,2-a]pyridin-6-yl)amino)-2-oxoethyl)-2,2-difluoropropanamide;
(58) N-(2-((8-((2,6-dimethylbenzyl)amino)-2,3-dimethylimidazo[1,2-a]pyridin-6-yl)amino)-2-oxoethyl)-3-hydroxycyclobutane-1-carboxamide;
(59) N-(2-((8-((2,6-dimethylbenzyl)amino)-2,3-dimethylimidazo[1,2-a]pyridin-6-yl)amino)-2-oxoethyl)-3-fluorocyclobutane-1-carboxamide;
(60) 3-(8-((2,6-dimethylbenzyl)amino)-2,3-dimethylimidazo[1,2-a]pyridin-6-yl)imidazolidine-2,4-dione;
(61) N-(8-((2,6-dimethylbenzyl)amino)-2,3-dimethylimidazo[1,2-a]pyridin-6-yl)morpholine-4-carboxamide;
(62) 2-(3-(8-((2,6-dimethylbenzyl)amino)-2,3-dimethylimidazo[1,2-a]pyridin-6-yl)ureido)ethyl acetate;
(63) (S)-2-amino-N-(8-((4-fluoro-2,6-dimethylbenzyl)amino)-2,3-dimethylimidazo[1,2-a]pyridin-6-yl)propanamide;
(64) 2-amino-N-(8-((4-fluoro-2,6-dimethylbenzyl)amino)-2,3-dimethylimidazo[1,2-a]pyridin-6-yl)acetamide;
(65) (S)-N-(8-((4-fluoro-2,6-dimethylbenzyl)amino)-2,3-dimethylimidazo[1,2-a]pyridin-6-yl)azetidine-2-carboxamide;
(66) N-(8-((2,6-dimethylbenzyl)amino)-2,3-dimethylimidazo[1,2-a]pyridin-6-yl)-2-azaspiro [3.3]heptane-2-carboxamide;
(67) N-(8-((2,6-dimethylbenzyl)amino)-2,3-dimethylimidazo[1,2-a]pyridin-6-yl)-2-azaspiro [3.3]heptane-2-carboxamide;
(68) N-(8-((2,6-dimethylbenzyl)amino)-2,3-dimethylimidazo[1,2-a]pyridin-6-yl)-1,3-dihydro-2H-pyrrolo[3,4-c] pyridine-2-carboxamide;
(69) N-(8-((2,6-dimethylbenzyl)amino)-2,3-dimethylimidazo[1,2-a]pyridin-6-yl)-3-oxoazetidine-1-carboxamide;
(70) ((8-((2,6-dimethylbenzyl)amino)-2,3-dimethylimidazo[1,2-a]pyridin-6-yl)carbamoyl)glycine;
(71) 3-(3-(8-((2,6-dimethylbenzyl)amino)-2,3-dimethylimidazo[1,2-a]pyridin-6-yl)ureido)propionic acid;
(72) 3-(8-((2,6-dimethylbenzyl)amino)-2,3-dimethylimidazo[1,2-a]pyridin-6-yl)-1,3,5-triazepane-2,4-dione;
(73) N-(2-(3-(8-((2,6-dimethylbenzyl)amino)-2,3-dimethylimidazo[1,2-a]pyridin-6-yl)ureido)ethyl)acetamide;
(74) 1-(8-((2,6-dimethylbenzyl)amino)-2,3-dimethylimidazo[1,2-a]pyridin-6-yl)-3-((tetrohydrofuran-3-yl)methyl) urea;
(75) (R)-N-(8-((2,6-dimethylbenzyl)amino)-2,3-dimethylimidazo[1,2-a]pyridin-6-yl)-2-methylazetidine-2-carboxamide;
(76) (R)-2-(azetidin-2-yl)-N-(8-((2,6-dimethylbenzyl)amino)-2,3-dimethylimidazo[1,2-a]pyridin-6-yl)acetamide;
(77) N-(8-((2,6-dimethylbenzyl)amino)-2,3-dimethylimidazo[1,2-a]pyridin-6-yl)-3-phenylazetidine-1-carboxamide;
(78) (S)-1-(2,3-dihydro-1H-inden-1-yl)-3-(8-((2,6-dimethylbenzyl)amino)-2,3-dimethylimidazo[1,2-a]pyridin-6-yl)urea;
(79) (S)-N2-(8-((2,6-dimethylbenzyl)amino)-2,3-dimethylimidazo[1,2-a]pyridin-6-yl)-N1-methylazetidine-1,2-dicarboxamide;

(80) (R)-1-(2,3-dihydro-1H-inden-1-yl)-3-(8-((2,6-dimethylbenzyl)amino)-2,3-dimethylimidazo[1,2-a]pyridin-6-yl)urea;
(81) N-(8-((2,6-dimethylbenzyl)amino)-2,3-dimethylimidazo[1,2-a]pyridin-6-yl)-2-(2-oxoazetidin-1-yl)acetamide;
(82) N-(8-((2,6-dimethylbenzyl)amino)-2,3-dimethylimidazo[1,2-a]pyridin-6-yl)-4-phenylpiperidine-1-carboxamide;
(83) N-(8-((2,6-dimethylbenzyl-l)amino)-2,3-dimethylimidazo[1,2-a]pyridin-6-yl)-3-phenylpiperidine-1-carboxamide;
(84) N-(8-((2,6-dimethylbenzyl)amino)-2,3-dimethylimidazo[1,2-a]pyridin-6-yl)-3-phenylpyrrolidine-1-carboxamide;
(85) (R)-N-(8-((2,6-dimethylbenzyl)amino)-2,3-dimethylimidazo[1,2-a]pyridin-6-yl)piperazine-2-carboxamide;
(86) N-(8-((4-fluoro-2,6-dimethylbenzyl)amino)-2,3-dimethylimidazo[1,2-a]pyridin-6-yl)-2-hydroxyacetamide;
(87) 1-(2-aminoethyl)-3-(8-((2,6-dimethylbenzyl)amino)-2,3-dimethylimidazo[1,2-a]pyridin-6-yl)-1-methylurea;
(88) 1-(8-((2,6-dimethylbenzyl)amino)-2,3-dimethylimidazo[1,2-a]pyridin-6-yl)-3-(2-hydroxyethyl)urea;
(89) (R)-N-(8-((2,6-dimethylbenzyl)amino)-2,3-dimethylimidazo[1,2-a]pyridin-6-yl)pyrrolidine-3-carboxamide;
(90) (R)-N-(8-((2,6-dimethylbenzyl)amino)-2,3-dimethylimidazo[1,2-a]pyridin-6-yl)piperidine-2-carboxamide;
(91) (R)-N-(8-((2,6-dimethylbenzyl)amino)-2,3-dimethylimidazo[1,2-a]pyridin-6-yl)-2-(pyrrolidin-2-yl)acetamide;
(92) $N^8$-(2,6-dimethylbenzyl)-$N^6$-isopropyl-2,3-dimethylimidazo[1,2-a]pyridin-6,8-diamine;
(93) (S)-N-(8-((2,6-dimethylbenzyl)amino)-2,3-dimethylimidazo[1,2-a]pyridin-6-yl)-2-(pyrrolidin-2-yl)acetamide;
(94) (S)-N-(8-((2,6-dimethylbenzyl)amino)-2,3-dimethylimidazo[1,2-a]pyridin-6-yl)piperazine-2-carboxamide;
(95) N-(8-((2,6-dimethylbenzyl)amino)-2,3-dimethylimidazo[1,2-a]pyridin-6-yl)-2-(2-hydroxyacetamido)acetamide;
(96) (S)-N-(8-((2,6-dimethylbenzyl)amino)-2,3-dimethylimidazo[1,2-a]pyridin-6-yl)-2-hydroxypropanamide;
(97) (R)-N-(8-((2,6-dimethylbenzyl)amino)-2,3-dimethylimidazo[1,2-a]pyridin-6-yl)-2-hydroxypropanamide;
(98) N-(8-((2,6-dimethylbenzyl)amino)-2,3-dimethylimidazo[1,2-a]pyridin-6-yl)-2-(methylamino)acetamide;
(99) 3-amino-N-(8-((2,6-dimethylbenzyl)amino)-2,3-dimethylimidazo[1,2-a]pyridin-6-yl)propanamide;
(100) N-(8-((4-fluoro-2,6-dimethylbenzyl)amino)-2,3-dimethylimidazo[1,2-a]pyridin-6-yl)-2-methoxyacetamide;
(101) N-(8-((4-fluoro-2,6-dimethylbenzyl)amino)-2,3-dimethylimidazo[1,2-a]pyridin-6-yl)-3-hydroxypropanamide;
(102) N-(8-((2,6-dimethylbenzyl)amino)-2,3-dimethylimidazo[1,2-a]pyridin-6-yl)-3-hydroxypropanamide;
(103) N-(8-((2,6-dimethylbenzyl)amino)-2,3-dimethylimidazo[1,2-a]pyridin-6-yl)piperazine-1-carboxamide;
(104) N-(8-((2,6-dimethylbenzyl)amino)-2,3-dimethylimidazo[1,2-a]pyridin-6-yl)benzamide;
(105) (S)-N-(8-((2,6-dimethylbenzyl)amino)-2,3-dimethylimidazo[1,2-a]pyridin-6-yl)pyrrolidine-3-carboxamide;
(106) (S)-N-(8-((2,6-dimethylbenzyl)amino)-2,3-dimethylimidazo[1,2-a]pyridin-6-yl)piperidine-2-carboxamide;
(107) (E)-4-(3-((8-((2,6-dimethylbenzyl)amino)-2,3-dimethylimidazo[1,2-a]pyridin-6-yl)amino)-3-oxoprop-1-en-1-yl)phenyl acetate;
(108) (E)-4-(3-((8-((2,6-dimethylbenzyl)amino)-2,3-dimethylimidazo[1,2-a]pyridin-6-yl)amino)-3-oxoprop-1-en-1-yl)-2-methoxyphenyl acetate;
(109) (S)-N-(8-((2,6-dimethylbenzyl)amino)-2,3-dimethylimidazo[1,2-a]pyridin-6-yl)aziridine-2-carboxamide;
(110) (S)-N-(8-((2,6-dimethylbenzyl)amino)-2,3-dimethylimidazo[1,2-a]pyridin-6-yl)-1-(2-hydroxyacetyl)pyrrolidine-3-carboxamide;
(111) (R)-1-((8-((4-fluoro-2,6-dimethylbenzyl)amino)-2,3-dimethylimidazo[1,2-a]pyridin-6-yl)amino)-1-oxopropan-2-yl acetate;
(112) (S)-1-((8-((4-fluoro-2,6-dimethylbenzyl)amino)-2,3-dimethylimidazo[1,2-a]pyridin-6-yl)amino)-1-oxopropan-2-yl acetate;
(113) 1-((8-((4-fluoro-2,6-dimethylbenzyl)amino)-2,3-dimethylimidazo[1,2-a]pyridin-6-yl)carbamoyl)cyclopropyl acetate;
(114) (S)-N-(8-((4-fluoro-2,6-dimethylbenzyl)amino)-2,3-dimethylimidazo[1,2-a]pyridin-6-yl)-2-hydroxypropanamide;
(115) N-(8-((4-fluoro-2,6-dimethylbenzyl)amino)-2,3-dimethylimidazo[1,2-a]pyridin-6-yl)-1-hydroxycyclopropane-1-carboxamide;
(116) 3-(8-((4-fluoro-2,6-dimethylbenzyl)amino)-2,3-dimethylimidazo[1,2-a]pyridin-6-yl)-1-methylimidazolidine-2,4-dione;
(117) (R)-N-(8-((4-fluoro-2,6-dimethylbenzyl)amino)-2,3-dimethylimidazo[1,2-a]pyridin-6-yl)-2-hydroxypropanamide;
(118) (S)-N-(8-((2,6-dimethylbenzyl)amino)-2,3-dimethylimidazo[1,2-a]pyridin-6-yl)piperidine-3-carboxamide;

(119) N-(8-((2,6-dimethylbenzyl)amino)-2,3-dimethylimidizo[1,2-a]pyridin-6-yl)-3-(2-hydroxyacetamido)propanamide;
(120) (S)-N-(8-((2,6-dimethylbenzyl)amino)-2,3-dimethylimidazo[1,2-a]pyridin-6-yl)-1-(2-hydroxyacetyl)piperidine-3-carboxamide;
(121) $N^8$-(2,6-dimethylbenzyl)-2,3-dimethylimidazo[1,2-a]pyridine-6,8-diamine; and
(122) $N^8$-(4-fluoro-2,6-dimethylbenzyl)-2,3-dimethylimidazo[1,2-a]pyridine-6,8-diamine.

**6.** The imidazopyridine compound of Claim 1 or a pharmaceutically acceptable salt thereof, wherein the compound or a pharmaceutically acceptable salt thereof has an inhibitory effect on proton pump activity.

**7.** A pharmaceutical composition for preventing or treating a gastrointestinal inflammatory disease or gastric acid-related disease, comprising an imidazopyridine compound of any one of claims 1 to 6 or a pharmaceutically acceptable salt thereof as an active ingredient.

**8.** The pharmaceutical composition preventing or treating a gastrointestinal inflammatory disease or gastric acid-related disease of claim 7, wherein the compound or a pharmaceutically acceptable salt thereof acts as a reversible proton pump inhibitor.

**9.** The pharmaceutical composition preventing or treating a gastrointestinal inflammatory disease or gastric acid-related disease of claim 7, wherein the compound or a pharmaceutically acceptable salt thereof suppresses gastric acid secretion.

**10.** The pharmaceutical composition preventing or treating a gastrointestinal inflammatory disease or gastric acid-related disease of claim 7, wherein the gastrointestinal inflammatory disease or gastric acid-related disease is at least one selected from the group consisting of peptic ulcer, gastric/duodenal ulcer, nonsteroidal anti-inflammatory drug (NSAID)-induced ulcer, Helicobacter pylori infection, functional dyspepsia, Zollinger-Ellison syndrome, gastritis, and gastroesophageal reflux disease (GERD) and non-erosive reflux disease (NERD).

**11.** A health functional food composition for preventing or improving a gastrointestinal inflammatory disease or gastric acid-related disease, comprising an imidazopyridine compound of any one of claims 1 to 6 or a pharmaceutically acceptable salt thereof as an active ingredient.

# INTERNATIONAL SEARCH REPORT

International application No.
**PCT/KR2024/013975**

**A. CLASSIFICATION OF SUBJECT MATTER**

**C07D 471/04**(2006.01)i; **A61K 31/437**(2006.01)i; **A61P 1/00**(2006.01)i; **A61P 1/04**(2006.01)i; **A61P 29/00**(2006.01)i; **A23L 33/10**(2016.01)i

According to International Patent Classification (IPC) or to both national classification and IPC

**B. FIELDS SEARCHED**

Minimum documentation searched (classification system followed by classification symbols)

C07D 471/04(2006.01); A61K 31/435(2006.01); A61K 31/437(2006.01); A61K 31/44(2006.01); C07D 471/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (registry, caplus) & keywords: 이미다조피리딘(imidazopyridine), 칼륨 경쟁적 위산 분비 억제제(Potassium-competitive acid blocker, P-CAB), 위장관 염증질환(gastrointestinal inflammatory disease), 위산-관련 질환(gastric acid-related disease)

**C. DOCUMENTS CONSIDERED TO BE RELEVANT**

| Category* | Citation of document, with indication, where appropriate, of the relevant passages | Relevant to claim No. |
|---|---|---|
| X | KR 10-2001-0072798 A (ASTRAZENECA AB) 31 July 2001 (2001-07-31)<br>See claims 1, 9-11, 16 and 17; paragraphs [0004], [0209], [0507] and [0508]; and example 1.69. | 1-11 |
| X | WO 2007-003386 A1 (GLAXO GROUP LIMITED et al.) 11 January 2007 (2007-01-11)<br>See claims 1, 21-26 and 30; and page 19, example 1. | 1,3-11 |
| X | BAILEY, N. et al. Orally active C-6 heteroaryl- and heterocyclyl-substituted imidazo[1,2-a]pyridine acid pump antagonists (APAs). Bioorganic & Medicinal Chemistry Letters. 2009, vol. 19, pp. 3602-3606.<br>See abstract; page 1; table 1; and compound 13. | 1,3-11 |
| A | US 4920129 A (SHIOKAWA, Y. et al.) 24 April 1990 (1990-04-24)<br>See claim 1. | 1-11 |
| A | WO 2004-026867 A2 (SCHERING CORPORATION) 01 April 2004 (2004-04-01)<br>See claim 1. | 1-11 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 December 2024** | **24 December 2024** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**
**Information on patent family members**

International application No.
**PCT/KR2024/013975**

| Patent document cited in search report | | | Publication date (day/month/year) | Patent family member(s) | | | Publication date (day/month/year) |
|---|---|---|---|---|---|---|---|
| KR | 10-2001-0072798 | A | 31 July 2001 | CN | 100329613 | A | 02 January 2002 |
| | | | | EP | 1105391 | A2 | 13 June 2001 |
| | | | | EP | 1105391 | B1 | 27 October 2004 |
| | | | | EP | 1411056 | A2 | 21 April 2004 |
| | | | | EP | 1411056 | A3 | 28 April 2004 |
| | | | | JP | 2002-523415 | A | 30 July 2002 |
| | | | | US | 2003-0220364 | A1 | 27 November 2003 |
| | | | | US | 2005-0154007 | A1 | 14 July 2005 |
| | | | | US | 6579884 | B1 | 17 June 2003 |
| | | | | US | 6790960 | B2 | 14 September 2004 |
| | | | | WO | 00-11000 | A2 | 02 March 2000 |
| | | | | WO | 00-11000 | A3 | 15 June 2000 |
| WO | 2007-003386 | A1 | 11 January 2007 | EP | 1896471 | A1 | 12 March 2008 |
| | | | | JP | 2009-500304 | A | 08 January 2009 |
| | | | | US | 2010-0311740 | A1 | 09 December 2010 |
| US | 4920129 | A | 24 April 1990 | CN | 100033628 | A | 05 July 1989 |
| | | | | EP | 0308917 | A2 | 29 March 1989 |
| | | | | EP | 0308917 | A3 | 11 July 1990 |
| | | | | JP | 01-151579 | A | 14 June 1989 |
| | | | | KR | 10-1989-0005100 | A | 11 May 1989 |
| WO | 2004-026867 | A2 | 01 April 2004 | CN | 100703414 | A | 30 November 2005 |
| | | | | EP | 1539756 | A2 | 15 June 2005 |
| | | | | EP | 1539756 | B1 | 14 November 2007 |
| | | | | JP | 2006-507254 | A | 02 March 2006 |
| | | | | JP | 2006-507254 | A5 | 05 October 2006 |
| | | | | JP | 4845379 | B2 | 28 December 2011 |
| | | | | KR | 10-2005-0052500 | A | 02 June 2005 |
| | | | | US | 2004-0097517 | A1 | 20 May 2004 |
| | | | | US | 2006-0030555 | A1 | 09 February 2006 |
| | | | | US | 6992080 | B2 | 31 January 2006 |
| | | | | US | 7452902 | B2 | 18 November 2008 |
| | | | | WO | 2004-026867 | A3 | 05 August 2004 |

Form PCT/ISA/210 (patent family annex) (July 2022)